# EUROPEAN PATENT SPECIFICATION

(11) **EP 4 462 102 B1**
(45) Date of publication and mention of the grant of the patent: **12.08.2026**
(21) Application number: 24175080.1
(22) Date of filing: 09.05.2024
(51) Int. Cl.: G01N 15/1434, G01N 15/14, G01N 15/149, G01N 15/10

(54) **METHODS FOR IMAGE-BASED DETECTION AND SORTING AND SYSTEMS FOR SAME**
VERFAHREN ZUR BILDBASIERTEN DETEKTION UND SORTIERUNG UND SYSTEME DAFÜR
PROCÉDÉS DE DÉTECTION ET DE TRI BASÉS SUR DES IMAGES ET SYSTÈMES ASSOCIÉS

(30) Priority: 09.05.2023 US 202363465059 P
(43) Date of publication of application: 13.11.2024
(73) Proprietor: BECTON, DICKINSON AND COMPANY, Franklin Lakes NJ 07417-1880 (US)
(72) Inventor: TYZNIK, Aaron, Cookeville, Tennessee 38506 (US); MIDDLEBROOK, Aaron Jacob, San Jose, California 95124 (US); DIEBOLD, Eric, Menlo Park, California 94025 (US); OWSLEY, Keegan, Campbell, California 95008 (US); SHI, Shirley, Fremont, California 94538 (US); LOMAS, Woodrow E., San Jose, California 95112 (US)
(74) Representative: Bals & Vogel Patentanwälte PartGmbB

(56) References cited:
- US-A1- 2020 302 606
- US-B2- 11 579 071
- SCHRAIVOGEL DANIEL ET AL: "High-speed fluorescence image-enabled cell sorting", vol. 375, no. 6578, 21 January 2022 (2022-01-21), US, pages 315 - 320, XP093094958, ISSN: 0036-8075, Retrieved from the Internet <URL:https://www.science.org/doi/pdf/10.1126/science.abj3013> [retrieved on 20231025], DOI: 10.1126/science.abj3013

## Description

### CROSS-REFERENCE TO RELATED APPLICATION

Pursuant to 35 U.S.C. § 119 (e), this application claims priority to the filing dates of United States Provisional Patent Application Serial No 63/465,059 filed May 9, 2023.

### INTRODUCTION

Flow-type particle sorting systems, such as sorting flow cytometers, are used to sort particles in a fluid sample based on at least one measured characteristic of the particles. In a flow-type particle sorting system, particles, such as analyte-bound beads or individual cells in a fluid suspension are passed in a stream by a detection region in which a sensor detects particles contained in the stream of the type to be sorted. The sensor, upon detecting a particle of the type to be sorted, triggers a sorting mechanism that selectively isolates the particle of interest.

Particle sensing typically is carried out by passing the fluid stream by a detection region in which the particles are exposed to irradiating light, from one or more lasers, and fluorescence from the particles is measured. Particles or components thereof can be labeled with fluorescent dyes to facilitate detection, and a multiplicity of different particles or components may be simultaneously detected by using spectrally distinct fluorescent dyes to label the different particles or components. Detection is carried out using one or more photosensors to facilitate the independent measurement of the fluorescence of each distinct fluorescent dye.

Using data generated from the detected light, distributions of the components can be recorded and where desired material may be sorted. To sort particles in the sample, a drop charging mechanism charges droplets of the flow stream containing a particle type to be sorted with an electrical charge at the break-off point of the flow stream. Droplets are passed through an electrostatic field and are deflected based on polarity and magnitude of charge on the droplet into one or more collection containers. Uncharged droplets are not deflected by the electrostatic field.

US 2020/302606 A1 and SCHRAIVOGEL DANIEL ET AL: "High-speed fluorescence image-enabled cell sorting", SCIENCE, vol. 375, no. 6578 21 January 2022 both disclose methods and devices for imaging cells according to the preamble of claim 1.

### SUMMARY

The present invention relates to a method for calculating cell parameters according to claim 1. Systems and integrated circuit devices (e.g., a field programmable gate array) for practicing the subject methods are also described. Non-transitory computer readable storage media are also provided.

In embodiments, the image data includes one or more waveforms generated in response to the measured light. An image is generated of the cell from the image data. Methods include calculating an image parameter based on one or more of the image data and the image of the cell. Calculating the cell parameter includes assessing a cell interaction parameter. The cell interaction parameter is an interaction between two or more cells in the sample. In some instances, calculating the cell interaction parameter includes assessing phagocytosis by one or more cells in the sample. In some instances, calculating the cell interaction parameter includes assessing tumor cell killing by one or more cells in the sample. In some instances, calculating the cell interaction parameter includes assessing internalization of a cell or cellular component by one or more cells in the sample. In some instances, calculating the cell interaction parameter includes assessing co-localization between two or more cells in the sample.

In some instances, the cell parameter includes a translocation parameter of a cellular component. In some instances, calculating the cell parameter includes assessing translocation of a cellular component within the cell (e.g., displacement of a cellular component such as a protein from one location in the cell to another location in the cell). In some instances, methods include assessing nuclear translocation of a cellular component within the cell.

In some examples, the cell parameter is a cell mitotic phase parameter. In some instances, calculating the cell parameter includes determining the mitotic phase of one or more cells in the sample. In some instances, the cell is identified as being in interphase. In some instances, the cell is identified as being in prophase. In some instances, the cell is identified as being in metaphase. In some instances, the cell is identified as being in anaphase. In some instances, the cell is identified as being in telophase. In certain instances, the cell is identified as being in cytokinesis.

In some embodiments, calculating the cell parameter includes identifying that a cell is a plant cell. In some instances, identifying the plant cell includes assessing the morphology of the cell. In some instances, one or more of the size of the cell, the shape of the cell and the degree of punctateness of the cell is assessed. In some instances, the cell is identified as being a plant cell by distinguishing the cell from cellular debris. In some instances, the cell is identified as being a plant distinguishing the cell from a cell doublet.

In some instances, methods include calculating one or more image parameters from a generated image of the cells. In some instances, the cell parameter is calculated based on the image parameter. In some instances, the image parameter is a quantitative image parameter. In some instances, the parameter is selected from center of mass, delta center of mass, diffusivity, eccentricity, long axis moment, maximum intensity, radial moment, short axis moment, size, total intensity, light loss by the cell nuclei, forward scattered light by the cell nuclei, side scattered light by the cell nuclei and combinations thereof. In some instances, the image parameter is calculated from images generated based on fluorescence measured from the cells, light loss measured for the cells, forward scattered light measured for the cells, side scattered light measured for the cells and combinations thereof.

In some embodiments, methods include classifying cells of the sample based on the calculated cell parameter. In some instances, the cells are classified based on waveforms generated from the measured light, generated images of the cell, image parameter calculated from the generated images of the cell or a combination thereof. In some instances, classifying the cells includes assigning the cells to one or more particle population clusters. In some instances, methods include determining one or more sorting gates of the classified cells of the sample. In some instances, the one or more sorting gates capture cells of a target particle population cluster and exclude particles of a non-target particle population cluster. In some instances, the sorting gates maximize the inclusion yield of cells of a target particle population cluster. In some instances, the sorting gates maximize the exclusion of cells of a non-target particle population cluster.

In some instances, methods include sorting cells of the sample into a plurality of sample containers. In some instances, the cells are sorted based on the generated images of the cells. In some instances, the cells are sorted based on the calculated image parameters.

Methods include irradiating the sample with frequency-modulated beams of light. The sample is irradiated in a flow stream. In some instances, the light source includes one or more lasers. In some instances, light is detected with a light detection system having a plurality of photodetectors. In some embodiments, one or more of the photodetectors is a photomultiplier tube. In some embodiments one or more of the photodetectors is a photodiode (e.g., an avalanche photodiode, APD). In certain embodiments, the light detection system includes a photodetector array, such as a photodetector array having a plurality of photodiodes or charged coupled devices (CCDs).

Aspects of the present disclosure also include systems for practicing the subject methods. Systems according to certain embodiments include a light source configured to irradiate cells of a sample in a flow stream with frequency-modulated beams of light, a light detection system having a photodetector for measuring light from the cells, and a processor with memory operably coupled to the processor where the memory includes instructions stored thereon, which when executed by the processor, cause the processor to generate image data of the cells from the measured light and calculate one or more cell parameters based on the generated image data.

In some embodiments, the image data includes one or more waveforms generated in response to the measured light. The memory includes instructions for generating an image of the cell from the image data. The memory includes instructions for calculating an image parameter based on one or more of the image data and the image of the cell. The memory includes instructions for assessing a cell interaction parameter. The memory includes instructions for assessing an interaction between two or more cells in the sample. In some instances, the memory includes instructions for assessing phagocytosis by one or more cells in the sample. In some instances, the memory includes instructions for assessing tumor cell killing by one or more cells in the sample. In some instances, the memory includes instructions for calculating assessing co-localization between two or more cells in the sample. In some instances, the memory includes instructions for assessing translocation of a cellular component within the cell. In some instances, the memory includes instructions for assessing nuclear translocation of a cellular component within the cell.

In some embodiments, the memory includes instructions for determining the mitotic phase of one or more cells in the sample. In some instances, the cell is identified as being in interphase. In some instances, the cell is identified as being in prophase. In some instances, the cell is identified as being in metaphase. In some instances, the cell is identified as being in anaphase. In some instances, the cell is identified as being in telophase. In certain instances, the cell is identified as being in cytokinesis.

In some embodiments, the memory includes instructions for identifying that a cell is a plant cell. In some instances, the memory includes instructions for identifying the plant cell by assessing the morphology of the cell. In some instances, the memory includes instructions for assessing one or more of the size of the cell, the shape of the cell and the degree of punctateness of the cell.

The memory includes instructions for calculating the cell parameter based on an image parameter generated from an image of the cell. The image parameter is a quantitative image parameter. In some instances, the image parameter is selected from the group consisting of center of mass, delta center of mass, diffusivity, long axis moment maximum intensity, short axis moment, size, total intensity, light loss by the cell nuclei, forward scattered light by the cell nuclei, side scattered light by the cell nuclei and combinations thereof. According to the present invention, eccentricity and radial moment are calculated based on the generated image data. In some instances, the memory includes instructions for calculating the image parameter from images generated based on fluorescence measured from the cells, light loss measured for the cells, forward scattered light measured for the cells, side scattered light measured for the cells and combinations thereof.

In some instances, the memory includes instructions for classifying the cells based on the image data, a generated image of the cell and a calculated image parameter or a combination thereof. In some instances, the memory includes instructions for classifying the cells by assigning the cells to one or more particle population clusters. In some embodiments, the memory includes instructions for determining one or more sorting gates for the classified cells of the sample. In some instances, the one or more sorting gates capture cells of a target particle population cluster and exclude particles of a non-target particle population cluster. In some instances, the memory includes instructions for calculating sorting gates that maximize the inclusion yield of cells of a target particle population cluster. In some instances, the memory includes instructions for calculating sorting gates that maximize the exclusion of cells of a non-target particle population cluster.

In certain embodiments, systems include a display for displaying a graphical user interface. In some instances, the graphical user interface is configured for manually inputting one or more of the sorting gates. In some instances, the sorting gates are manually input into the graphical user interface by drawing sorting gates on a scatter plot of the particle population clusters. In some instances, the sorting gate is a hyper-rectangular sorting gate.

Non-transitory computer readable storage medium having instructions with algorithm for calculating a cell parameter for cells of a sample are also described. Non-transitory computer readable storage medium according to certain embodiments has algorithm for irradiating a sample comprising cells in a flow stream with frequency-modulated beams of light, algorithm for measuring light from the cells in the sample, algorithm for generating image data of the cells from the measured light and algorithm for calculating one or more cell parameters based on the generated image data. In some instances, the image data includes one or more waveforms generated in response to the measured light.

The non-transitory computer readable storage medium includes an algorithm for generating an image of the cell from the image data. The non-transitory computer readable storage medium includes algorithm for calculating an image parameter based on one or more of the image data and the image of the cell. The non-transitory computer readable storage medium includes an algorithm for assessing a cell interaction parameter. The non-transitory computer readable storage medium includes an algorithm for assessing an interaction between two or more cells in the sample. In some instances, the non-transitory computer readable storage medium includes an algorithm for assessing phagocytosis by one or more cells in the sample. In some instances, the non-transitory computer readable storage medium includes algorithm for assessing tumor cell killing by one or more cells in the sample. In some instances, the non-transitory computer readable storage medium includes an algorithm for calculating assessing co-localization between two or more cells in the sample. In some instances, the non-transitory computer readable storage medium includes an algorithm for assessing translocation of a cellular component within the cell. In some instances, the non-transitory computer readable storage medium includes an algorithm for assessing nuclear translocation of a cellular component within the cell.

In some embodiments, the non-transitory computer readable storage medium includes an algorithm for determining the mitotic phase of one or more cells in the sample. In some instances, the cell is identified as being in interphase. In some instances, the cell is identified as being in prophase. In some instances, the cell is identified as being in metaphase. In some instances, the cell is identified as being in anaphase. In some instances, the cell is identified as being in telophase. In certain instances, the cell is identified as being in cytokinesis.

In some embodiments, the non-transitory computer readable storage medium includes an algorithm for identifying that a cell is a plant cell. In some instances, the non-transitory computer readable storage medium includes algorithm for identifying the plant cell by assessing the morphology of the cell. In some instances, the non-transitory computer readable storage medium includes algorithm for assessing one or more of the size of the cell, the shape of the cell and the degree of punctateness of the cell.

The non-transitory computer readable storage medium includes an algorithm for calculating the cell parameter based on an image parameter generated from an image of the cell. The image parameter is a quantitative image parameter. In some instances, the image parameter is selected from the group consisting of center of mass, delta center of mass, diffusivity, long axis moment, maximum intensity, short axis moment, size, total intensity, light loss by the cell nuclei, forward scattered light by the cell nuclei, side scattered light by the cell nuclei and combinations thereof. According to the present invention, eccentricity and radial moment are calculated based on the generated image data. In some instances, the non-transitory computer readable storage medium includes algorithm for calculating the image parameter from images generated based on fluorescence measured from the cells, light loss measured for the cells, forward scattered light measured for the cells, side scattered light measured for the cells and combinations thereof.

In some instances, the non-transitory computer readable storage medium includes an algorithm for classifying the cells based on the image data, a generated image of the cell and a calculated image parameter or a combination thereof. In some instances, the non-transitory computer readable storage medium includes an algorithm for classifying the cells by assigning the cells to one or more particle population clusters. In some embodiments, the non-transitory computer readable storage medium includes an algorithm for determining one or more sorting gates for the classified cells of the sample. In some instances, the one or more sorting gates capture cells of a target particle population cluster and exclude particles of a non-target particle population cluster. In some instances, the m non-transitory computer readable storage medium includes algorithm for calculating sorting gates that maximize the inclusion yield of cells of a target particle population cluster. In some instances, the non-transitory computer readable storage medium includes algorithm for calculating sorting gates that maximize the exclusion of cells of a non-target particle population cluster.

### BRIEF DESCRIPTION OF THE FIGURES

The invention may be best understood from the following detailed description when read in conjunction with the accompanying drawings. Included in the drawings are the following figures:
**FIG. 1** depicts a flowchart for calculating cell parameters for cells of a sample according to certain embodiments.

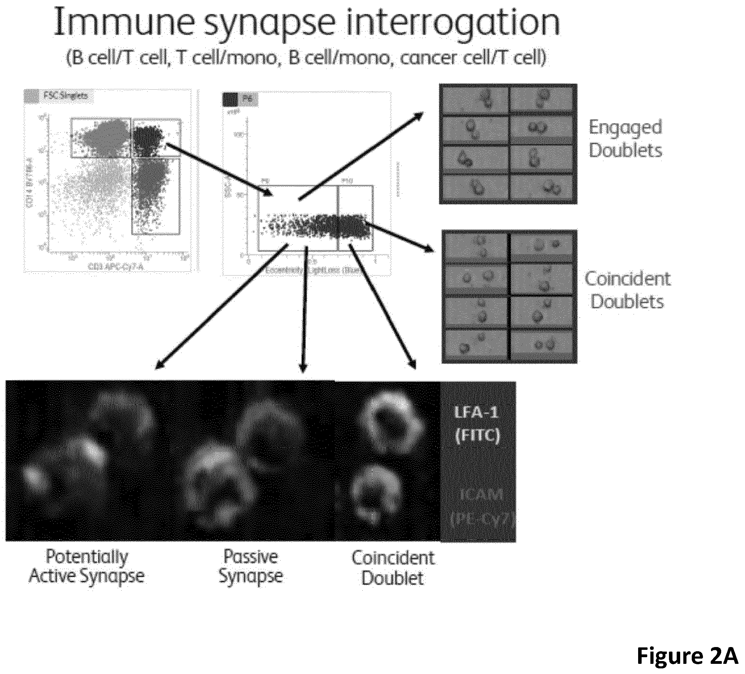

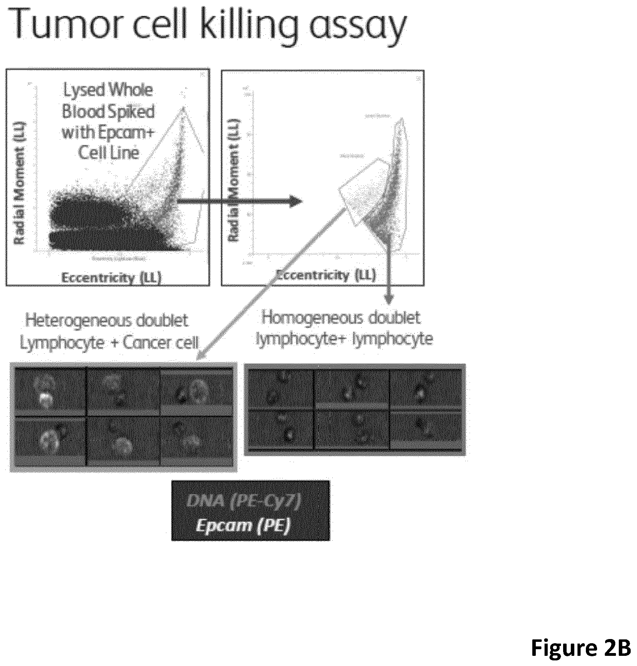

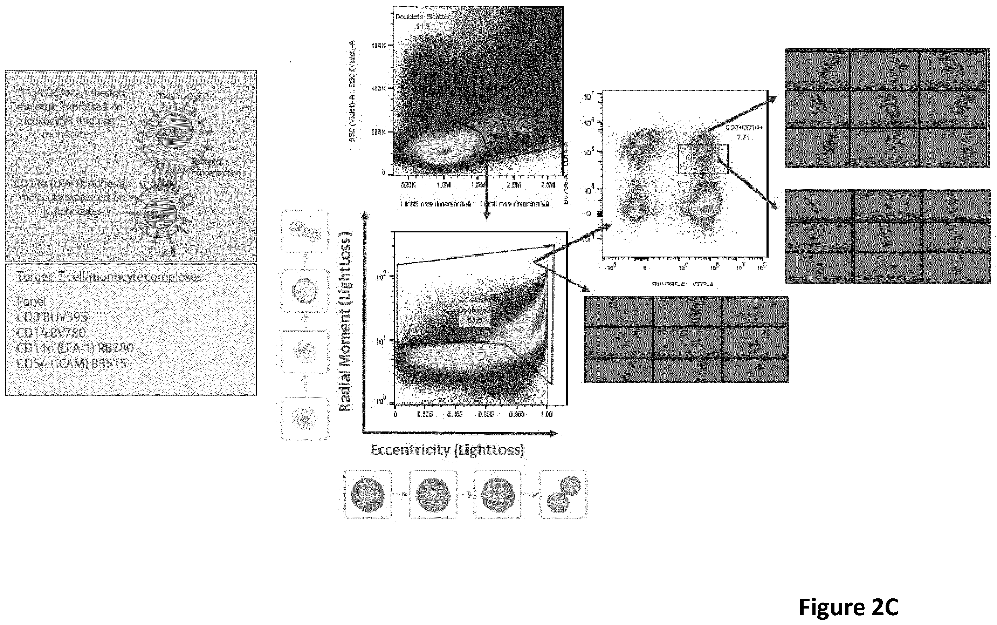

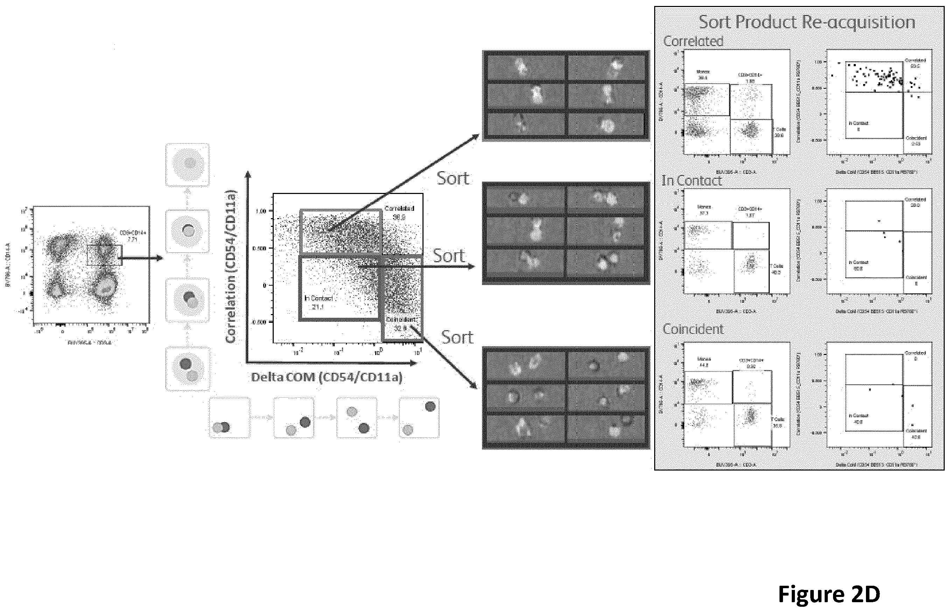

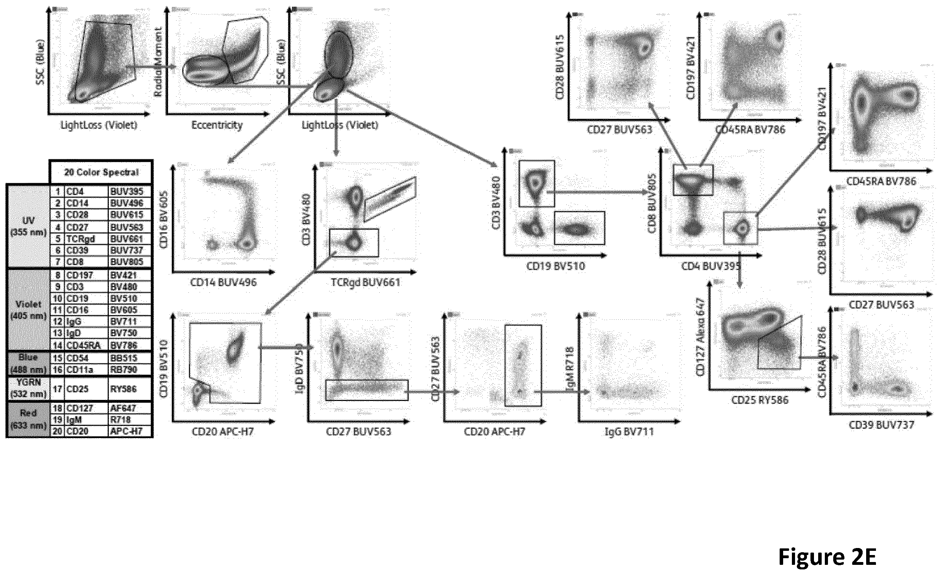

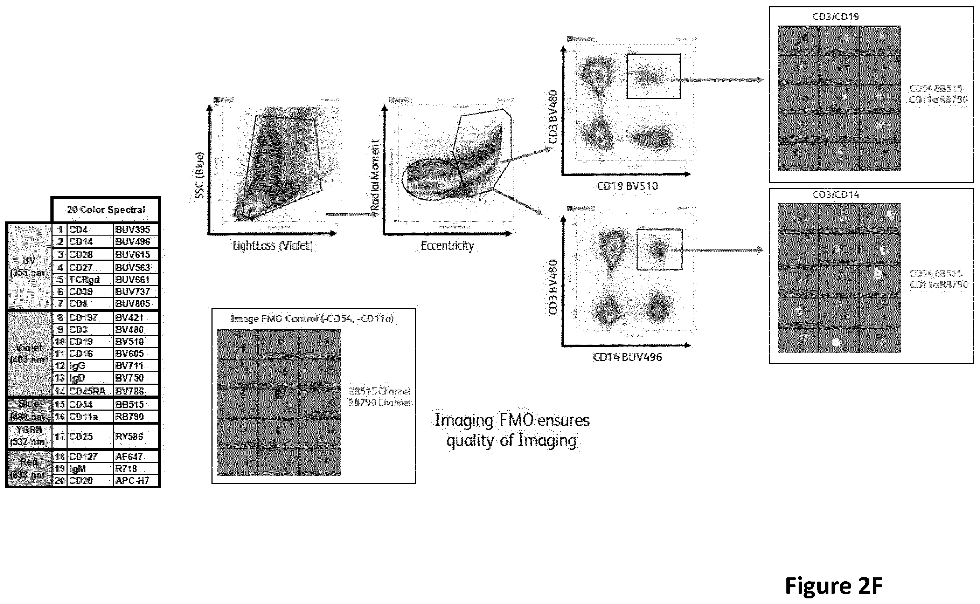

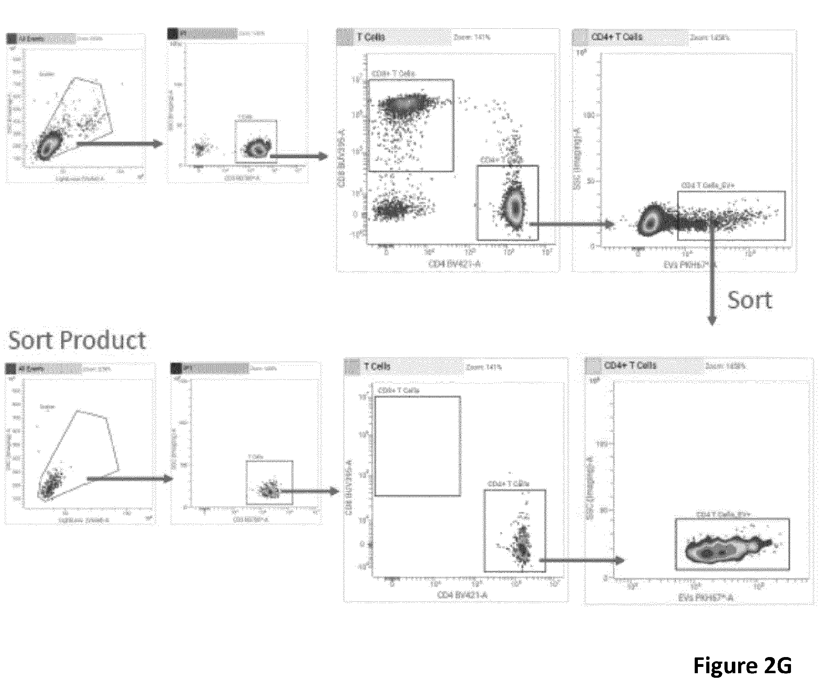

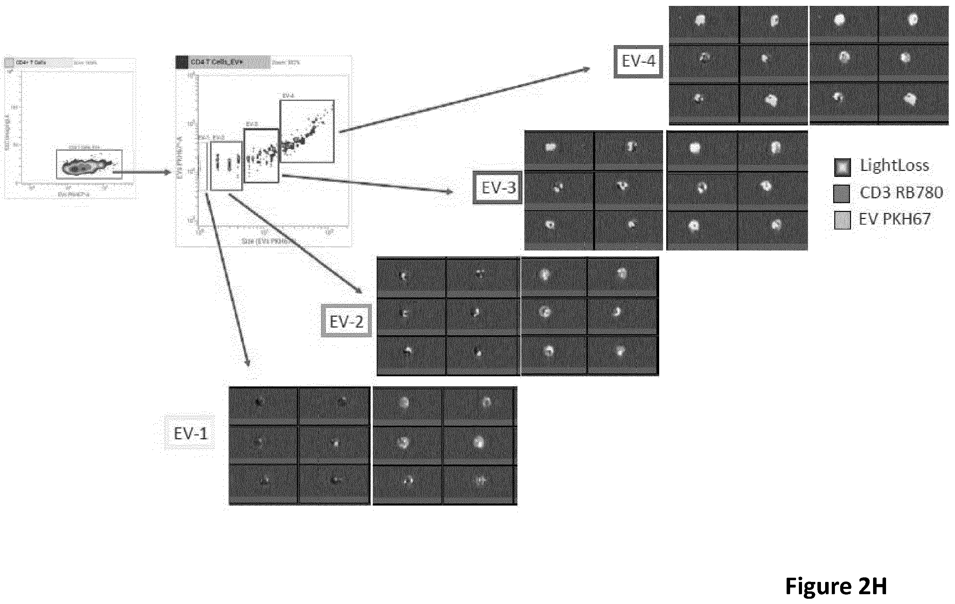

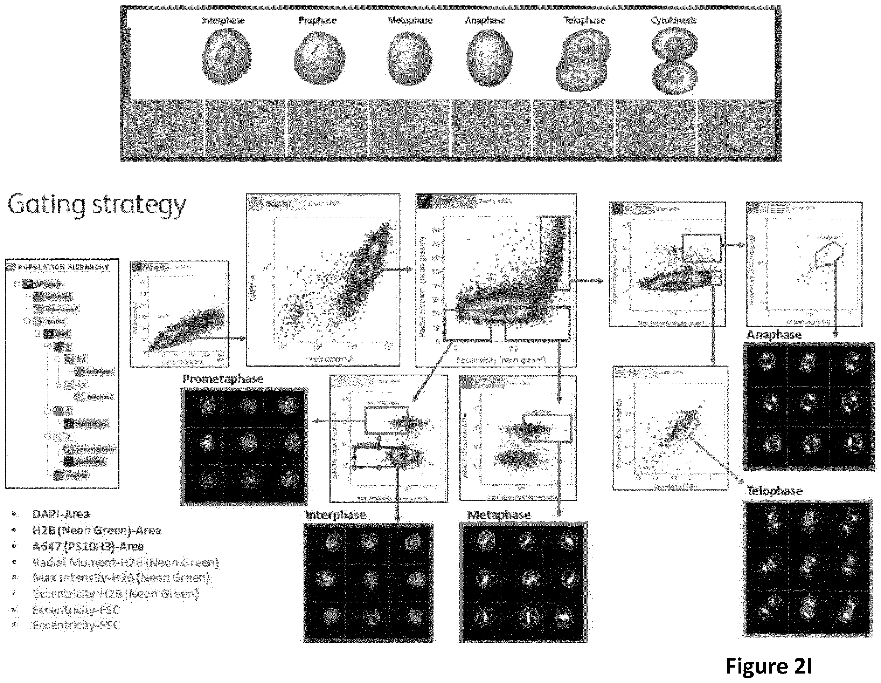

**FIG. 2** depicts cell parameters determined using an imaging cell flow cytometer for cells in a sample according to certain embodiments. **FIG. 2A** depicts calculating a cell-cell interaction parameter by immune synapse interrogation. **FIG. 2B** depicts visualization of tumor cell killing assay by an imaging flow cytometer according to certain embodiments. **FIG. 2C** depicts a visualization of T cell-monocyte cell-cell interaction complexes according to certain embodiments. **FIG. 2D** depicts calculating sorting gates for the T cell-monocyte complexes according to certain embodiments. **FIG. 2E** depicts classifying a T cell-monocyte complexes using a 20-color spectral panel as imaging parameters according to certain embodiments. **FIG. 2F** depicts calculating cell-cell interaction parameters for T cell-monocyte complexes with a multi-color fluorescence panel according to certain embodiments. **FIG. 2G** depicts classifying and sorting extracellular vesicles-CD4 T cell complexes based on cell-cell interaction parameters according to certain embodiments. **FIG. 2H** depicts images of different complexes formed between EVs and CD4 T cells according to certain embodiments. **FIG. 21** depicts calculating cell parameters for gating and sorting cells in different mitotic phases according to certain embodiments.
**FIG. 3A** depicts an image-enabled particle sorter according to certain embodiments. **FIG. 3B** depicts image-enabled particle sorting data processing according to certain embodiments.
**FIG. 4A** depicts a functional block diagram of a particle analysis system according to certain embodiments. **FIG. 4B** depicts a flow cytometer according to certain embodiments.
**FIG. 5** depicts a functional block diagram for one example of a particle analyzer control system according to certain embodiments.
**FIG. 6A** depicts a schematic drawing of a particle sorter system according to certain embodiments.
**FIG. 6B** depicts a schematic drawing of a particle sorter system according to certain embodiments.

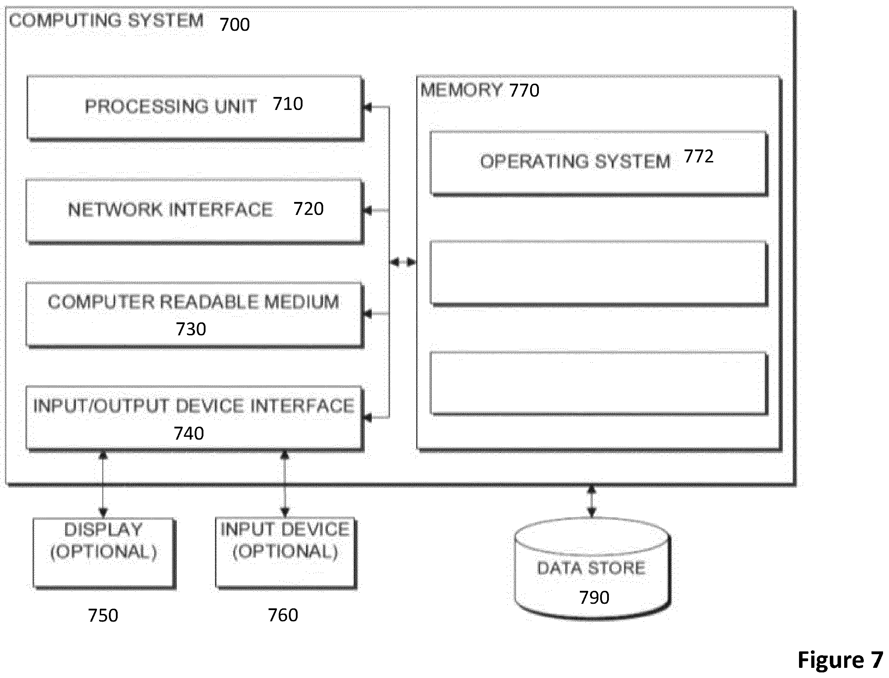

**FIG. 7** depicts a block diagram of a computing system according to certain embodiments.

### DETAILED DESCRIPTION

Aspects of the present disclosure include methods for calculating one or more cell parameters of cells in a sample. Methods according to certain embodiments include irradiating a sample comprising cells in a flow stream with frequency-modulated beams of light, measuring light from the cells in the sample, generating image data of the cells from the measured light and calculating one or more cell parameters based on the generated image data. Systems and integrated circuit devices (e.g., a field programmable gate array) for practicing the subject methods are also described. Non-transitory computer readable storage media are also provided.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about". The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope of the present invention. Any recited method can be carried out in the order of events recited or in any other order which is logically possible.

As summarized above, the present disclosure provides for calculating a cell parameter for cells of a sample in a flow stream. In further describing embodiments of the disclosure, methods for generating one or more of image data, an image from the image data and calculated image parameters and calculating cell parameters as well as in some embodiments generating sorting gates to sort cells having specific characteristics are first described in greater detail. Next, systems, integrated circuited devices and non-transitory computer readable storage medium having programming to practice the subject methods, by assessing the cell parameters and calculating sorting gates for sorting cells are also provided.

### METHODS FOR CALCULATING CELL PARAMETERS FOR CELLS OF A SAMPLE IN A FLOW STREAM

Aspects of the present disclosure include methods for calculating cell parameters of cells in a sample. In some embodiments, the subject methods provide a high-throughput and robust, yet simplified protocol for determining quantitative image data, including for generating information-rich images and calculating image parameters in a multitude of photodetector channels. The subject methods provide for applications such as assessing morphology of cells as well as cell organelles, cell interaction assays (e.g., tumor cell killing assays, internalization assays, cell-cell interaction assays, co-localization determination and phagocytosis assays). In addition, the subject methods provide for determining the cell cycle phase of each individual cell in the sample in a high-throughput fashion and can be used to gate and sort cells in a determined cell cycle phase. In certain instances, the subject methods can be used to distinguish and sort plant cells of a sample as well as identify and sort rare cells (e.g., circulating tumor cells or diseased cells).

In some embodiments, calculating cell parameters as described herein can increase the precision in characterizing the cells of sample, such as for viability, activation or status of the cells (e.g., for monitoring disease progression) by 5% or more, such as by 10% or more, such as by 15% or more, such as by 25% or more, such as by 50% or more, such as by 75% or more and including by 99% or more. In some instances, the present disclosure provides for high-throughput assays of cell samples such as where cell-cell interactions can be determined for a large number of cells using imaging data. For example, the subject methods can provide calculating a plurality of cell parameters for a sample of cells in 10 minutes or less, such as in 9 minutes or less, such as in 8 minutes or less, such as in 7 minutes or less, such as in 6 minutes or less, such as in 5 minutes or less, such as in 4 minutes or less, such as in 3 minutes or less, such as 2 minutes or less and including in 1 minute or less. As described in greater detail below, in some instances the assessed cells can be sorted and cell samples having cell parameters of interest can be prepared such as where 50% or more of the cells in the sample are suitable for use in a downstream biological assay (e.g., gene sequencing protocol), such as 60% or more, such as 70% or more, such as 80% or more, such as 90% or more, such as 95% or more, such as 97% or more and including 99% or more.

The cell parameters are assessed based at least on image data of the cells. In some instances, the image data includes waveforms generated in response to light measured from the sample that is irradiated with frequency-modulated light (as described in greater detail below). In some instances, the image data includes images generated from the waveforms. In some instances, the image data includes image parameters which are calculated from the waveforms, the generated images and a combination thereof. In some instances, the cell parameters are determined only from calculated image parameters (e.g., radial moment, eccentricity, etc.) In some instances, the cell parameters are determined only from the images of the particles and not from another data source, such as for example data signal waveforms. In some instances, the cell parameters are determined using a combination of a generated image of the particles and data signal waveforms generated in response to measured light from irradiated particles.

In practicing the subject methods according to certain embodiments, light from a sample (e.g., a sample is irradiated with a light source) having cells in a flow stream is measured with a light detection system having a photodetector. A "biological sample" can refer to a whole organism, plant, fungi or a subset of animal tissues, cells or component parts which may in certain instances be found in blood, mucus, lymphatic fluid, synovial fluid, cerebrospinal fluid, saliva, bronchoalveolar lavage, amniotic fluid, amniotic cord blood, urine, vaginal fluid and semen. As such, a "biological sample" refers to both the native organism or a subset of its tissues as well as to a homogenate, lysate or extract prepared from the organism or a subset of its tissues, including but not limited to, for example, plasma, serum, spinal fluid, lymph fluid, sections of the skin, respiratory, gastrointestinal, cardiovascular, and genitourinary tracts, tears, saliva, milk, blood cells, tumors, organs. Biological samples may be any type of organismic tissue, including both healthy and diseased tissue (e.g., cancerous, malignant, necrotic, etc.). In certain embodiments, the biological sample is a liquid sample, such as blood or derivative thereof, e.g., plasma, tears, urine, semen, etc., where in some instances the sample is a blood sample, including whole blood, such as blood obtained from venipuncture or fingerstick (where the blood may or may not be combined with any reagents prior to assay, such as preservatives, anticoagulants, etc.).

In certain embodiments the source of the sample is a "mammal" or "mammalian", where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In some instances, the subjects are humans. The methods may be applied to samples obtained from human subjects of both genders and at any stage of development (i.e., neonates, infant, juvenile, adolescent, adult), where in certain embodiments the human subject is a juvenile, adolescent or adult. While the present invention may be applied to samples from a human subject, it is to be understood that the methods may also be carried-out on samples from other animal subjects (that is, in "non-human subjects") such as, but not limited to, birds, mice, rats, dogs, cats, livestock and horses.

In some embodiments, a sample having cells (e.g., in a flow stream of a flow cytometer) is irradiated with light from a light source. In some embodiments, the light source is a broadband light source, emitting light having a broad range of wavelengths, such as for example, spanning 50 nm or more, such as 100 nm or more, such as 150 nm or more, such as 200 nm or more, such as 250 nm or more, such as 300 nm or more, such as 350 nm or more, such as 400 nm or more and including spanning 500 nm or more. For example, one suitable broadband light source emits light having wavelengths from 200 nm to 1500 nm. Another example of a suitable broadband light source includes a light source that emits light having wavelengths from 400 nm to 1000 nm. Where methods include irradiating with a broadband light source, broadband light source protocols of interest may include, but are not limited to, a halogen lamp, deuterium arc lamp, xenon arc lamp, stabilized fiber-coupled broadband light source, a broadband LED with continuous spectrum, superluminescent emitting diode, semiconductor light emitting diode, wide spectrum LED white light source, an multi-LED integrated white light source, among other broadband light sources or any combination thereof.

In other embodiments, methods include irradiating with a narrow band light source emitting a particular wavelength or a narrow range of wavelengths, such as for example with a light source which emits light in a narrow range of wavelengths like a range of 50 nm or less, such as 40 nm or less, such as 30 nm or less, such as 25 nm or less, such as 20 nm or less, such as 15 nm or less, such as 10 nm or less, such as 5 nm or less, such as 2 nm or less and including light sources which emit a specific wavelength of light (i.e., monochromatic light). Where methods include irradiating with a narrow band light source, narrow band light source protocols of interest may include, but are not limited to, a narrow wavelength LED, laser diode or a broadband light source coupled to one or more optical bandpass filters, diffraction gratings, monochromators or any combination thereof.

In certain embodiments, methods include irradiating the sample with one or more lasers. As discussed above, the type and number of lasers will vary depending on the sample as well as desired light collected and may be a gas laser, such as a helium-neon laser, argon laser, krypton laser, xenon laser, nitrogen laser, CO₂ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof. In other instances, the methods include irradiating the flow stream with a dye laser, such as a stilbene, coumarin or rhodamine laser. In yet other instances, methods include irradiating the flow stream with a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof. In still other instances, methods include irradiating the flow stream with a solid-state laser, such as a ruby laser, an Nd:YAG laser, NdCrYAG laser, Er:YAG laser, Nd:YLF laser, Nd:YVO₄ laser, Nd:yCa₄O(BO₃)₃ laser, Nd:YCOB laser, titanium sapphire laser, thulim YAG laser, ytterbium YAG laser, ytterbium₂O₃ laser or cerium doped lasers and combinations thereof.

The sample may be irradiated with one or more of the above-mentioned light sources, such as 2 or more light sources, such as 3 or more light sources, such as 4 or more light sources, such as 5 or more light sources and including 10 or more light sources. The light source may include any combination of types of light sources. For example, in some embodiments, the methods include irradiating the sample in the flow stream with an array of lasers, such as an array having one or more gas lasers, one or more dye lasers and one or more solid-state lasers.

The sample may be irradiated with wavelengths ranging from 200 nm to 1500 nm, such as from 250 nm to 1250 nm, such as from 300 nm to 1000 nm, such as from 350 nm to 900 nm and including from 400 nm to 800 nm. For example, where the light source is a broadband light source, the sample may be irradiated with wavelengths from 200 nm to 900 nm. In other instances, where the light source includes a plurality of narrow band light sources, the sample may be irradiated with specific wavelengths in the range from 200 nm to 900 nm. For example, the light source may be plurality of narrow band LEDs (1 nm - 25 nm) each independently emitting light having a range of wavelengths between 200 nm to 900 nm. In other embodiments, the narrow band light source includes one or more lasers (such as a laser array) and the sample is irradiated with specific wavelengths ranging from 200 nm to 700 nm, such as with a laser array having gas lasers, excimer lasers, dye lasers, metal vapor lasers and solid-state laser as described above.

Where more than one light source is employed, the sample may be irradiated with the light sources simultaneously or sequentially, or a combination thereof. For example, the sample may be simultaneously irradiated with each of the light sources. In other embodiments, the flow stream is sequentially irradiated with each of the light sources. Where more than one light source is employed to irradiate the sample sequentially, the time each light source irradiates the sample may independently be 0.001 microseconds or more, such as 0.01 microseconds or more, such as 0.1 microseconds or more, such as 1 microsecond or more, such as 5 microseconds or more, such as 10 microseconds or more, such as 30 microseconds or more and including 60 microseconds or more. For example, methods may include irradiating the sample with the light source (e.g. laser) for a duration which ranges from 0.001 microseconds to 100 microseconds, such as from 0.01 microseconds to 75 microseconds, such as from 0.1 microseconds to 50 microseconds, such as from 1 microsecond to 25 microseconds and including from 5 microseconds to 10 microseconds. In embodiments where sample is sequentially irradiated with two or more light sources, the duration sample is irradiated by each light source may be the same or different.

The time period between irradiation by each light source may also vary, as desired, being separated independently by a delay of 0.001 microseconds or more, such as 0.01 microseconds or more, such as 0.1 microseconds or more, such as 1 microsecond or more, such as 5 microseconds or more, such as by 10 microseconds or more, such as by 15 microseconds or more, such as by 30 microseconds or more and including by 60 microseconds or more. For example, the time period between irradiation by each light source may range from 0.001 microseconds to 60 microseconds, such as from 0.01 microseconds to 50 microseconds, such as from 0.1 microseconds to 35 microseconds, such as from 1 microsecond to 25 microseconds and including from 5 microseconds to 10 microseconds. In certain embodiments, the time period between irradiation by each light source is 10 microseconds. In embodiments where sample is sequentially irradiated by more than two (i.e., 3 or more) light sources, the delay between irradiation by each light source may be the same or different.

The sample may be irradiated continuously or in discrete intervals. In some instances, methods include irradiating the sample in the sample with the light source continuously. In other instances, the sample in is irradiated with the light source in discrete intervals, such as irradiating every 0.001 millisecond, every 0.01 millisecond, every 0.1 millisecond, every 1 millisecond, every 10 milliseconds, every 100 milliseconds and including every 1000 milliseconds, or some other interval.

Depending on the light source, the sample may be irradiated from a distance which varies such as 0.01 mm or more, such as 0.05 mm or more, such as 0.1 mm or more, such as 0.5 mm or more, such as 1 mm or more, such as 2.5 mm or more, such as 5 mm or more, such as 10 mm or more, such as 15 mm or more, such as 25 mm or more and including 50 mm or more. Also, the angle or irradiation may also vary, ranging from 10° to 90°, such as from 15° to 85°, such as from 20° to 80°, such as from 25° to 75° and including from 30° to 60°, for example at a 90° angle.

In embodiments, methods include irradiating the cell sample with frequency-modulated beams of light. In some instances, methods include irradiating the sample with two or more beams of frequency shifted light. As described above, a light beam generator component may be employed having a laser and an acousto-optic device for frequency shifting the laser light. In these embodiments, methods include irradiating the acousto-optic device with the laser. Depending on the desired wavelengths of light produced in the output laser beam (e.g., for use in irradiating a sample in a flow stream), the laser may have a specific wavelength that varies from 200 nm to 1500 nm, such as from 250 nm to 1250 nm, such as from 300 nm to 1000 nm, such as from 350 nm to 900 nm and including from 400 nm to 800 nm. The acousto-optic device may be irradiated with one or more lasers, such as 2 or more lasers, such as 3 or more lasers, such as 4 or more lasers, such as 5 or more lasers and including 10 or more lasers. The lasers may include any combination of types of lasers. For example, in some embodiments, the methods include irradiating the acousto-optic device with an array of lasers, such as an array having one or more gas lasers, one or more dye lasers and one or more solid-state lasers.

Where more than one laser is employed, the acousto-optic device may be irradiated with the lasers simultaneously or sequentially, or a combination thereof. For example, the acousto-optic device may be simultaneously irradiated with each of the lasers. In other embodiments, the acousto-optic device is sequentially irradiated with each of the lasers. Where more than one laser is employed to irradiate the acousto-optic device sequentially, the time each laser irradiates the acousto-optic device may independently be 0.001 microseconds or more, such as 0.01 microseconds or more, such as 0.1 microseconds or more, such as 1 microsecond or more, such as 5 microseconds or more, such as 10 microseconds or more, such as 30 microseconds or more and including 60 microseconds or more. For example, methods may include irradiating the acousto-optic device with the laser for a duration which ranges from 0.001 microseconds to 100 microseconds, such as from 0.01 microseconds to 75 microseconds, such as from 0.1 microseconds to 50 microseconds, such as from 1 microsecond to 25 microseconds and including from 5 microseconds to 10 microseconds. In embodiments where the acousto-optic device is sequentially irradiated with two or more lasers, the duration the acousto-optic device is irradiated by each laser may be the same or different.

The time period between irradiation by each laser may also vary, as desired, being separated independently by a delay of 0.001 microseconds or more, such as 0.01 microseconds or more, such as 0.1 microseconds or more, such as 1 microsecond or more, such as 5 microseconds or more, such as by 10 microseconds or more, such as by 15 microseconds or more, such as by 30 microseconds or more and including by 60 microseconds or more. For example, the time period between irradiation by each light source may range from 0.001 microseconds to 60 microseconds, such as from 0.01 microseconds to 50 microseconds, such as from 0.1 microseconds to 35 microseconds, such as from 1 microsecond to 25 microseconds and including from 5 microseconds to 10 microseconds. In certain embodiments, the time period between irradiation by each laser is 10 microseconds. In embodiments where the acousto-optic device is sequentially irradiated by more than two (i.e., 3 or more) lasers, the delay between irradiation by each laser may be the same or different.

The acousto-optic device may be irradiated continuously or in discrete intervals. In some instances, methods include irradiating the acousto-optic device with the laser continuously. In other instances, the acousto-optic device is irradiated with the laser in discrete intervals, such as irradiating every 0.001 millisecond, every 0.01 millisecond, every 0.1 millisecond, every 1 millisecond, every 10 milliseconds, every 100 milliseconds and including every 1000 milliseconds, or some other interval.

Depending on the laser, the acousto-optic device may be irradiated from a distance which varies such as 0.01 mm or more, such as 0.05 mm or more, such as 0.1 mm or more, such as 0.5 mm or more, such as 1 mm or more, such as 2.5 mm or more, such as 5 mm or more, such as 10 mm or more, such as 15 mm or more, such as 25 mm or more and including 50 mm or more. Also, the angle or irradiation may also vary, ranging from 10° to 90°, such as from 15° to 85°, such as from 20° to 80°, such as from 25° to 75° and including from 30° to 60°, for example at a 90° angle.

In embodiments, methods include applying radiofrequency drive signals to the acousto-optic device to generate angularly deflected laser beams. Two or more radiofrequency drive signals may be applied to the acousto-optic device to generate an output laser beam with the desired number of angularly deflected laser beams, such as 3 or more radiofrequency drive signals, such as 4 or more radiofrequency drive signals, such as 5 or more radiofrequency drive signals, such as 6 or more radiofrequency drive signals, such as 7 or more radiofrequency drive signals, such as 8 or more radiofrequency drive signals, such as 9 or more radiofrequency drive signals, such as 10 or more radiofrequency drive signals, such as 15 or more radiofrequency drive signals, such as 25 or more radiofrequency drive signals, such as 50 or more radiofrequency drive signals and including 100 or more radiofrequency drive signals.

The angularly deflected laser beams produced by the radiofrequency drive signals each have an intensity based on the amplitude of the applied radiofrequency drive signal. In some embodiments, methods include applying radiofrequency drive signals having amplitudes sufficient to produce angularly deflected laser beams with a desired intensity. In some instances, each applied radiofrequency drive signal independently has an amplitude from about 0.001 V to about 500 V, such as from about 0.005 V to about 400 V, such as from about 0.01 V to about 300 V, such as from about 0.05 V to about 200 V, such as from about 0.1 V to about 100 V, such as from about 0.5 V to about 75 V, such as from about 1 V to 50 V, such as from about 2 V to 40 V, such as from 3 V to about 30 V and including from about 5 V to about 25 V. Each applied radiofrequency drive signal has, in some embodiments, a frequency of from about 0.001 MHz to about 500 MHz, such as from about 0.005 MHz to about 400 MHz, such as from about 0.01 MHz to about 300 MHz, such as from about 0.05 MHz to about 200 MHz, such as from about 0.1 MHz to about 100 MHz, such as from about 0.5 MHz to about 90 MHz, such as from about 1 MHz to about 75 MHz, such as from about 2 MHz to about 70 MHz, such as from about 3 MHz to about 65 MHz, such as from about 4 MHz to about 60 MHz and including from about 5 MHz to about 50 MHz.

In these embodiments, the angularly deflected laser beams in the output laser beam are spatially separated. Depending on the applied radiofrequency drive signals and desired irradiation profile of the output laser beam, the angularly deflected laser beams may be separated by 0.001 µm or more, such as by 0.005 µm or more, such as by 0.01 µm or more, such as by 0.05 µm or more, such as by 0.1 µm or more, such as by 0.5 µm or more, such as by 1 µm or more, such as by 5 µm or more, such as by 10 µm or more, such as by 100 µm or more, such as by 500 µm or more, such as by 1000 µm or more and including by 5000 µm or more. In some embodiments, the angularly deflected laser beams overlap, such as with an adjacent angularly deflected laser beam along a horizontal axis of the output laser beam. The overlap between adjacent angularly deflected laser beams (such as overlap of beam spots) may be an overlap of 0.001 µm or more, such as an overlap of 0.005 µm or more, such as an overlap of 0.01 µm or more, such as an overlap of 0.05 µm or more, such as an overlap of 0.1 µm or more, such as an overlap of 0.5 µm or more, such as an overlap of 1 µm or more, such as an overlap of 5 µm or more, such as an overlap of 10 µm or more and including an overlap of 100 µm or more.

In certain instances, the sample is irradiated in the flow stream with a plurality of beams of frequency-shifted light and images of the cell mitochondria in the flow stream are generated such as described in Diebold, et al. Nature Photonics Vol. 7(10); 806-810 (2013), as well as described in U.S. Patent Nos. 9,423,353; 9,784,661; 9,983,132; 10,006,852; 10,078,045; 10,036,699; 10,222,316; 10,288,546; 10,324,019; 10,408,758; 10,451,538; 10,620,111; and U.S. Patent Publication Nos. 2017/0133857; 2017/0328826; 2017/0350803; 2018/0275042; 2019/0376895 and 2019/0376894.

Light from irradiated cells in the sample is conveyed to a light detection system as described in greater detail below and measured by the plurality of photodetectors. In some embodiments, methods include measuring the collected light over a range of wavelengths (e.g., 200 nm - 1000 nm). For example, methods may include collecting spectra of light over one or more of the wavelength ranges of 200 nm - 1000 nm. In yet other embodiments, methods include measuring collected light at one or more specific wavelengths. For example, the collected light may be measured at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof.

The collected light may be measured continuously or in discrete intervals. In some instances, methods include taking measurements of the light continuously. In other instances, the light is measured in discrete intervals, such as measuring light every 0.001 millisecond, every 0.01 millisecond, every 0.1 millisecond, every 1 millisecond, every 10 milliseconds, every 100 milliseconds and including every 1000 milliseconds, or some other interval.

Measurements of the collected light may be taken one or more times during the subject methods, such as 2 or more times, such as 3 or more times, such as 5 or more times and including 10 or more times. In certain embodiments, light from the sample is measured 2 or more times, with the data in certain instances being averaged.

Light from the cells in the sample may be measured at one or more wavelengths of, such as at 5 or more different wavelengths, such as at 10 or more different wavelengths, such as at 25 or more different wavelengths, such as at 50 or more different wavelengths, such as at 100 or more different wavelengths, such as at 200 or more different wavelengths, such as at 300 or more different wavelengths and including measuring the collected light at 400 or more different wavelengths.

In some embodiments, methods include further adjusting the light from the sample before detecting the light. For example, the light from the sample source may be passed through one or more lenses, mirrors, pinholes, slits, gratings, light refractors, and any combination thereof. In some instances, the collected light is passed through one or more focusing lenses, such as to reduce the profile of the light. In other instances, the emitted light from the sample is passed through one or more collimators to reduce light beam divergence.

In embodiments, image data is generated from the measured light. In some instances, the image data includes data waveforms generated in one or more photodetector channels. In some embodiments, one or more images of the cells in the sample are generated. The image may be generated from detected fluorescence, detected light absorption, detected light scatter, detected light emission or any combination thereof. In some instances, the image is generated from fluorescence from labelled mitochondria. In some instances, the image is generated from light absorption detected from the sample, such as from a brightfield light detector. In these instances, the image is generated based on brightfield image data from the particle in the flow stream. In other instances, the image is generated from light scatter detected from the sample, such as from a side scatter detector, a forward scatter detector or a combination of a side scatter detector and forward scatter detector. In these instances, the image is generated based on scattered light image data. In yet other instances, the image is generated from emitted light from the sample. In still other instances, the image is generated from a combination of detected light absorption, detected light scatter and detected light emission. In certain instances, images of cell mitochondria are generated from fluorescence from the labelled mitochondria and one or more light loss, side-scattered light and forward scattered light.

One or more images may be generated from the measured light. In some embodiments, a single image is generated for each cell in the sample from each form of detected light. In other embodiments, a plurality of images are generated for each cell, such as 2 or more, such as 3 or more, such as 5 or more, such as 10 or more and including 25 or more images for each cell. For example, a first image of the cell is generated from fluorescence; a second image of the cell is generated from detected light absorption and a third image is generated from detected light scatter. In other embodiments, two or more images are generated from each form of detected light, such as 3 or more, such as 4 or more, such as 5 or more and including 10 or more images or a combination thereof.

In certain embodiments, frequency-encoded data (e.g., frequency-encoded spatial data) is generated from the measured light from cells that are fluorescently-labelled in the flow stream. In some instances, one or more images are generated from the frequency-encoded data. The frequency-encoded data may be generated in one or more detection channels, such as 2 or more, such as 3 or more, such as 4 or more, such as 5 or more, such as 6 or more and including 8 or more detection channels. In some embodiments, the frequency-encoded data includes data components taken (or derived) from light from different detectors, such as from fluorescence detection channels, detected light absorption or detected light scatter. In some instances, the frequency-encoded data is phase-corrected. In some instances, the frequency-encoded data used to generate images of the cells is phase corrected by performing a transform on the frequency-encoded data. In one example, the frequency-encoded data is phase corrected by performing a Fourier transform (FT) of the frequency-encoded data. In another example, the frequency-encoded is phase corrected by performing a discrete Fourier transform (DFT) of the frequency-encoded data. In yet another example, the frequency-encoded data is phase corrected by performing a short time Fourier transform (STFT) of the frequency-encoded data. In certain embodiments, methods include performing a transform of the frequency encoded data without performing any mathematical imaginary computations (i.e., only performing computations for mathematical real computations of the transform) to generate an image from the frequency-encoded data.

In some instances, methods include generating one or more greyscale images of the cells. The term "greyscale" is used herein in its conventional sense to refer to images of the cells in the flow stream that are composed of varying shades of gray that are based on the intensity of light at each pixel. In some embodiments, a pixel intensity threshold is determined from the greyscale image where the pixel intensity threshold value is used to convert each pixel into a binary value that is used to generate the image of the cell. In certain instances, the image of the cells is a binary pixel image of the cell such as where each pixel is assigned a binary pixel value of 1 (e.g., where the intensity of the pixel exceeds a predetermined threshold) or a binary pixel value of 0 (e.g., where the intensity of the pixel falls below a predetermined threshold).

Two or more image parameters are calculated from the generated images of the cells. In some instances, a center of mass image parameter is calculated from the generated image. In some instances, a delta center of mass image parameter is calculated from the generated image. In some instances, a diffusivity image parameter is calculated from the generated image. According to the invention, an eccentricity image parameter is calculated from the generated image. In some instances, a long axis moment image parameter is calculated from the generated image. In some instances, a maximum intensity image parameter is calculated from the generated image. According to the invention, a radial moment image parameter is calculated from the generated image. In some instances, a short axis moment image parameter is calculated from the generated image. In some instances, a particle size image parameter is calculated from the generated image. In some instances, a total intensity image parameter is calculated from the generated image. In some instances, a particle light loss image parameter is calculated from the generated image. In some instances, a forward scattered light image parameter is calculated from the generated image. In some instances, a side scattered light image parameter is calculated from the generated image. In some instances, an image moment is calculated from the generated image. The term "image moment" is used herein in its conventional sense to refer to a weighted average of pixel intensities in an image. In some instances, the center of mass may be calculated from the image moment of the image. In other instances, the orientation of the cell may be calculated from the image moment of the image. In still other instances, the eccentricity of the cell may be calculated from the image moment of the image.

In certain embodiments, imaging parameters calculated from the generated image for use in generating a gating strategy (as described below) summarized in Table 1.

**Table 1**

| | Imaging parameters |
|---|---|
| 1 | FSC-A :: FSC-A |
| 2 | FSC-H :: FSC-H |
| 3 | FSC-T :: FSC-T |
| 4 | FSC-W :: FSC-W |
| 5 | SSC (Imaging)-A :: SSC (Imaging)-A |
| 6 | SSC (Imaging)-H :: SSC (Imaging)-H |
| 7 | SSC (Imaging)-T :: SSC (Imaging)-T |
| 8 | SSC (Imaging)-W :: SSC (Imaging)-W |
| 9 | SSC (Violet)-A :: SSC (Violet)-A |
| 10 | SSC (Violet)-H :: SSC (Violet)-H |
| 11 | SSC (Violet)-T :: SSC (Violet)-T |
| 12 | SSC (Violet)-W :: SSC (Violet)-W |
| 13 | Center of Mass (X) (FSC) |
| 14 | Center of Mass (X) (LightLoss (Imaging)) |
| 15 | Center of Mass (X) (SSC (Imaging)) |
| 16 | Center of Mass (Y) (FSC) |
| 17 | Center of Mass (Y) (LightLoss (Imaging)) |
| 18 | Center of Mass (Y) (SSC (Imaging)) |
| 19 | Delta CoM (LightLoss (Imaging)_FSC) |
| 20 | Delta CoM (SSC (Imaging)_FSC) |
| 21 | Delta CoM (SSC (Imaging)_LightLoss (Imaging)) |
| 22 | Diffusivity (FSC) |
| 23 | Diffusivity (LightLoss (Imaging)) |
| 24 | Diffusivity (SSC (Imaging)) |
| 25 | Eccentricity (FSC) |
| 26 | Eccentricity (LightLoss (Imaging)) |
| 27 | Eccentricity (SSC (Imaging)) |
| 28 | LightLoss (Imaging)-A :: LightLoss (Imaging)-A |
| 29 | LightLoss (Imaging)-H :: LightLoss (Imaging)-H |
| 30 | LightLoss (Imaging)-T :: LightLoss (Imaging)-T |
| 31 | LightLoss (Imaging)-W :: LightLoss (Imaging)-W |
| 32 | LightLoss (Violet)-A :: LightLoss (Violet)-A |
| 33 | LightLoss (Violet)-H :: LightLoss (Violet)-H |
| 34 | LightLoss (Violet)-T :: LightLoss (Violet)-T |
| 35 | LightLoss (Violet)-W :: LightLoss (Violet)-W |
| 36 | Long Axis Moment (FSC) |
| 37 | Long Axis Moment (LightLoss (Imaging)) |
| 38 | Long Axis Moment (SSC (Imaging)) |
| 39 | Max Intensity (FSC) |
| 40 | Max Intensity (LightLoss (Imaging)) |
| 41 | Max Intensity (SSC (Imaging)) |
| 42 | Radial Moment (FSC) |
| 43 | Radial Moment (LightLoss (Imaging)) |
| 44 | Radial Moment SSC (Imaging)) |
| 45 | Short Axis Moment (FSC) |
| 46 | Short Axis Moment (LightLoss (Imaging)) |
| 47 | Short Axis Moment (SSC (Imaging)) |
| 48 | Size (FSC) |
| 49 | Size (LightLoss (Imaging)) |
| 50 | Size (SSC (Imaging)) |
| 51 | Total Intensity (FSC) |
| 52 | Total Intensity (LightLoss (Imaging)) |
| 53 | Total Intensity (SSC (Imaging)) |

In some embodiments, methods include contacting the cells with one or more fluorophores to fluorescently label the cells. The term "labelling" is used herein in its conventional sense to refer to the coupling to or conjugation of one or more components of the cells of the sample to one or more detection components, such as components that are detectable by luminescence (e.g., fluorescence, phosphorescence, etc.), contrast agents, radioisotopes, among others. For example, labelling may include coupling the particles to the detection component by non-covalent interactions such as hydrogen bonding, dipole-dipole bonding, ionic bonding or through one or more covalent bonds.

In certain instances, fluorochromes of interest may include, but are not limited to, a bodipy dye, a coumarin dye, a rhodamine dye, an acridine dye, an anthraquinone dye, an arylmethane dye, a diarylmethane dye, a chlorophyll containing dye, a triarylmethane dye, an azo dye, a diazonium dye, a nitro dye, a nitroso dye, a phthalocyanine dye, a cyanine dye, an asymmetric cyanine dye, a quinon-imine dye, an azine dye, an eurhodin dye, a safranin dye, an indamin, an indophenol dye, a fluorine dye, an oxazine dye, an oxazone dye, a thiazine dye, a thiazole dye, a xanthene dye, a fluorene dye, a pyronin dye, a fluorine dye, a rhodamine dye, a phenanthridine dye, squaraines, bodipys, squarine roxitanes, naphthalenes, coumarins, oxadiazoles, anthracenes, pyrenes, acridines, arylmethines, or tetrapyrroles and a combination thereof. In certain embodiments, conjugates may include two or more dyes, such as two or more dyes selected from a bodipy dye, a coumarin dye, a rhodamine dye, an acridine dye, an anthraquinone dye, an arylmethane dye, a diarylmethane dye, a chlorophyll containing dye, a triarylmethane dye, an azo dye, a diazonium dye, a nitro dye, a nitroso dye, a phthalocyanine dye, a cyanine dye, an asymmetric cyanine dye, a quinon-imine dye, an azine dye, an eurhodin dye, a safranin dye, an indamin, an indophenol dye, a fluorine dye, an oxazine dye, an oxazone dye, a thiazine dye, a thiazole dye, a xanthene dye, a fluorene dye, a pyronin dye, a fluorine dye, a rhodamine dye, a phenanthridine dye, squaraines, bodipys, squarine roxitanes, naphthalenes, coumarins, oxadiazoles, anthracenes, pyrenes, acridines, arylmethines, or tetrapyrroles and a combination thereof.

In certain embodiments, fluorochromes of interest may include but are not limited to fluorescein isothiocyanate (FITC), a phycoerythrin (PE) dye, a peridinin chlorophyll protein-cyanine dye (e.g., PerCP-Cy5.5), a phycoerythrin-cyanine (PE-Cy) dye (PE-Cy7), an allophycocyanin (APC) dye (e.g., APC-R700), an allophycocyanin-cyanine dye (e.g., APC-Cy7), a coumarin dye (e.g., V450 or V500). In certain instances, fluorochromes may include one or more of 1,4-bis-(o-methylstyryl)-benzene (bis-MSB 1,4-bis[2-(2-methylphenyl)ethenyl]-benzene), a C510 dye, a C6 dye, nile red dye, a T614 dye (e.g., N-[7-(methanesulfonamido)-4-oxo-6-phenoxychromen-3-yl]formamide), LDS 821 dye ((2-(6-(p-dimethylaminophenyl)-2,4-neopentylene-1,3,5-hexatrienyl)-3-ethylbenzothiazolium perchlorate), an mFluor dye (e.g., an mFluor Red dye such as mFluor 780NS).

Fluorochromes of interest may include, but are not limited to, Fluorescein, Hydroxycoumarin, Aminocoumarin, Methoxycoumarin, Cascade Blue, Pacific Blue, Pacific Orange, Lucifer yellow, NBD, R-Phycoerythrin (PE), PE-Cy5 conjugates, PE-Cy7 conjugates, Red 613, PerCP, TruRed, FluorX, BODIPY-FL, TRITC, X-Rhodamine, Lissamine Rhodamine B, Texas Red, Allophycocyanin (APC), APC-Cy7 conjugates, Cy2, Cy3, Cy3B, Cy3.5, Cy5, Cy5.5, Cy7, Hoechst 33342, DAPI, Hoechst 33258, SYTOX Blue, Chromomycin A3, Mithramycin, YOYO-1, Ethidium Bromide, Acridine Orange, SYTOX Green, TOTO-1, TO-PRO-1, Thiazole Orange, Propidium Iodide (PI), LDS 751, 7-AAD, SYTOX Orange, TOTO-3, TO-PRO-3, DRAQ5, Indo-1, Fluo-3, DCFH, DHR, SNARF, Y66H, Y66F, EBFP, EBFP2, Azurite, GFPuv, T-Sapphire, TagBFP, Cerulean, mCFP, ECFP, CyPet, Y66W, dKeima-Red, mKeima-Red, TagCFP, AmCyan1, mTFP1 (Teal), S65A, Midoriishi-Cyan, Wild Type GFP, S65C, TurboGFP, TagGFP, TagGFP2, AcGFP1, S65L, Emerald, S65T, EGFP, Azami-Green, ZsGreen1, Dronpa-Green, TagYFP, EYFP, Topaz, Venus, mCitrine, YPet, TurboYFP, PhiYFP, PhiYFP-m, ZsYellow1, mBanana, Kusabira-Orange, mOrange, mOrange2, mKO, TurboRFP, tdTomato, DsRed-Express2, TagRFP, DsRed monomer, DsRed2 ("RFP"), mStrawberry, TurboFP602, AsRed2, mRFP1, J-Red, mCherry, HcRed1, mKate2, Katushka (TurboFP635), mKate (TagFP635), TurboFP635, mPlum, mRaspberry, mNeptune, E2-Crimson, Monochlorobimane, Calcein, Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, Alexa Fluor 750, Alexa Fluor 790, and HyPer, or the like. In some embodiments, the fluorochromes are selected from: 7-AAD, Alexa Fluor 488, Alexa Fluor 647, Alexa Fluor 700, AmCyan, APC, APC-Cy7, APC-H7, APC-R700, BB660-P2, BB790-P, BUV395, BUV615, BUV661, BV570, BV605, BV650, BV711, BV750, BV786, BYG584-P, Calcein AM, Calcein Blue AM, CFSE, DAPI, DRAQ5, DRAQ7, FITC, Fluo-4 AM, FVS440UV, FVS450, FVS510, FVS520, FVS570, FVS575V, FVS620, FVS660, FVS700, FVS780, Indo-1 Hi, Indo-1 Lo, JC-1, MitoStatus Red, MitoStatus TMRE, Pacific Blue, PE, PE-CF594, PE-Cy5, PE-Cy7, PerCP, PerCP-Cy5.5, PI, R718, RB545, RB613, RB744, RB780, RY586, RY610, V450, V500, Via-Probe Green, Via-Probe Red, VPD450, Alexa Fluor 532, Alexa Fluor 561, Alexa Fluor 660, APC-eFluor 780, APC/Fire 750, APC/Fire 810, BV785, eBFP, eCFP, eFluor 450, eFluor 506, eFluor 660, eGFP, eYFP, Hoechst 33258, KIRAVIA Blue 520, mCherry, NFB510, NFB530, NFB555, NFB585, NFB610-70S, NFB660-120S, NFR660, NFR685, NFR700, NFR710, NFY570, NFY590, NFY610, NFY660, NFY690, NFY700, NFY730, Pacific Orange, PE-Cy5.5, PE-eFluor 610, PE/Dazzle 594, PE/Fire 640, PE/Fire 700, PE/Fire 810, PerCP-eFluor 710, SB436, SB600, SB645, SB702, SB780, Spark Blue 550, Spark Blue 574, Spark NIR 685, Spark UV 387, Spark Violet 423, Spark Violet 538, Spark YG 581, Spark YG 593, and tdTomato.

In some instances, the fluorochrome is a polymeric dye (e.g., fluorescent polymeric dyes). Fluorescent polymeric dyes that find use in the subject methods and systems are varied. In some instances of the method, the polymeric dye includes a conjugated polymer. Conjugated polymers (CPs) are characterized by a delocalized electronic structure which includes a backbone of alternating unsaturated bonds (e.g., double and/or triple bonds) and saturated (e.g., single bonds) bonds, where π-electrons can move from one bond to the other. As such, the conjugated backbone may impart an extended linear structure on the polymeric dye, with limited bond angles between repeat units of the polymer. For example, proteins and nucleic acids, although also polymeric, in some cases do not form extended-rod structures but rather fold into higher-order three-dimensional shapes. In addition, CPs may form "rigid-rod" polymer backbones and experience a limited twist (e.g., torsion) angle between monomer repeat units along the polymer backbone chain. In some instances, the polymeric dye includes a CP that has a rigid rod structure. The structural characteristics of the polymeric dyes can have an effect on the fluorescence properties of the molecules.

Polymeric dyes of interest include, but are not limited to, those dyes described in U.S. Patent Nos. 7,270,956; 7,629,448; 8,158,444; 8,227,187; 8,455,613; 8,575,303; 8,802,450; 8,969,509; 9,139,869; 9,371,559; 9,547,008; 10,094,838; 10,302,648; 10,458,989; 10,641,775 and 10,962,546 **;** and Gaylord et al., J. Am. Chem. Soc., 2001, 123 (26), pp 6417-6418; Feng et al., Chem. Soc. Rev., 2010,39, 2411-2419; and Traina et al., J. Am. Chem. Soc., 2011, 133 (32), pp 12600-12607. Specific polymeric dyes that may be employed include, but are not limited to, BD Horizon Brilliant^{™} Dyes, such as BD Horizon Brilliant^{™} Violet Dyes (e.g., BV421, BV510, BV605, BV650, BV711, BV786); BD Horizon Brilliant^{™} Ultraviolet Dyes (e.g., BUV395, BUV496, BUV737, BUV805); and BD Horizon Brilliant^{™} Blue Dyes (e.g., BB515) (BD Biosciences, San Jose, CA). Any fluorochromes that are known to a skilled artisan-including, but not limited to, those described above-or are yet to be discovered may be employed in the subject methods.

Calculating a cell parameter according to the present disclosure includes assessing a cell interaction parameter. The term "interaction" is used herein in its conventional sense to refer to physical or functional coupling of two or more cells. In some instances, the cells are in physical contact with each other. In some instances, the cells are co-localized with each other in a generated image, such as where the cells are positioned within 1 µm or less of each other, such as 0.5 µm or less, such as 0.1 µm or less, such as 0.05 µm or less, such as 0.01 µm or less, such as 0.005 µm or less, such as 0.001 µm or less and including within 0.0001 µm or less of each other. In some instances, one or more cells are internalized within a cell.

In some instances, the cell interaction is active interaction between the cells, such as where the two or more cells are in active communication with each other (e.g., through an active synapse). In other instances, the cell interaction is a passive interaction between the cells, such as where the two or more cells are in passive communication with each other (e.g., through a passive synapse). In other instances, the cell interaction is a coincident doublet in the generated image of the cells. In other instances, the cell interaction is an engaged doublet in the generated image of the cells. In some instances, calculating the cell interaction parameter includes assessing internalization of a cell or cellular component by one or more cells in the sample. In some instances, calculating the cell interaction parameter includes assessing co-localization between two or more cells in the sample. In some instances, calculating a cell parameter includes assessing a phagocytosis cell interaction.

In certain instances, calculating a cell parameter includes assessing a tumor cell killing interaction, such as where one of the cells is a cancer cell and the interacting cell is a lymphocyte. In some instances, assessing the tumor cell killing interaction includes a heterogenous doublet lymphocyte and cancer cell. In some instances, assessing the tumor cell killing interaction includes a homogeneous doublet lymphocyte and lymphocyte.

In some embodiments, calculating a cell parameter includes assessing a translocation parameter of a cellular component within the cells of the sample. In some instances, calculating the cell parameter includes determining the displacement of a cellular component between different organelles within the cell. In some instances, the cell parameter characterizes the type of component being translocated within the cells. For example, in some instances calculating a cell parameter includes assessing protein translocation within the cell. In certain instances, methods include assessing transcription factor translocation. In some instances, calculating a cell parameter includes assessing lipid translocation within the cell. In some instances, calculating a cell parameter includes assessing polysaccharide translocation within the cell. In some instances, calculating a cell parameter includes assessing nucleic acid translocation within the cell. In certain instances, calculating a cell parameter includes assessing translocation of a cell signaling compound. In other instances, calculating a cell parameter includes assessing translocation of a cell growth factor within the cell. In certain embodiments, calculating a cell parameter includes measuring nuclear translocation, such as where a cellular component (e.g., NF-κβ transcription factor) is moved from the cytoplasm to the nucleus. In some instances, calculating a cell parameter includes measuring translocation of a component from the nucleus to the cytoplasm. In some instances, calculating a cell parameter includes measuring translocation of a component from the cytoplasm to the mitochondria. In some instances, calculating a cell parameter includes measuring translocation of a component from the mitochondria to the cytoplasm.

In some embodiments, the cell parameter is a cell mitotic phase parameter. In some instances, calculating the cell parameter includes determining the mitotic phase of one or more cells in the sample. In some instances, methods include identifying a cell as being in interphase. In some instances, methods include identifying a cell as being in prophase. In some instances, methods include identifying a cell as being in metaphase. In some instances, methods include identifying a cell as being in anaphase. In some instances, methods include identifying a cell as being in telophase. In certain instances, methods include identifying a cell as being in cytokinesis. In certain instances, the mitotic phase parameter is assessed from generated images of the cell mitochondria. In some instances, the mitotic phase parameter is assessed using image parameters calculated from the generated images of the mitochondria.

In some instances, the morphology of the cell mitochondria is assessed to determine the mitotic phase of the cell. In some instances, the morphology of the cell mitochondria is determined based on a radial moment image parameter. In some instances, the image parameter includes the radial moment of the fluorophore used to stain the mitochondria. In some instances, the radial moment image parameter is calculated from images generated based on fluorescence measured from the cell mitochondria, light loss measured for the cell mitochondria, forward scattered light measured for the cell mitochondria, side scattered light measured for the cell mitochondria and combinations thereof.

In some embodiments, the mitochondria are labelled with one or more labels. In some embodiments, the mitochondria are labelled with one or more lipophilic cationic fluorophore. In some instances, the mitochondria are labelled with a fluorophore that forms a covalent bond with a mitochondrial protein. In some instances, the lipophilic cationic fluorophore has a thiol-reactive moiety for binding the fluorophore to the mitochondrial protein. In some instances, the mitochondria are labelled with a fluorophore that forms a covalent bond with a mitochondrial lipid. In some instances, the mitochondria are labelled with a fluorophore that stains the inner mitochondrial membrane. In certain embodiments, the mitochondria are labelled with a mitochondrial specific dye, such as a MitoTracker dye. In some instances, the mitochondria are labelled with a dye that is or includes a dye component of: MitoTracker Green FM dye, MitoTracker Red FM dye, MitoTracker DeepRed dye, MitoTracker Orange dye, MitoTracker Red dye, MitoTracker DeepRed FM dye, MitoTracker Red CMXRos dye or a combination thereof. In some embodiments, the mitochondria are labelled with a mitochondrial labelling dye that is or includes a dye component from one or more of: 10-N-nonyl-acridine orange, BrdU, CellLight Mitochondria-GFP, chomabodies, Cox8 Snap Tag, DiOC₆, IraZolve-Mito, JC-1, JC-9, mito-Dronpa, mito-PA-mCherryl, Mito-QC, MitoBlue, Mitorotor-1, MitoSOX, MitoTimer, Mt-Keima, PK Mito Orange (PKMO), rhodamine 123, SNARF-1 dye, SPLICS, styryl dyes DASPMI, terpyridyl cyclometalated Ir (III) complexes, tetramethylrhodamine methylester, mitochondrial fluorescent proteins (red, yellow, orange, green, blue and cyan), ZP1-halotag and combinations thereof.

In some instances, the mitotic phase parameter is calculated from the generated images of the cells. In some instances, a cell mitochondria size image parameter is calculated from the generated image. In some instances, a cell mitochondria fluorescence radial moment image parameter is calculated from the generated image. In some instances, a cell mitochondria light loss image parameter is calculated from the generated image. In some instances, a forward scattered light radial moment image parameter is calculated from the generated image. In some instances, a side scattered light radial moment image parameter is calculated from the generated image. In some instances, an image moment is calculated from the generated image. The term image moment is used herein in its conventional sense to refer to a weighted average of pixel intensities in an image. In some instances, the center of mass of the cell mitochondria may be calculated from the image moment of the image. In other instances, the orientation of the cell mitochondria may be calculated from the image moment of the image. In still other instances, the eccentricity of the cell mitochondria may be calculated from the image moment of the image.

In some embodiments, the calculated image parameter is a radial moment of the fluorophore. In some instances, methods include calculating pixel density of the generated image of the cell mitochondria. In some instances, the number of pixels in a horizontal row of the image of the cell mitochondria are calculated. In some instances, the number of pixels in a vertical column of the image of the cell mitochondria are calculated. In certain instances, the absolute number of pixels found in the image of cell mitochondria is determined. In some instances, the centroid of the cell mitochondria is determined from one or more of the generated images. In some instances, the image parameter is calculated by determining the sum of the pixels in the generated image weighted by the distance of each pixel from the centroid of the cell mitochondria.

In some embodiments, methods include determining a cell parameter for assessing plant cell biology. In some instances, calculating a cell parameter according to the present disclosure includes identifying that a cell in the sample is a plant cell. In some instances, calculating the cell parameter provides for distinguishing a plant cell from cellular debris in the sample. In some instances, methods include calculating a cell parameter to characterize plant cells of different sizes. In some instances, calculating the cell parameter includes identifying that the plant cell is a parenchyma cell. In some instances, calculating the cell parameter includes identifying that the plant cell is a collenchyma cell. In some instances, calculating the cell parameter includes identifying that the plant cell is a sclerenchyma cell. In some instances, calculating the cell parameter includes identifying that the plant cell is a xylem cell (e.g., tracheid). In some instances, calculating the cell parameter includes identifying that the plant cell is a phloem cell. In certain instances, calculating the cell parameter includes identifying that cells of the sample include protoplasts.

In some instances, calculating the cell parameter includes determining the developmental stage of the plant cell. In some instances, methods include determining that the plant cell is undergoing cell division. In some instances, methods include determining that the plant cell is undergoing cell elongation. In some instances, methods include determining that the plant cell is undergoing cell differentiation.

In some embodiments, calculating the cell parameter includes assessing the morphology of the plant cell. In some instances, assessing the morphology of the plan cell includes evaluating the size of the plant cell. In some instances, the size of each plant cell in the sample is measured. In some instances, methods for determining the size of each plant cell in the sample includes comparing the size of the plant cell with a predetermined reference size (using a reference image of plant cells of interest), such as where the size of each cell is determined to be 50% or more of the predetermined reference size, such as 60% or more, such as 70% or more, such as 80% or more, such as 90% or more, such as 95% or more, such as 97% or more, such as 98% or more, such as 99% or more of the predetermined reference size. In some instances, methods include determining the number or percentage of cells in the sample that have a size that is within 15% or less of a predetermined reference size range, such as within 10% or less, such as within 5% or less, such as within 4% or less, such as within 3% or less, such as within 2% or less, such as within 1% or less and including within 0.5% or less of a predetermined reference size range. In some instances, methods include determining that the sample is a plant cell sample when the cells in the sample have a size that is within a reference size range.

In some embodiments, assessing the morphology of the cells includes evaluating a degree of punctateness of the cells. In some instances, the punctateness of each cell in the sample is measured. In some instances, methods for determining the punctateness of each cell in the sample includes comparing the punctateness of the cell with a predetermined reference punctateness (using a reference image of the cell), such as where the punctateness of each cell is determined to be 50% or more of the predetermined reference size, such as 60% or more, such as 70% or more, such as 80% or more, such as 90% or more, such as 95% or more, such as 97% or more, such as 98% or more, such as 99% or more of the predetermined reference punctateness. In some instances, methods include determining the number or percentage of cell in the sample that have a punctateness that is within 15% or less of a predetermined reference size range, such as within 10% or less, such as within 5% or less, such as within 4% or less, such as within 3% or less, such as within 2% or less, such as within 1% or less and including within 0.5% or less of a predetermined reference size range. In some instances, methods include determining that the sample is a plant cell sample when the cells in the sample have a punctateness that is within a reference punctateness range.

In some embodiments, assessing the morphology of the plant cells includes evaluating the shape of the cells. In some instances, methods include determining the percentage of cells having a particular shape. In certain instances depending on the type of cell sample and desired shape of the cells, methods include determining that cells of the sample are plant cells when the percentage of cells having a particular desired shape exceeds a predetermined threshold, such as where 50% or more of the cells in the sample have a desired shape such as 60% or more, such as 70% or more, such as 80% or more, such as 90% or more, such as 95% or more, such as 97% or more, such as 98% or more, such as 99% or more and including where 99.5% or more of the cells in the sample have a particular desired shape.

In some embodiments, calculating a cell parameter includes determining that a cell belongs to a rare cell population. The term rare cell is used herein in its conventional sense to refer to cells in the sample in which the abundance of the type of cell is 0.1 % of the cell population or less, such as 0.05% of the cell population or less, such as 0.01% of the cell population or less, such as 0.005% of the cell population or less, such as 0.001% of the cell population or less, such as 0.0005% of the cell population or less and including where the type of cell is 0.0001% of the cell population or less. In some instances, calculating a cell parameter includes determining that the rare cell is a stem cell. In some instances, calculating a cell parameter includes determining that the rare cell is a circulating endothelial cell. In some instances, calculating a cell parameter includes determining that the rare cell is a circulating tumor (malignant or benign) cell. In some instances, calculating a cell parameter includes determining that the rare cell is a residual disease cell, such as a disease of the blood or bone marrow.

In some embodiments, methods include classifying the cells of the sample based on the image data (e.g., waveforms generated in each photodetector channel), generated images of the cell, a calculated image parameter or a combination thereof. In some instances, classifying the cells includes assigning the cells to one or more particle population clusters. In some embodiments, cells of the sample are classified by a machine learning algorithm that uses reference images of the cells or one or more reference image parameters as training data sets. In some instances, the machine learning algorithm is a dynamic algorithm that updates based on generated images and determined image parameters of the cells. Any convenient machine learning algorithm can be implemented where machine learning algorithms of interest can include, but are not limited to a linear regression algorithm, a logistic regression algorithm, a Naïve Bayes algorithm, a k-nearest neighbor (kNN) algorithm, a Random forest algorithm, decision tree algorithm, a support vector machine algorithm, a gradient boosting algorithm and a clustering algorithm. In certain embodiments, the machine learning algorithm is a neural network. In some instances, the machine learning algorithm is a neural network such as an artificial neural network, a convolutional neural network or a recurrent neural network. In certain instances, the machine learning algorithm is a python script.

In some embodiments, classifying cells of the sample includes assigning each cell in the sample to a particle population cluster. As used herein, a "population", or "subpopulation" of classified cells refers to a group of cells that possess related parameters (for example, delta center of mass, radial moment, eccentricity) such that measured data form a cluster in the data space. Thus, populations are recognized as clusters in the data. Conversely, each particle population cluster may be interpreted as corresponding to a compound population of a particular category of cell (e.g., cell-cell interaction, internalization, phagocytosis, tumor cell killing, exhibiting cellular component translocation, cell morphology, mitotic cell phase, plant cell developmental stage, etc.), although clusters that correspond to noise or background typically also are observed. A particle population cluster may be defined in a subset of the dimensions, e.g., with respect to a subset of the measured image parameters, which corresponds to compound populations that differ in only a subset of the measured image parameters or features extracted from the measurements of the particle.

In some embodiments, methods include determining one or more sorting gates for the classified cells of the sample. In some instances, methods include determining sorting gates for cells of a particular mitotic phase. In some instances, methods include determining sorting gates for rare cell populations. In some instances, methods include determining sorting gates for identified plant cells. In some instances, methods include determining sorting gates for plant cells having an identified developmental stage. In some instances, methods include determining sorting gates for cells exhibiting translocation of a cellular component. In some instances, methods include determining sorting gates for cells having a desired morphology. In some instances, methods include determining sorting gates for cell-cell interaction (e.g., tumor cell killing interaction, internalization interaction, phagocytosis interaction, passive synapse interaction, active synapse interaction, co-localization)

The term "gate" is used herein in its conventional sense to refer to a classifier boundary identifying a subset of data of interest. In some instances, a gate can bound a group of events of particular interest. In addition, "gating" may refer to the process of classifying the data using a defined gate for a given set of data, where the gate can be one or more regions of interest combined with Boolean logic. In some embodiments, a gate identifies particles exhibiting the same image parameters. Examples of methods for gating have been described in, for example, U.S. Pat. Nos. 4,845,653; 5,627,040; 5,739,000; 5,795,727; 5,962,238; 6,014,904; 6,944,338; and 8,990,047. In some embodiments, the gate bounds a particle population cluster from one or more different samples that has previously been determined (e.g., by a user), to correspond to properties of interest.

In some instances, the one or more sorting gates capture cells of a target particle population cluster and exclude particles of a non-target particle population cluster. In some instances, the sorting gates maximize the inclusion yield of cells of a target particle population cluster. In some instances, the sorting gates are configured to maximize the inclusion yield of a target particle population cluster, such as where the sorting gates are configured to generate an inclusion yield of cells of a target particle population cluster of 50% or more, such as 55% or more, such as 60% or more, such as 65% or more, such as 70% or more, such as 75% or more, such as 80% or more, such as 85% or more, such as 90% or more, such as 95% or more, such as 97% or more, such as 99% or more and including determining a sorting gate that is configured to generate an inclusion yield of cells of a target particle population cluster of 99.9% or more. In some instances, the sorting gates are configured to maximize the purity yield of cells of a target particle population cluster, such as where the sorting gates are configured to generate a purity yield of cells of a target particle population cluster of 50% or more, such as 55% or more, such as 60% or more, such as 65% or more, such as 70% or more, such as 75% or more, such as 80% or more, such as 85% or more, such as 90% or more, such as 95% or more, such as 97% or more, such as 99% or more and including determining a sorting gate that is configured to generate a purity yield of cells of target particle population cluster of 99.9% or more.

In some instances, the sorting gates are configured to maximize the exclusion of particles of a non-target particle population. In some instances, the sorting gates are configured to exclude 50% or more of particles of non-target particle populations, such as 55% or more, such as 60% or more, such as 65% or more, such as 70% or more, such as 75% or more, such as 80% or more, such as 85% or more, such as 90% or more, such as 95% or more, such as 97% or more, such as 99% or more and including determining a sorting gate that is configured to exclude 99.9% or more of particles of a non-target particle population cluster. In some instances, the sorting gates exclude particles of a non-target particle population cluster based on a calculated Mahalanobis distance from a target particle population cluster.

In certain embodiments, generating the sorting gates includes an Fβ score, where the Fβ score is a weighted harmonic mean of the inclusion of particles of a target particle population cluster and the exclusion of particles of a non-target particle population cluster. The beta (β) parameter of the Fβ score will bias the gating strategy toward either yield (e.g., increased inclusion of target particles) or purity (e.g., increased exclusion of non-target particles) of the sample. For example, where the Fβ score is equal to 1, the purity and yield have equal contributions to the gating strategy. Where the Fβ score is equal to 2, the gating strategy emphasizes yield over purity. Where the Fβ score is equal to 0.5, the gating strategy will emphasize purity over yield. In some embodiments, the Fβ score ranges from 0.1 to 10, such as from 0.2 to 9.5, such as from 0.3 to 9, such as from 0.4 to 8.5, such as from 0.5 to 8, such as from 0.5 to 7, such as from 0.5 to 6, such as from 0.5 to 5, such as from 0.5 to 4, such as from 0.5 to 3, such as from 0.5 to 2. In some instances, the generated sorting gates of interest have an Fβ score of 1 or more. In some instances, the generated sorting gates have an Fβ score of less than 1.

In some instances, methods include assessing the sorting gates of the gating strategy and adjusting one or more of the generated sorting gates. The adjustment may be made based on a metric indicating the accuracy of the sorting gates according to the desired sorting strategy. In some instances, the metric may be an accuracy (e.g., purity) metric where the purity is compared to a predetermined threshold. The metric may be generated based on a confidence of the classifiers included in the sort strategy. In other instances, the metric may be a yield metric where the yield of target cells of a particle population cluster is compared to a predetermined threshold.

In some embodiments, methods include generating a sorting decision based on the determined sorting gates for the cells of the sample. In some instances, generating a particle sorting decision according to the present disclosure includes a gating strategy of 8 sorting gates or less, such as 7 sorting gates or less, such as 6 sorting gates or less, such as 5 sorting gates or less, such as 4 sorting gates or less, such as 3 sorting gates or less and including 2 sorting gates or less. In some embodiments, methods include generating sorting gates using a graphical display (as described below) that displays one or more analysis algorithms for applying the classification parameters to the image parameters determined for the cells.

In some embodiments, methods include displaying the gating strategy on the graphical user interface. For example, the analysis algorithm may be one or more of a spectral compensation matrix, a clustering algorithm and a t-Distributed Stochastic Neighbor Embedding (t-SNE) algorithm. In some instances, the analysis algorithm is applied to the particle population cluster by dragging an icon of the analysis algorithm onto the particle population cluster. In other instances, the particle population cluster is selected and the analysis algorithm is applied by selecting from a drop-down menu. In certain instances, the analysis algorithm is a spectral unmixing algorithm, such as described in U.S. Patent No. 11,009,400 and International Patent Application No. PCT/US2021/46741 filed on August 19, 2021. In some instances, the gating strategy is determined using computational sorting algorithms such as described with in U.S. Patent No. 11,513,054. In certain instances, the sorting gates may be determined using computer software such as HyperFinder (e.g., as described in Bonavia, et al. Frontiers in Immunology 2022; 13: 1007016) and Computational Sorting with HyperFinder, FlowJo Software and BD FACSDiva Software (Becton Dickinson, 2021). In certain embodiments, the gating strategy is developed on a separate computational system (e.g., a different computer system or network) and communicated to a flow cytometer for implementing the gating strategy, such as for example using a particle sorter (e.g, having a sort decision module) of the flow cytometer.

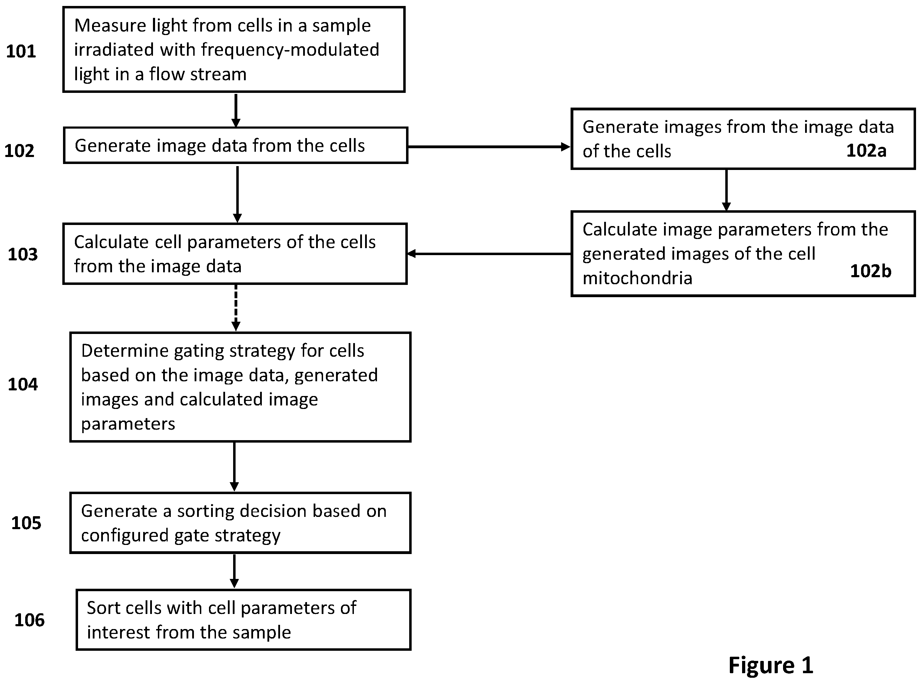

Figure 1 depicts a flowchart for calculating cell parameters for cells of a sample according to certain embodiments. Light from cells in a sample irradiated with frequency-modulated beams of light in a flow stream is measured with a photodetector at step 101. In some instances, methods include irradiating the cells of the sample in a flow stream, such as with radiofrequency-shifted beams of laser light. In some instances, frequency-encoded data signals are generated from the measured light. Image data (e.g., digital waveforms generated by the light detection system in one or more photodetector channels) of the cells are generated at step 102. In some instances, images of the cells are generated (step 102a). In some instances, the images visualize a cell-cell interaction (co-localization, internalization, phagocytosis, tumor cell killing). In some instances, the images visualize a cellular component translocation (e.g., nuclear translocation of a transcription factor from the cytoplasm to the nucleus). In some instances, the images visualize cells of a rare cell population. In some instances, the images visualize plant cells. In some instances, the images visualize one or more mitotic phases (e.g., anaphase, telophase, etc.). In some instances, the images visualize a cell developmental phase.

In some instances, image parameters (e.g., radial moment) are calculated from the generated images at step 102b. In some instances, methods include calculating a plurality of image parameters for each cell, such as 50 or more image parameters. Cell parameters for cells of the sample are calculated at step 103. Where cells are sorted, such as to isolate cells having particular cell parameters of interest (e.g., internalized cells or cells in a particular mitotic phase), a gating strategy is determined for the cells at step 104. A gating strategy having 1 or more applied gates (e.g., from 4 to 8 applied gates) is developed and a sorting decision at step 105 can be generated using the gating strategy. In some instances, cells are sorted into different containers (step 106), such as with a droplet sorter (e.g., having a droplet charging device, a piezoelectric transducer for generating distinct droplets with a cell and deflection plates for deflecting the droplets into different sample containers).

In certain embodiments, methods include sorting one or more of the cells of the sample based on the calculated cell parameters. The term "sorting" is used herein in its conventional sense to refer to separating components (e.g., droplets containing cells, droplets containing non-cellular particles such as biological macromolecules) of a sample and in some instances, delivering the separated components to one or more sample collection containers. For example, methods may include sorting 2 or more components of the sample, such as 3 or more components, such as 4 or more components, such as 5 or more components, such as 10 or more components, such as 15 or more components and including sorting 25 or more components of the sample.

In sorting particles, methods include data acquisition, analysis and recording, such as with a computer, where multiple data channels record data from each detector used in obtaining the overlapping spectra of the plurality of fluorophores associated with the particle. In these embodiments, analysis includes spectrally resolving light (e.g., by calculating the spectral unmixing matrix) from the plurality of fluorophores having overlapping spectra that are associated with the particle and identifying the particle based on the estimated abundance of each fluorophore associated with the particle. This analysis may be conveyed to a sorting system which is configured to generate a set of digitized parameters based on the particle classification.

In some embodiments, methods for sorting components of sample include sorting cells of the sample with a particle sorting module having deflector plates, such as described in U.S. Patent Publication No. 2017/0299493, filed on March 28, 2017. In certain embodiments, cells of the sample are sorted using a sort decision module having a plurality of sort decision units, such as those described in U.S. Patent Publication No. 2020/0256781**.** In some embodiments, the subject systems include a particle sorting module having deflector plates, such as described in U.S. Patent Publication No. 2017/0299493, filed on March 28, 2017.

### SYSTEMS FOR CALCULATING CELL PARAMETERS FOR CELLS OF A SAMPLE IN A FLOW STREAM

As summarized above, aspects of the present disclosure include a system for calculating cell parameters of cells in a sample in a flow stream. Systems according to certain embodiments include a light source configured to irradiate cells of a sample in a flow stream with frequency-modulated beams of light, a light detection system having a photodetector for measuring light from the cells, and a processor with memory operably coupled to the processor where the memory includes instructions stored thereon, which when executed by the processor, cause the processor to generate image data of the cells from the measured light and calculate one or more cell parameters based on the generated image data.

In embodiments, the light source may be any suitable broadband or narrow band source of light. Depending on the components in the sample (e.g., cells with cell-cell interactions, cells exhibiting intracellular translocation, etc.), the light source may be configured to emit wavelengths of light that vary, ranging from 200 nm to 1500 nm, such as from 250 nm to 1250 nm, such as from 300 nm to 1000 nm, such as from 350 nm to 900 nm and including from 400 nm to 800 nm. For example, the light source may include a broadband light source emitting light having wavelengths from 200 nm to 900 nm. In other instances, the light source includes a narrow band light source emitting a wavelength ranging from 200 nm to 900 nm. For example, the light source may be a narrow band LED (1 nm - 25 nm) emitting light having a wavelength ranging between 200 nm to 900 nm. In certain embodiments, the light source is a laser. In some instances, the subject systems include a gas laser, such as a helium-neon laser, argon laser, krypton laser, xenon laser, nitrogen laser, CO₂ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof. In others instances, the subject systems include a dye laser, such as a stilbene, coumarin or rhodamine laser. In yet other instances, lasers of interest include a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof. In still other instances, the subject systems include a solid-state laser, such as a ruby laser, an Nd:YAG laser, NdCrYAG laser, Er:YAG laser, Nd:YLF laser, Nd:YVO₄ laser, Nd:yCa₄O(BO₃)₃ laser, Nd:YCOB laser, titanium sapphire laser, thulim YAG laser, ytterbium YAG laser, ytterbium₂O₃ laser or cerium doped lasers and combinations thereof.

In other embodiments, the light source is a non-laser light source, such as a lamp, including but not limited to a halogen lamp, deuterium arc lamp, xenon arc lamp, a light-emitting diode, such as a broadband LED with continuous spectrum, superluminescent emitting diode, semiconductor light emitting diode, wide spectrum LED white light source, an multi-LED integrated. In some instances, the non-laser light source is a stabilized fiber-coupled broadband light source, white light source, among other light sources or any combination thereof.

The light source may be positioned any suitable distance from the sample (e.g., the flow stream in a flow cytometer), such as at a distance of 0.001 mm or more from the flow stream, such as 0.005 mm or more, such as 0.01 mm or more, such as 0.05 mm or more, such as 0.1 mm or more, such as 0.5 mm or more, such as 1 mm or more, such as 5 mm or more, such as 10 mm or more, such as 25 mm or more and including at a distance of 100 mm or more. In addition, the light source irradiate the sample at any suitable angle (e.g., relative the vertical axis of the flow stream), such as at an angle ranging from 10° to 90°, such as from 15° to 85°, such as from 20° to 80°, such as from 25° to 75° and including from 30° to 60°, for example at a 90° angle.

The light source may be configured to irradiate the sample continuously or in discrete intervals. In some instances, systems include a light source that is configured to irradiate the sample continuously, such as with a continuous wave laser that continuously irradiates the flow stream at the interrogation point in a flow cytometer. In other instances, systems of interest include a light source that is configured to irradiate the sample at discrete intervals, such as every 0.001 milliseconds, every 0.01 milliseconds, every 0.1 milliseconds, every 1 millisecond, every 10 milliseconds, every 100 milliseconds and including every 1000 milliseconds, or some other interval. Where the light source is configured to irradiate the sample at discrete intervals, systems may include one or more additional components to provide for intermittent irradiation of the sample with the light source. For example, the subject systems in these embodiments may include one or more laser beam choppers, manually or computer controlled beam stops for blocking and exposing the sample to the light source.

In some embodiments, the light source is a laser. Lasers of interest may include pulsed lasers or continuous wave lasers. For example, the laser may be a gas laser, such as a helium-neon laser, argon laser, krypton laser, xenon laser, nitrogen laser, CO₂ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof; a dye laser, such as a stilbene, coumarin or rhodamine laser; a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof; a solid-state laser, such as a ruby laser, an Nd:YAG laser, NdCrYAG laser, Er:YAG laser, Nd:YLF laser, Nd:YVO₄ laser, Nd:yCa₄O(BO₃)₃ laser, Nd:YCOB laser, titanium sapphire laser, thulim YAG laser, ytterbium YAG laser, ytterbium₂O₃ laser or cerium doped lasers and combinations thereof; a semiconductor diode laser, optically pumped semiconductor laser (OPSL), or a frequency doubled- or frequency tripled implementation of any of the above mentioned lasers.

In some embodiments, the light source is a light beam generator that is configured to generate two or more beams of frequency shifted light. In some instances, the light beam generator includes a laser, a radiofrequency generator configured to apply radiofrequency drive signals to an acousto-optic device to generate two or more angularly deflected laser beams. In these embodiments, the laser may be a pulsed lasers or continuous wave laser. For example lasers in light beam generators of interest may be a gas laser, such as a helium-neon laser, argon laser, krypton laser, xenon laser, nitrogen laser, CO2 laser, CO laser, argon-fluorine (ArF) excimer laser, krypton-fluorine (KrF) excimer laser, xenon chlorine (XeCl) excimer laser or xenon-fluorine (XeF) excimer laser or a combination thereof; a dye laser, such as a stilbene, coumarin or rhodamine laser; a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof; a solid-state laser, such as a ruby laser, an Nd:YAG laser, NdCrYAG laser, Er:YAG laser, Nd:YLF laser, Nd:YVO₄ laser, Nd:yCa₄O(BO₃)₃ laser, Nd:YCOB laser, titanium sapphire laser, thulim YAG laser, ytterbium YAG laser, ytterbium₂O₃ laser or cerium doped lasers and combinations thereof.

The acousto-optic device may be any convenient acousto-optic protocol configured to frequency shift laser light using applied acoustic waves. In certain embodiments, the acousto-optic device is an acousto-optic deflector. The acousto-optic device in the subject system is configured to generate angularly deflected laser beams from the light from the laser and the applied radiofrequency drive signals. The radiofrequency drive signals may be applied to the acousto-optic device with any suitable radiofrequency drive signal source, such as a direct digital synthesizer (DDS), arbitrary waveform generator (AWG), or electrical pulse generator.

In embodiments, a controller is configured to apply radiofrequency drive signals to the acousto-optic device to produce the desired number of angularly deflected laser beams in the output laser beam, such as being configured to apply 3 or more radiofrequency drive signals, such as 4 or more radiofrequency drive signals, such as 5 or more radiofrequency drive signals, such as 6 or more radiofrequency drive signals, such as 7 or more radiofrequency drive signals, such as 8 or more radiofrequency drive signals, such as 9 or more radiofrequency drive signals, such as 10 or more radiofrequency drive signals, such as 15 or more radiofrequency drive signals, such as 25 or more radiofrequency drive signals, such as 50 or more radiofrequency drive signals and including being configured to apply 100 or more radiofrequency drive signals.

In some instances, to produce an intensity profile of the angularly deflected laser beams in the output laser beam, the controller is configured to apply radiofrequency drive signals having an amplitude that varies such as from about 0.001 V to about 500 V, such as from about 0.005 V to about 400 V, such as from about 0.01 V to about 300 V, such as from about 0.05 V to about 200 V, such as from about 0.1 V to about 100 V, such as from about 0.5 V to about 75 V, such as from about 1 V to 50 V, such as from about 2 V to 40 V, such as from 3 V to about 30 V and including from about 5 V to about 25 V. Each applied radiofrequency drive signal has, in some embodiments, a frequency of from about 0.001 MHz to about 500 MHz, such as from about 0.005 MHz to about 400 MHz, such as from about 0.01 MHz to about 300 MHz, such as from about 0.05 MHz to about 200 MHz, such as from about 0.1 MHz to about 100 MHz, such as from about 0.5 MHz to about 90 MHz, such as from about 1 MHz to about 75 MHz, such as from about 2 MHz to about 70 MHz, such as from about 3 MHz to about 65 MHz, such as from about 4 MHz to about 60 MHz and including from about 5 MHz to about 50 MHz.

In certain embodiments, the controller has a processor having memory operably coupled to the processor such that the memory includes instructions stored thereon, which when executed by the processor, cause the processor to produce an output laser beam with angularly deflected laser beams having a desired intensity profile. For example, the memory may include instructions to produce two or more angularly deflected laser beams with the same intensities, such as 3 or more, such as 4 or more, such as 5 or more, such as 10 or more, such as 25 or more, such as 50 or more and including memory may include instructions to produce 100 or more angularly deflected laser beams with the same intensities. In other embodiments, the memory may include instructions to produce two or more angularly deflected laser beams with different intensities, such as 3 or more, such as 4 or more, such as 5 or more, such as 10 or more, such as 25 or more, such as 50 or more and including memory may include instructions to produce 100 or more angularly deflected laser beams with different intensities.

In certain embodiments, the controller has a processor having memory operably coupled to the processor such that the memory includes instructions stored thereon, which when executed by the processor, cause the processor to produce an output laser beam having increasing intensity from the edges to the center of the output laser beam along the horizontal axis. In these instances, the intensity of the angularly deflected laser beam at the center of the output beam may range from 0.1% to about 99% of the intensity of the angularly deflected laser beams at the edge of the output laser beam along the horizontal axis, such as from 0.5% to about 95%, such as from 1% to about 90%, such as from about 2% to about 85%, such as from about 3% to about 80%, such as from about 4% to about 75%, such as from about 5% to about 70%, such as from about 6% to about 65%, such as from about 7% to about 60%, such as from about 8% to about 55% and including from about 10% to about 50% of the intensity of the angularly deflected laser beams at the edge of the output laser beam along the horizontal axis. In other embodiments, the controller has a processor having memory operably coupled to the processor such that the memory includes instructions stored thereon, which when executed by the processor, cause the processor to produce an output laser beam having an increasing intensity from the edges to the center of the output laser beam along the horizontal axis. In these instances, the intensity of the angularly deflected laser beam at the edges of the output beam may range from 0.1% to about 99% of the intensity of the angularly deflected laser beams at the center of the output laser beam along the horizontal axis, such as from 0.5% to about 95%, such as from 1% to about 90%, such as from about 2% to about 85%, such as from about 3% to about 80%, such as from about 4% to about 75%, such as from about 5% to about 70%, such as from about 6% to about 65%, such as from about 7% to about 60%, such as from about 8% to about 55% and including from about 10% to about 50% of the intensity of the angularly deflected laser beams at the center of the output laser beam along the horizontal axis. In yet other embodiments, the controller has a processor having memory operably coupled to the processor such that the memory includes instructions stored thereon, which when executed by the processor, cause the processor to produce an output laser beam having an intensity profile with a Gaussian distribution along the horizontal axis. In still other embodiments, the controller has a processor having memory operably coupled to the processor such that the memory includes instructions stored thereon, which when executed by the processor, cause the processor to produce an output laser beam having a top hat intensity profile along the horizontal axis.

In embodiments, light beam generators of interest may be configured to produce angularly deflected laser beams in the output laser beam that are spatially separated. Depending on the applied radiofrequency drive signals and desired irradiation profile of the output laser beam, the angularly deflected laser beams may be separated by 0.001 µm or more, such as by 0.005 µm or more, such as by 0.01 µm or more, such as by 0.05 µm or more, such as by 0.1 µm or more, such as by 0.5 µm or more, such as by 1 µm or more, such as by 5 µm or more, such as by 10 µm or more, such as by 100 µm or more, such as by 500 µm or more, such as by 1000 µm or more and including by 5000 µm or more. In some embodiments, systems are configured to produce angularly deflected laser beams in the output laser beam that overlap, such as with an adjacent angularly deflected laser beam along a horizontal axis of the output laser beam. The overlap between adjacent angularly deflected laser beams (such as overlap of beam spots) may be an overlap of 0.001 µm or more, such as an overlap of 0.005 µm or more, such as an overlap of 0.01 µm or more, such as an overlap of 0.05 µm or more, such as an overlap of 0.1 µm or more, such as an overlap of 0.5 µm or more, such as an overlap of 1 µm or more, such as an overlap of 5 µm or more, such as an overlap of 10 µm or more and including an overlap of 100 µm or more.

In certain instances, light beam generators configured to generate two or more beams of frequency shifted light include laser excitation modules as described in Diebold, et al. Nature Photonics Vol. 7(10); 806-810 (2013) as well as described in U.S. Patent Nos. 9,423,353; 9,784,661; 9,983,132; 10,006,852; 10,078,045; 10,036,699; 10,222,316; 10,288,546; 10,324,019; 10,408,758; 10,451,538; 10,620,111; and U.S. Patent Publication Nos. 2017/0133857; 2017/0328826; 2017/0350803; 2018/0275042; 2019/0376895 and 2019/0376894.

In embodiments, systems include a light detection system having a plurality of photodetectors for measuring light from the cells in the sample. In some instances, one or more photodetectors of the light detection system are light loss photodetectors. In some instances, one or more photodetectors of the light detection system are configured to measure scattered light. In certain instances, one or more photodetectors of the light detection system are configured to measure side-scattered light. In certain instances, one or more photodetectors of the light detection system are configured to measure forward-scattered light. In certain instances, one or more photodetectors of the light detection system are configured to measure back-scattered light. Photodetectors of interest may include, but are not limited to optical sensors, such as active-pixel sensors (APSs), avalanche photodiodes (APDs), image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes, phototransistors, quantum dot photoconductors or photodiodes and combinations thereof, among other photodetectors. In certain embodiments, light from a sample is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors.

In some embodiments, light detection systems of interest include a plurality of photodetectors. In some instances, the light detection system includes a plurality of solid-state detectors such as photodiodes. In certain instances, the light detection system includes a photodetector array, such as an array of photodiodes. In these embodiments, the photodetector array may include 4 or more photodetectors, such as 10 or more photodetectors, such as 25 or more photodetectors, such as 50 or more photodetectors, such as 100 or more photodetectors, such as 250 or more photodetectors, such as 500 or more photodetectors, such as 750 or more photodetectors and including 1000 or more photodetectors. For example, the detector may be a photodiode array having 4 or more photodiodes, such as 10 or more photodiodes, such as 25 or more photodiodes, such as 50 or more photodiodes, such as 100 or more photodiodes, such as 250 or more photodiodes, such as 500 or more photodiodes, such as 750 or more photodiodes and including 1000 or more photodiodes.

The photodetectors may be arranged in any geometric configuration as desired, where arrangements of interest include, but are not limited to a square configuration, rectangular configuration, trapezoidal configuration, triangular configuration, hexagonal configuration, heptagonal configuration, octagonal configuration, nonagonal configuration, decagonal configuration, dodecagonal configuration, circular configuration, oval configuration as well as irregular patterned configurations. The photodetectors in the photodetector array may be oriented with respect to the other (as referenced in an X-Z plane) at an angle ranging from 10° to 180°, such as from 15° to 170°, such as from 20° to 160°, such as from 25° to 150°, such as from 30° to 120° and including from 45° to 90°. The photodetector array may be any suitable shape and may be a rectilinear shape, e.g., squares, rectangles, trapezoids, triangles, hexagons, etc., curvilinear shapes, e.g., circles, ovals, as well as irregular shapes, e.g., a parabolic bottom portion coupled to a planar top portion. In certain embodiments, the photodetector array has a rectangular-shaped active surface.

Each photodetector (e.g., photodiode) in the array may have an active surface with a width that ranges from 5 µm to 250 µm, such as from 10 µm to 225 µm, such as from 15 µm to 200 µm, such as from 20 µm to 175 µm, such as from 25 µm to 150 µm, such as from 30 µm to 125 µm and including from 50 µm to 100 µm and a length that ranges from 5 µm to 250 µm, such as from 10 µm to 225 µm, such as from 15 µm to 200 µm, such as from 20 µm to 175 µm, such as from 25 µm to 150 µm, such as from 30 µm to 125 µm and including from 50 µm to 100 µm, where the surface area of each photodetector (e.g., photodiode) in the array ranges from 25 to µm² to 10000 µm², such as from 50 to µm² to 9000 µm², such as from 75 to µm² to 8000 µm², such as from 100 to µm² to 7000 µm², such as from 150 to µm² to 6000 µm² and including from 200 to µm² to 5000 µm².

The size of the photodetector array may vary depending on the amount and intensity of the light, the number of photodetectors and the desired sensitivity and may have a length that ranges from 0.01 mm to 100 mm, such as from 0.05 mm to 90 mm, such as from 0.1 mm to 80 mm, such as from 0.5 mm to 70 mm, such as from 1 mm to 60 mm, such as from 2 mm to 50 mm, such as from 3 mm to 40 mm, such as from 4 mm to 30 mm and including from 5 mm to 25 mm. The width of the photodetector array may also vary, ranging from 0.01 mm to 100 mm, such as from 0.05 mm to 90 mm, such as from 0.1 mm to 80 mm, such as from 0.5 mm to 70 mm, such as from 1 mm to 60 mm, such as from 2 mm to 50 mm, such as from 3 mm to 40 mm, such as from 4 mm to 30 mm and including from 5 mm to 25 mm. As such, the active surface of the photodetector array may range from 0.1 mm² to 10000 mm², such as from 0.5 mm² to 5000 mm², such as from 1 mm² to 1000 mm², such as from 5 mm² to 500 mm², and including from 10 mm² to 100 mm².

Photodetectors of interest are configured to measure collected light at one or more wavelengths, such as at 2 or more wavelengths, such as at 5 or more different wavelengths, such as at 10 or more different wavelengths, such as at 25 or more different wavelengths, such as at 50 or more different wavelengths, such as at 100 or more different wavelengths, such as at 200 or more different wavelengths, such as at 300 or more different wavelengths and including measuring light emitted by a sample in the flow stream at 400 or more different wavelengths.

In some embodiments, photodetectors are configured to measure collected light over a range of wavelengths (e.g., 200 nm - 1000 nm). In certain embodiments, photodetectors of interest are configured to collect spectra of light over a range of wavelengths. For example, systems may include one or more detectors configured to collect spectra of light over one or more of the wavelength ranges of 200 nm - 1000 nm. In yet other embodiments, detectors of interest are configured to measure light from the sample in the flow stream at one or more specific wavelengths. For example, systems may include one or more detectors configured to measure light at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof.

The light detection system is configured to measure light continuously or in discrete intervals. In some instances, photodetectors of interest are configured to take measurements of the collected light continuously. In other instances, the light detection system is configured to take measurements in discrete intervals, such as measuring light every 0.001 millisecond, every 0.01 millisecond, every 0.1 millisecond, every 1 millisecond, every 10 milliseconds, every 100 milliseconds and including every 1000 milliseconds, or some other interval.

In embodiments, systems are configured to generate one or more images of the cells in the sample. In some embodiments, systems include memory having instructions stored thereon for generating the image from detected light absorption, detected light scatter, detected light emission or any combination thereof. In some instances, the memory includes instructions for generating the image from light absorption detected from the sample, such as from a brightfield light detector. In some instances, the memory includes instructions for generating the image from light scatter detected from the sample, such as from a side scatter detector, a forward scatter detector or a combination of a side scatter detector and forward scatter detector. In some instances, the memory includes instructions for generating the image from emitted light from the sample. In other instances, the memory includes instructions for generating the image from a combination of detected light absorption and detected light scatter. In certain instances, the memory includes instructions for generating images of the cell mitochondria (e.g., to calculate mitotic phase of a cell) from fluorescence from the labelled mitochondria and one or more light loss, side-scattered light and forward scattered light.

In some instances, the memory includes instructions to generate image data, such as digital waveforms generated in one or more photodetector channels. In some instances, the memory includes instructions for calculating image parameters directly from the waveforms. In some instances, the memory includes instructions for calculating image parameters from the image data, the generated images or a combination thereof. In some instances, the memory includes instructions for determining the cell parameters only from calculated image parameters (e.g., radial moment, eccentricity, etc.) In some instances, the memory includes instructions for determining the cell parameters only from the images of the particles and not from another data source, such as for example data signal waveforms. In some instances, the memory includes instructions for determining the cell parameters using a combination of a generated image of the particles and data signal waveforms generated in response to measured light from irradiated particles.

In some embodiments, the memory includes instructions for generating one or more images from the measured light. In some embodiments, the memory includes instructions for generating a single image for each cell in the sample from each form of detected light. In other embodiments, the memory includes instructions for generating a plurality of images for each cell, such as 2 or more, such as 3 or more, such as 5 or more, such as 10 or more and including 25 or more images for each cell. For example, the memory includes instructions for generating a first image of the cell from fluorescence detected from the labelled cell; instructions for generating a second image of the cell from detected light absorption and instructions for generating a third image from detected light scatter. In other embodiments, the memory includes instructions to generate two or more images from each form of detected light, such as 3 or more, such as 4 or more, such as 5 or more and including 10 or more images or a combination thereof.

In some embodiments, systems include memory having instructions for generating frequency-encoded data (e.g., frequency-encoded spatial data) from the measured light from the cells of the sample in the flow stream. In some instances, the memory includes instructions for generating one or more images from the frequency-encoded data. The frequency-encoded data may be generated in one or more detection channels, such as 2 or more, such as 3 or more, such as 4 or more, such as 5 or more, such as 6 or more and including 8 or more detection channels. In some embodiments, the frequency-encoded data includes data components taken (or derived) from light from different detectors, such as detected light absorption or detected light scatter. In some instances, the memory includes instructions for phase-correcting the frequency-encoded data. In some instances, the memory includes instructions to generate phase-corrected images of the cells by performing a transform on the frequency-encoded data. In one example, the memory includes instructions to phase correct the frequency-encoded data by performing a Fourier transform (FT) of the frequency-encoded data. In another example, the memory includes instructions to phase correct the frequency-encoded data by performing a discrete Fourier transform (DFT) of the frequency-encoded data. In yet another example, the memory includes instructions to phase correct the frequency-encoded data by performing a short time Fourier transform (STFT) of the frequency-encoded data. In certain embodiments, the memory includes instructions for performing a transform of the frequency encoded data without performing any mathematical imaginary computations (i.e., only performing computations for mathematical real computations of the transform) to generate an image from the frequency-encoded data.

In some instances, the memory includes instructions to generate one or more greyscale images of the cells of the sample. The term "greyscale" is used herein in its conventional sense to refer to images of the cells in the flow stream that are composed of varying shades of gray that are based on the intensity of light at each pixel. In some embodiments, the memory includes instructions to determine a pixel intensity threshold from the greyscale image where the pixel intensity threshold value is used to convert each pixel into a binary value that is used to generate the image of the cells. In certain instances, the image of the cells and cell mitochondria is a binary pixel image of the particle such as where each pixel is assigned a binary pixel value of 1 (e.g., where the intensity of the pixel exceeds a predetermined threshold) or a binary pixel value of 0 (e.g., where the intensity of the pixel falls below a predetermined threshold).

Systems include a memory having instructions for calculating one or more image parameters from the generated images of the cells. In some instances, the memory includes instructions for calculating a center of mass image parameter from the generated image. In some instances, the memory includes instructions for calculating a delta center of mass image parameter from the generated image. In some instances, the memory includes instructions for calculating a diffusivity image parameter from the generated image. The memory includes instructions for calculating an eccentricity image parameter from the generated image. In some instances, the memory includes instructions for calculating a long axis moment image parameter from the generated image. In some instances, the memory includes instructions for calculating a maximum intensity image parameter from the generated image. The memory includes instructions for calculating a radial moment image parameter from the generated image. In some instances, the memory includes instructions for calculating a short axis moment image parameter from the generated image. In some instances, the memory includes instructions for calculating a particle size image parameter from the generated image. In some instances, the memory includes instructions for calculating a total intensity image parameter from the generated image. In some instances, the memory includes instructions for calculating a particle light loss image parameter from the generated image. In some instances, the memory includes instructions for calculating a forward scattered light image parameter from the generated image. In some instances, the memory includes instructions for calculating a side scattered light image parameter from the generated image. In some instances, the memory includes instructions for calculating an image moment from the generated image. In some instances, the memory includes instructions for calculating the center of mass from the image moment of the image. In other instances, the memory includes instructions for calculating the orientation of the cell from the image moment of the image. In still other instances, the memory includes instructions for calculating the eccentricity of the cell from the image moment of the image.

The memory includes instructions for calculating a cell parameter by assessing a cell interaction parameter. The term "interaction" is used herein in its conventional sense to refer to physical or functional coupling of two or more cells. In some instances, the cells are in physical contact with each other. In some instances, the cells are co-localized with each other in a generated image, such as where the cells are positioned within 1 µm or less of each other, such as 0.5 µm or less, such as 0.1 µm or less, such as 0.05 µm or less, such as 0.01 µm or less, such as 0.005 µm or less, such as 0.001 µm or less and including within 0.0001 µm or less of each other. In some instances, one or more cells are internalized within a cell.

In some instances, the cell interaction is active interaction between the cells, such as where the two or more cells are in active communication with each other (e.g., through an active synapse). In other instances, the cell interaction is a passive interaction between the cells, such as where the two or more cells are in passive communication with each other (e.g., through a passive synapse). In other instances, the cell interaction is a coincident doublet in the generated image of the cells. In other instances, the cell interaction is an engaged doublet in the generated image of the cells. In some instances, the memory includes instructions for calculating the cell interaction parameter by assessing internalization of a cell or cellular component by one or more cells in the sample. In some instances, the memory includes instructions for calculating the cell interaction parameter by assessing co-localization between two or more cells in the sample. In some instances, the memory includes instructions for calculating a cell parameter includes by a phagocytosis cell interaction.

In certain instances, the memory includes instructions for calculating a cell parameter by assessing a tumor cell killing interaction, such as where one of the cells is a cancer cell and the interacting cell is a lymphocyte. In some instances, assessing the tumor cell killing interaction includes a heterogenous doublet lymphocyte and cancer cell. In some instances, assessing the tumor cell killing interaction includes a homogeneous doublet lymphocyte and lymphocyte.

In some embodiments, the memory includes instructions for calculating a cell parameter by assessing a translocation parameter of a cellular component within the cells of the sample. In some instances, the memory includes instructions for calculating the cell parameter by determining the displacement of a cellular component between different organelles within the cell. In some instances, the cell parameter characterizes the type of component being translocated within the cells. For example, in some instances the memory includes instructions for calculating a cell parameter by assessing protein translocation within the cell. In certain instances, the memory includes instructions for assessing transcription factor translocation. In some instances, the memory includes instructions for calculating a cell parameter by assessing lipid translocation within the cell. In some instances, the memory includes instructions for calculating a cell parameter by assessing polysaccharide translocation within the cell. In some instances, the memory includes instructions for calculating a cell parameter by assessing nucleic acid translocation within the cell. In certain instances, the memory includes instructions for calculating a cell parameter by assessing translocation of a cell signaling compound. In other instances, the memory includes instructions for calculating a cell parameter by assessing translocation of a cell growth factor within the cell. In certain embodiments, the memory includes instructions for calculating a cell parameter by measuring nuclear translocation, such as where a cellular component (e.g., NF-κβ transcription factor) is moved from the cytoplasm to the nucleus. In some instances, the memory includes instructions for calculating a cell parameter by measuring translocation of a component from the nucleus to the cytoplasm. In some instances, the memory includes instructions for calculating a cell parameter by measuring translocation of a component from the cytoplasm to the mitochondria. In some instances, the memory includes instructions for calculating a cell parameter by measuring translocation of a component from the mitochondria to the cytoplasm.

In some embodiments, the cell parameter is a cell mitotic phase parameter. In some instances, the memory includes instructions for calculating the cell parameter by determining the mitotic phase of one or more cells in the sample. In some instances, the memory includes instructions for identifying a cell as being in interphase. In some instances, the memory includes instructions for identifying a cell as being in prophase. In some instances, the memory includes instructions for identifying a cell as being in metaphase. In some instances, the memory includes instructions for identifying a cell as being in anaphase. In some instances, the memory includes instructions for identifying a cell as being in telophase. In certain instances, the memory includes instructions for identifying a cell as being in cytokinesis. In certain instances, the memory includes instructions for assessing the mitotic phase parameter from generated images of the cell mitochondria. In some instances, the memory includes instructions for assessing the mitotic phase parameter using image parameters calculated from the generated images of the mitochondria.

In some instances, the memory includes instructions for assessing the morphology of the cell mitochondria to determine the mitotic phase of the cell. In some instances, the memory includes instructions for determining the morphology of the cell mitochondria based on a radial moment image parameter. In some instances, the image parameter includes the radial moment of the fluorophore used to stain the mitochondria. In some instances, the memory includes instructions for calculating the radial moment image parameter from images generated based on fluorescence measured from the cell mitochondria, light loss measured for the cell mitochondria, forward scattered light measured for the cell mitochondria, side scattered light measured for the cell mitochondria and combinations thereof.

In some instances, the memory includes instructions to calculate the mitotic phase parameter from the generated images of the cells. In some instances, the memory includes instructions for calculating a cell mitochondria size image parameter from the generated image. In some instances, the memory includes instructions for calculating a cell mitochondria fluorescence radial moment image parameter from the generated image. In some instances, the memory includes instructions for a cell mitochondria light loss image parameter from the generated image. In some instances, the memory includes instructions for a forward scattered light radial moment image parameter from the generated image. In some instances, the memory includes instructions for a side scattered light radial moment image parameter from the generated image. In some instances, the memory includes instructions for an image moment from the generated image. In some instances, the center of mass of the cell mitochondria may be calculated from the image moment of the image. In other instances, the orientation of the cell mitochondria may be calculated from the image moment of the image. In still other instances, the eccentricity of the cell mitochondria may be calculated from the image moment of the image.

In some embodiments, the calculated image parameter is a radial moment of the fluorophore. In some instances, the memory includes instructions for calculating pixel density of the generated image of the cell mitochondria. In some instances, the memory includes instructions for calculating the number of pixels in a horizontal row of the image of the cell mitochondria. In some instances, the memory includes instructions for calculating the number of pixels in a vertical column of the image of the cell mitochondria. In certain instances, the memory includes instructions for calculating the absolute number of pixels found in the image of cell mitochondria. In some instances, the memory includes instructions for calculating the centroid of the cell mitochondria from one or more of the generated images. In some instances, the memory includes instructions for calculating the image parameter by determining the sum of the pixels in the generated image weighted by the distance of each pixel from the centroid of the cell mitochondria.

In some embodiments, the memory includes instructions for determining a cell parameter for assessing plant cell biology of one or more cells of the sample. In some instances, the memory includes instructions for calculating a cell parameter by identifying that a cell in the sample is a plant cell. In some instances, the memory includes instructions for calculating the cell parameter by distinguishing a plant cell from cellular debris in the sample. In some instances, the memory includes instructions for calculating a cell parameter to characterize plant cells of different sizes. In some instances, the memory includes instructions for identifying that the plant cell is a parenchyma cell. In some instances, the memory includes instructions for identifying that the plant cell is a collenchyma cell. In some instances, the memory includes instructions for identifying that the plant cell is a sclerenchyma cell. In some instances, the memory includes instructions for identifying that the plant cell is a xylem cell (e.g., tracheid). In some instances, the memory includes instructions for identifying that the plant cell is a phloem cell. In certain instances, the memory includes instructions for identifying that cells of the sample include protoplasts.

In some instances, the memory includes instructions for determining the developmental stage of the plant cell. In some instances, the memory includes instructions for determining that the plant cell is undergoing cell division. In some instances, the memory includes instructions for determining that the plant cell is undergoing cell elongation. In some instances, the memory includes instructions for determining that the plant cell is undergoing cell differentiation.

In some embodiments, the memory includes instructions for assessing the morphology of the plant cell. In some instances, the memory includes instructions for assessing the morphology of the plan cell by evaluating the size of the plant cell. In some instances, the size of each plant cell in the sample is measured. In some instances, the memory includes instructions for determining the size of each plant cell in the sample by comparing the size of the plant cell with a predetermined reference size (using a reference image of plant cells of interest), such as where the size of each cell is determined to be 50% or more of the predetermined reference size, such as 60% or more, such as 70% or more, such as 80% or more, such as 90% or more, such as 95% or more, such as 97% or more, such as 98% or more, such as 99% or more of the predetermined reference size. In some instances, the memory includes instructions for determining the number or percentage of cells in the sample that have a size that is within 15% or less of a predetermined reference size range, such as within 10% or less, such as within 5% or less, such as within 4% or less, such as within 3% or less, such as within 2% or less, such as within 1% or less and including within 0.5% or less of a predetermined reference size range. In some instances, the memory includes instructions for determining that the sample is a plant cell sample when the cells in the sample have a size that is within a reference size range.

In some embodiments, the memory includes instructions for assessing the morphology of the cells by evaluating a degree of punctateness of the cells. In some instances, the punctateness of each cell in the sample is measured. In some instances, the memory includes instructions for determining the punctateness of each cell in the sample by comparing the punctateness of the cell with a predetermined reference punctateness (using a reference image of the cell), such as where the punctateness of each cell is determined to be 50% or more of the predetermined reference size, such as 60% or more, such as 70% or more, such as 80% or more, such as 90% or more, such as 95% or more, such as 97% or more, such as 98% or more, such as 99% or more of the predetermined reference punctateness. In some instances, the memory includes instructions for determining the number or percentage of cell in the sample that have a punctateness that is within 15% or less of a predetermined reference size range, such as within 10% or less, such as within 5% or less, such as within 4% or less, such as within 3% or less, such as within 2% or less, such as within 1% or less and including within 0.5% or less of a predetermined reference size range. In some instances, the memory includes instructions for determining that the sample is a plant cell sample when the cells in the sample have a punctateness that is within a reference punctateness range.

In some embodiments, the memory includes instructions for assessing the morphology of the plant cells by evaluating the shape of the cells. In some instances, the memory includes instructions for determining the percentage of cells having a particular shape. In certain instances depending on the type of cell sample and desired shape of the cells, the memory includes instructions for determining that cells of the sample are plant cells when the percentage of cells having a particular desired shape exceeds a predetermined threshold, such as where 50% or more of the cells in the sample have a desired shape such as 60% or more, such as 70% or more, such as 80% or more, such as 90% or more, such as 95% or more, such as 97% or more, such as 98% or more, such as 99% or more and including where 99.5% or more of the cells in the sample have a particular desired shape.

In some embodiments, the memory includes instructions for calculating a cell parameter by determining that a cell belongs to a rare cell population. The term rare cell is used herein in its conventional sense to refer to cells in the sample in which the abundance of the type of cell is 0.1% of the cell population or less, such as 0.05% of the cell population or less, such as 0.01% of the cell population or less, such as 0.005% of the cell population or less, such as 0.001% of the cell population or less, such as 0.0005% of the cell population or less and including where the type of cell is 0.0001% of the cell population or less. In some instances, the memory includes instructions for calculating a cell parameter by determining that the rare cell is a stem cell. In some instances, the memory includes instructions for calculating a cell parameter by determining that the rare cell is a circulating endothelial cell. In some instances, the memory includes instructions for calculating a cell parameter by determining that the rare cell is a circulating tumor (malignant or benign) cell. In some instances, the memory includes instructions for calculating a cell parameter by determining that the rare cell is a residual disease cell, such as a disease of the blood or bone marrow.

In some instances, the system includes a processor with memory for running a machine learning algorithm that is a dynamic algorithm that classifies cells of the sample. The system may be configured to implement any convenient machine learning algorithm where machine learning algorithms of interest can include, but are not limited to a linear regression algorithm, a logistic regression algorithm, a Naïve Bayes algorithm, a k-nearest neighbor (kNN) algorithm, a Random forest algorithm, decision tree algorithm, a support vector machine algorithm, a gradient boosting algorithm and a clustering algorithm. In certain embodiments, systems are configured to implement a neural network. In some instances, the machine learning algorithm is a neural network such as an artificial neural network, a convolutional neural network or a recurrent neural network. In certain instances, the system is configured to implement a python script.

In some embodiments, the memory includes instructions for determining one or more sorting gates for cells of sample, such as for classifying cells of the sample by assigning each cell in the sample to a particle population cluster. In some instances, the memory includes instructions for generating one or more sorting gates that capture cells of a target particle population cluster and exclude cells of a non-target particle population cluster. In some instances, the memory includes instructions for determining sorting gates that are configured to maximize the inclusion yield of the cells of a target particle population cluster, such as where the sorting gates are configured to generate an inclusion yield of cells of a target particle population cluster of 50% or more, such as 55% or more, such as 60% or more, such as 65% or more, such as 70% or more, such as 75% or more, such as 80% or more, such as 85% or more, such as 90% or more, such as 95% or more, such as 97% or more, such as 99% or more and including determining a sorting gate that is configured to generate an inclusion yield of cells of a target particle population cluster of 99.9% or more. In some instances, the memory includes instructions for determining sorting gates that are configured to maximize the purity yield of the cells of a target particle population cluster, such as where the sorting gates are configured to generate a purity yield of cells of a target particle population cluster of 50% or more, such as 55% or more, such as 60% or more, such as 65% or more, such as 70% or more, such as 75% or more, such as 80% or more, such as 85% or more, such as 90% or more, such as 95% or more, such as 97% or more, such as 99% or more and including determining a sorting gate that is configured to generate a purity yield of cells of a target particle population cluster of 99.9% or more.

In some instances, the memory includes instructions for determining sorting gates that are configured to maximize the exclusion of cells of a non-target particle population. In some instances, the memory includes instructions for determining sorting gates that are configured to exclude 50% or more of cells of non-target particle populations, such as 55% or more, such as 60% or more, such as 65% or more, such as 70% or more, such as 75% or more, such as 80% or more, such as 85% or more, such as 90% or more, such as 95% or more, such as 97% or more, such as 99% or more and including determining a sorting gate that is configured to exclude 99.9% or more of cells of a non-target particle population cluster. In some instances, the memory includes instructions for determining sorting gates that exclude cells of a non-target particle population cluster based on a calculated Mahalanobis distance from a target particle population cluster.

In certain embodiments, the memory includes instructions for generating the sorting gates using an Fβ score, where the Fβ score is a weighted harmonic mean of the inclusion of particles of a target particle population cluster and the exclusion of particles of a non-target particle population cluster. The beta (β) parameter of the Fβ score will bias the gating strategy toward either yield (e.g., increased inclusion of target particles) or purity (e.g., increased exclusion of non-target particles) of the sample. For example, where the Fβ score is equal to 1, the purity and yield have equal contributions to the gating strategy. Where the Fβ score is equal to 2, the gating strategy emphasizes yield over purity. Where the Fβ score is equal to 0.5, the gating strategy will emphasize purity over yield. In some embodiments, the Fβ score ranges from 0.1 to 10, such as from 0.2 to 9.5, such as from 0.3 to 9, such as from 0.4 to 8.5, such as from 0.5 to 8, such as from 0.5 to 7, such as from 0.5 to 6, such as from 0.5 to 5, such as from 0.5 to 4, such as from 0.5 to 3, such as from 0.5 to 2. In some instances, the generated sorting gates of interest have an Fβ score of 1 or more. In some instances, the generated sorting gates have an Fβ score of less than 1.

In some instances, the memory includes instructions for assessing the sorting gates of the gating strategy and adjusting one or more of the generated sorting gates. The adjustment may be made based on a metric indicating the accuracy of the sorting gates according to the desired sorting strategy. In some instances, the metric may be an accuracy (e.g., purity) metric where the purity is compared to a predetermined threshold. The metric may be generated based on a confidence of the classifiers included in the sort strategy. In other instances, the metric may be a yield metric where the yield of target cells of a particle population cluster is compared to a predetermined threshold.

In some embodiments, the memory includes instructions for generating a sorting decision based on the determined sorting gates for the cells of the sample. In some instances, the memory includes instructions for generating a particle sorting decision using a gating strategy of 8 sorting gates or less, such as 7 sorting gates or less, such as 6 sorting gates or less, such as 5 sorting gates or less, such as 4 sorting gates or less, such as 3 sorting gates or less and including 2 sorting gates or less. In some embodiments, the memory includes instructions for generating sorting gates using a graphical display that displays one or more analysis algorithms for applying the classification parameters to the image parameters determined for the cells.

In some embodiments, the memory includes instructions for generating sorting gates using a graphical display that displays one or more analysis algorithms for applying the determined classification parameters to the image parameters determined for the cells. In some embodiments, systems include a display for visualizing the gating strategy on a graphical user interface.

In some instances, the graphical user interface applies an analysis algorithm for generating the gating strategy. For example, the analysis algorithm may be one or more of a spectral compensation matrix, a clustering algorithm and a t-Distributed Stochastic Neighbor Embedding (t-SNE) algorithm. In some instances, the analysis algorithm is applied to the particle population cluster by dragging an icon of the analysis algorithm onto the particle population cluster. In other instances, the particle population cluster is selected and the analysis algorithm is applied by selecting from a drop-down menu. In certain instances, the analysis algorithm is a spectral unmixing algorithm, such as described in U.S. Patent No. 11,009,400 and International Patent Application No. PCT/US2021/46741 filed on August 19, 2021.

In some instances, systems include memory having instructions for determining the gating strategy using computational sorting algorithms such as described with in U.S. Patent No. 11,513,054. In certain instances, the system includes memory having computer software for determining the sorting gate such as HyperFinder (e.g., as described in Bonavia, et al. Frontiers in Immunology 2022; 13: 1007016) and Computational Sorting with HyperFinder, FlowJo Software and BD FACSDiva Software (Becton Dickinson, 2021). In certain embodiments, the gating strategy is developed on a separate computational system (e.g., a different computer system or network) and communicated to a flow cytometer for implementing the gating strategy, such as for example using a particle sorter (e.g, having a sort decision module) of the flow cytometer.

In some embodiments, systems for generating the gating strategy are part of or operationally coupled to a particle analyzer system (e.g., a flow cytometer) for generating flow cytometer data described herein.

In some instances, systems include an integrated circuit device programmed to implement one or more of the methods described above. In some instances, the integrated circuit includes programming for generating an image of the cells. In some instances, the integrated circuit includes programming for calculating an image parameter from the generated image of the cells. In some embodiments, integrated circuit devices of interest include a field programmable gate array (FPGA). In other embodiments, integrated circuit devices include an application specific integrated circuit (ASIC). In yet other embodiments, integrated circuit devices include a complex programmable logic device (CPLD).

Systems according to some embodiments, may include a display and operator input device. Operator input devices may, for example, be a keyboard, mouse, or the like. The processing module includes a processor which has access to a memory having instructions stored thereon for performing the steps of the subject methods. The processing module may include an operating system, a graphical user interface (GUI) controller, a system memory, memory storage devices, and input-output controllers, cache memory, a data backup unit, and many other devices. The processor may be a commercially available processor or it may be one of other processors that are or will become available. The processor executes the operating system and the operating system interfaces with firmware and hardware in a well-known manner, and facilitates the processor in coordinating and executing the functions of various computer programs that may be written in a variety of programming languages, such as Java, Perl, C++, other high level or low-level languages, as well as combinations thereof, as is known in the art. The operating system, typically in cooperation with the processor, coordinates and executes functions of the other components of the computer. The operating system also provides scheduling, input-output control, file and data management, memory management, and communication control and related services, all in accordance with known techniques. The processor may be any suitable analog or digital system. In some embodiments, the processor includes analog electronics which provide feedback control, such as for example negative feedback control.

The system memory may be any of a variety of known or future memory storage devices. Examples include any commonly available random-access memory (RAM), magnetic medium such as a resident hard disk or tape, an optical medium such as a read and write compact disc, flash memory devices, or other memory storage device. The memory storage device may be any of a variety of known or future devices, including a compact disk drive, a tape drive, a removable hard disk drive, or a diskette drive. Such types of memory storage devices typically read from, and/or write to, a program storage medium (not shown) such as, respectively, a compact disk, magnetic tape, removable hard disk, or floppy diskette. Any of these program storage media, or others now in use or that may later be developed, may be considered a computer program product. As will be appreciated, these program storage media typically store a computer software program and/or data. Computer software programs, also called computer control logic, typically are stored in system memory and/or the program storage device used in conjunction with the memory storage device.

In some embodiments, a computer program product is described comprising a computer usable medium having control logic (computer software program, including program code) stored therein. The control logic, when executed by the processor the computer, causes the processor to perform functions described herein. In other embodiments, some functions are implemented primarily in hardware using, for example, a hardware state machine. Implementation of the hardware state machine so as to perform the functions described herein will be apparent to those skilled in the relevant arts.

Memory may be any suitable device in which the processor can store and retrieve data, such as magnetic, optical, or solid-state storage devices (including magnetic or optical disks or tape or RAM, or any other suitable device, either fixed or portable). The processor may include a general-purpose digital microprocessor suitably programmed from a computer readable medium carrying necessary program code. Programming can be provided remotely to processor through a communication channel, or previously saved in a computer program product such as memory or some other portable or fixed computer readable storage medium using any of those devices in connection with memory. For example, a magnetic or optical disk may carry the programming, and can be read by a disk writer/reader. Systems of the invention also include programming, e.g., in the form of computer program products, algorithms for use in practicing the methods as described above. Programming according to the present invention can be recorded on computer readable media, e.g., any medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; portable flash drive; and hybrids of these categories such as magnetic/optical storage media.

The processor may also have access to a communication channel to communicate with a user at a remote location. By remote location is meant the user is not directly in contact with the system and relays input information to an input manager from an external device, such as a computer connected to a Wide Area Network ("WAN"), telephone network, satellite network, or any other suitable communication channel, including a mobile telephone (i.e., smartphone).

In some embodiments, systems according to the present disclosure may be configured to include a communication interface. In some embodiments, the communication interface includes a receiver and/or transmitter for communicating with a network and/or another device. The communication interface can be configured for wired or wireless communication, including, but not limited to, radio frequency (RF) communication (e.g., Radio-Frequency Identification (RFID), Zigbee communication protocols, WiFi, infrared, wireless Universal Serial Bus (USB), Ultra-Wide Band (UWB), Bluetooth^{®} communication protocols, and cellular communication, such as code division multiple access (CDMA) or Global System for Mobile communications (GSM).

In one embodiment, the communication interface is configured to include one or more communication ports, e.g., physical ports or interfaces such as a USB port, an RS-232 port, or any other suitable electrical connection port to allow data communication between the subject systems and other external devices such as a computer terminal (for example, at a physician's office or in hospital environment) that is configured for similar complementary data communication.

In one embodiment, the communication interface is configured for infrared communication, Bluetooth^{®} communication, or any other suitable wireless communication protocol to enable the subject systems to communicate with other devices such as computer terminals and/or networks, communication enabled mobile telephones, personal digital assistants, or any other communication devices which the user may use in conjunction.

In one embodiment, the communication interface is configured to provide a connection for data transfer utilizing Internet Protocol (IP) through a cell phone network, Short Message Service (SMS), wireless connection to a personal computer (PC) on a Local Area Network (LAN) which is connected to the internet, or WiFi connection to the internet at a WiFi hotspot.

In one embodiment, the subject systems are configured to wirelessly communicate with a server device via the communication interface, e.g., using a common standard such as 802.11 or Bluetooth^{®} RF protocol, or an IrDA infrared protocol. The server device may be another portable device, such as a smart phone, Personal Digital Assistant (PDA) or notebook computer; or a larger device such as a desktop computer, appliance, etc. In some embodiments, the server device has a display, such as a liquid crystal display (LCD), as well as an input device, such as buttons, a keyboard, mouse or touch-screen.

In some embodiments, the communication interface is configured to automatically or semi-automatically communicate data stored in the subject systems, e.g., in an optional data storage unit, with a network or server device using one or more of the communication protocols and/or mechanisms described above.

Output controllers may include controllers for any of a variety of known display devices for presenting information to a user, whether a human or a machine, whether local or remote. If one of the display devices provides visual information, this information typically may be logically and/or physically organized as an array of picture elements. A graphical user interface (GUI) controller may include any of a variety of known or future software programs for providing graphical input and output interfaces between the system and a user, and for processing user inputs. The functional elements of the computer may communicate with each other via system bus. Some of these communications may be accomplished in alternative embodiments using network or other types of remote communications. The output manager may also provide information generated by the processing module to a user at a remote location, e.g., over the Internet, phone or satellite network, in accordance with known techniques. The presentation of data by the output manager may be implemented in accordance with a variety of known techniques. As some examples, data may include SQL, HTML or XML documents, email or other files, or data in other forms. The data may include Internet URL addresses so that a user may retrieve additional SQL, HTML, XML, or other documents or data from remote sources. The one or more platforms present in the subject systems may be any type of known computer platform or a type to be developed in the future, although they typically will be of a class of computer commonly referred to as servers. However, they may also be a main-frame computer, a work station, or other computer type. They may be connected via any known or future type of cabling or other communication system including wireless systems, either networked or otherwise. They may be co-located or they may be physically separated. Various operating systems may be employed on any of the computer platforms, possibly depending on the type and/or make of computer platform chosen. Appropriate operating systems include Windows 10, Windows NT^{®}, Windows XP, Windows 7, Windows 8, iOS, Sun Solaris, Linux, OS/400, Compaq Tru64 Unix, SGI IRIX, Siemens Reliant Unix, Ubuntu, Zorin OS and others.

In certain embodiments, the subject systems include one or more optical adjustment components for adjusting the light such as light irradiated onto the sample (e.g., from a laser) or light collected from the sample (e.g., scattered, fluorescence). For example, the optical adjustment may be to increase the dimensions of the light, the focus of the light or to collimate the light. In some instances, optical adjustment is a magnification protocol so as to increase the dimensions of the light (e.g., beam spot), such as increasing the dimensions by 5% or more, such as by 10% or more, such as by 25% or more, such as by 50% or more and including increasing the dimensions by 75% or more. In other embodiments, optical adjustment includes focusing the light so as to reduce the light dimensions, such as by 5% or greater, such as by 10% or greater, such as by 25% or greater, such as by 50% or greater and including reducing the dimensions of the beam spot by 75% or greater. In certain embodiments, optical adjustment includes collimating the light. The term "collimate" is used in its conventional sense to refer to the optically adjusting the collinearity of light propagation or reducing divergence by the light of from a common axis of propagation. In some instances, collimating includes narrowing the spatial cross section of a light beam (e.g., reducing the beam profile of a laser)

In some embodiments, the optical adjustment component is a focusing lens having a magnification ratio of from 0.1 to 0.95, such as a magnification ratio of from 0.2 to 0.9, such as a magnification ratio of from 0.3 to 0.85, such as a magnification ratio of from 0.35 to 0.8, such as a magnification ratio of from 0.5 to 0.75 and including a magnification ratio of from 0.55 to 0.7, for example a magnification ratio of 0.6. For example, the focusing lens is, in certain instances, a double achromatic de-magnifying lens having a magnification ratio of about 0.6. The focal length of the focusing lens may vary, ranging from 5 mm to 20 mm, such as from 6 mm to 19 mm, such as from 7 mm to 18 mm, such as from 8 mm to 17 mm, such as from 9 mm to 16 and including a focal length ranging from 10 mm to 15 mm. In certain embodiments, the focusing lens has a focal length of about 13 mm.

In other embodiments, the optical adjustment component is a collimator. The collimator may be any convenient collimating protocol, such as one or more mirrors or curved lenses or a combination thereof. For example, the collimator is in certain instances a single collimating lens. In other instances, the collimator is a collimating mirror. In yet other instances, the collimator includes two lenses. In still other instances, the collimator includes a mirror and a lens. Where the collimator includes one or more lenses, the focal length of the collimating lens may vary, ranging from 5 mm to 40 mm, such as from 6 mm to 37.5 mm, such as from 7 mm to 35 mm, such as from 8 mm to 32.5 mm, such as from 9 mm to 30 mm, such as from 10 mm to 27.5 mm, such as from 12.5 mm to 25 mm and including a focal length ranging from 15 mm to 20 mm.

In some embodiments, the subject systems include a flow cell nozzle having a nozzle orifice configured to flow a flow stream through the flow cell nozzle. The subject flow cell nozzle has an orifice which propagates a fluidic sample to a sample interrogation region, where in some embodiments, the flow cell nozzle includes a proximal cylindrical portion defining a longitudinal axis and a distal frustoconical portion which terminates in a flat surface having the nozzle orifice that is transverse to the longitudinal axis. The length of the proximal cylindrical portion (as measured along the longitudinal axis) may vary ranging from 1 mm to 15 mm, such as from 1.5 mm to 12.5 mm, such as from 2 mm to 10 mm, such as from 3 mm to 9 mm and including from 4 mm to 8 mm. The length of the distal frustoconical portion (as measured along the longitudinal axis) may also vary, ranging from 1 mm to 10 mm, such as from 2 mm to 9 mm, such as from 3 mm to 8 mm and including from 4 mm to 7 mm. The diameter of the of the flow cell nozzle chamber may vary, in some embodiments, ranging from 1 mm to 10 mm, such as from 2 mm to 9 mm, such as from 3 mm to 8 mm and including from 4 mm to 7 mm.

In certain instances, the nozzle chamber does not include a cylindrical portion and the entire flow cell nozzle chamber is frustoconically shaped. In these embodiments, the length of the frustoconical nozzle chamber (as measured along the longitudinal axis transverse to the nozzle orifice), may range from 1 mm to 15 mm, such as from 1.5 mm to 12.5 mm, such as from 2 mm to 10 mm, such as from 3 mm to 9 mm and including from 4 mm to 8 mm. The diameter of the proximal portion of the frustoconical nozzle chamber may range from 1 mm to 10 mm, such as from 2 mm to 9 mm, such as from 3 mm to 8 mm and including from 4 mm to 7 mm.

In embodiments, the sample flow stream emanates from an orifice at the distal end of the flow cell nozzle. Depending on the desired characteristics of the flow stream, the flow cell nozzle orifice may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. In certain embodiments, flow cell nozzle of interest has a circular orifice. The size of the nozzle orifice may vary, in some embodiments ranging from 1 µm to 20000 µm, such as from 2 µm to 17500 µm, such as from 5 µm to 15000 µm, such as from 10 µm to 12500 µm, such as from 15 µm to 10000 µm, such as from 25 µm to 7500 µm, such as from 50 µm to 5000 µm, such as from 75 µm to 1000 µm, such as from 100 µm to 750 µm and including from 150 µm to 500 µm. In certain embodiments, the nozzle orifice is 100 µm.

In some embodiments, the flow cell nozzle includes a sample injection port configured to provide a sample to the flow cell nozzle. In embodiments, the sample injection system is configured to provide suitable flow of sample to the flow cell nozzle chamber. Depending on the desired characteristics of the flow stream, the rate of sample conveyed to the flow cell nozzle chamber by the sample injection port may be1 µL/sec or more, such as 2 µL/sec or more, such as 3 µL/sec or more, such as 5 µL/sec or more, such as 10 µL/sec or more, such as 15 µL/sec or more, such as 25 µL/sec or more, such as 50 µL/sec or more, such as 100 µL/sec or more, such as 150 µL/sec or more , such as 200 µL/sec or more, such as 250 µL/sec or more, such as 300 µL/sec or more, such as 350 µL/sec or more, such as 400 µL/sec or more, such as 450 µL/sec or more and including 500 µL/sec or more. For example, the sample flow rate may range from 1 µL/sec to about 500 µL/sec, such as from 2 µL/sec to about 450 µL/sec, such as from 3 µL/sec to about 400 µL/sec, such as from 4 µL/sec to about 350 µL/sec, such as from 5 µL/sec to about 300 µL/sec, such as from 6 µL/sec to about 250 µL/sec, such as from 7 µL/sec to about 200 µL/sec, such as from 8 µL/sec to about 150 µL/sec, such as from 9 µL/sec to about 125 µL/sec and including from 10 µL/sec to about 100 µL/sec.

The sample injection port may be an orifice positioned in a wall of the nozzle chamber or may be a conduit positioned at the proximal end of the nozzle chamber. Where the sample injection port is an orifice positioned in a wall of the nozzle chamber, the sample injection port orifice may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, etc., as well as irregular shapes, e.g., a parabolic bottom portion coupled to a planar top portion. In certain embodiments, the sample injection port has a circular orifice. The size of the sample injection port orifice may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm.

In certain instances, the sample injection port is a conduit positioned at a proximal end of the flow cell nozzle chamber. For example, the sample injection port may be a conduit positioned to have the orifice of the sample injection port in line with the flow cell nozzle orifice. Where the sample injection port is a conduit positioned in line with the flow cell nozzle orifice, the cross-sectional shape of the sample injection tube may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The orifice of the conduit may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm. The shape of the tip of the sample injection port may be the same or different from the cross-section shape of the sample injection tube. For example, the orifice of the sample injection port may include a beveled tip having a bevel angle ranging from 1° to 10°, such as from 2° to 9°, such as from 3° to 8°, such as from 4° to 7° and including a bevel angle of 5°.

In some embodiments, the flow cell nozzle also includes a sheath fluid injection port configured to provide a sheath fluid to the flow cell nozzle. In embodiments, the sheath fluid injection system is configured to provide a flow of sheath fluid to the flow cell nozzle chamber, for example in conjunction with the sample to produce a laminated flow stream of sheath fluid surrounding the sample flow stream. Depending on the desired characteristics of the flow stream, the rate of sheath fluid conveyed to the flow cell nozzle chamber by the may be 25µL/sec or more, such as 50 µL/sec or more, such as 75 µL/sec or more, such as 100 µL/sec or more, such as 250 µL/sec or more, such as 500 µL/sec or more, such as 750 µL/sec or more, such as 1000 µL/sec or more and including 2500 µL/sec or more. For example, the sheath fluid flow rate may range from 1 µL/sec to about 500 µL/sec, such as from 2 µL/sec to about 450 µL/sec, such as from 3 µL/sec to about 400 µL/sec, such as from 4 µL/sec to about 350 µL/sec, such as from 5 µL/sec to about 300 µL/sec, such as from 6 µL/sec to about 250 µL/sec, such as from 7 µL/sec to about 200 µL/sec, such as from 8 µL/sec to about 150 µL/sec, such as from 9 µL/sec to about 125 µL/sec and including from 10 µL/sec to about 100 µL/sec.

In some embodiments, the sheath fluid injection port is an orifice positioned in a wall of the nozzle chamber. The sheath fluid injection port orifice may be any suitable shape where cross-sectional shapes of interest include, but are not limited to: rectilinear cross sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The size of the sample injection port orifice may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm.

The subject systems, in certain instances, include a sample interrogation region in fluid communication with the flow cell nozzle orifice. In these instances, a sample flow stream emanates from an orifice at the distal end of the flow cell nozzle and particles in the flow stream may be irradiated with a light source at the sample interrogation region. The size of the interrogation region may vary depending on the properties of the flow nozzle, such as the size of the nozzle orifice and sample injection port size. In embodiments, the interrogation region may have a width that is 0.01 mm or more, such as 0.05 mm or more, such as 0.1 mm or more, such as 0.5 mm or more, such as 1 mm or more, such as 2 mm or more, such as 3 mm or more, such as 5 mm or more and including 10 mm or more. The length of the interrogation region may also vary, ranging in some instances along 0.01 mm or more, such as 0.1 mm or more, such as 0.5 mm or more, such as 1 mm or more, such as 1.5 mm or more, such as 2 mm or more, such as 3 mm or more, such as 5 mm or more, such as 10 or more, such as 15 mm or more, such as 20 mm or more, such as 25 mm or more and including 50 mm or more.

The interrogation region may be configured to facilitate irradiation of a planar cross-section of an emanating flow stream or may be configured to facilitate irradiation of a diffuse field (e.g., with a diffuse laser or lamp) of a predetermined length. In some embodiments, the interrogation region includes a transparent window that facilitates irradiation of a predetermined length of an emanating flow stream, such as 1 mm or more, such as 2 mm or more, such as 3 mm or more, such as 4 mm or more, such as 5 mm or more and including 10 mm or more. Depending on the light source used to irradiate the emanating flow stream (as described below), the interrogation region may be configured to pass light that ranges from 100 nm to 1500 nm, such as from 150 nm to 1400 nm, such as from 200 nm to 1300 nm, such as from 250 nm to 1200 nm, such as from 300 nm to 1100 nm, such as from 350 nm to 1000 nm, such as from 400 nm to 900 nm and including from 500 nm to 800 nm. As such, the interrogation region may be formed from any transparent material which passes the desired range of wavelength, including but not limited to optical glass, borosilicate glass, Pyrex glass, ultraviolet quartz, infrared quartz, sapphire as well as plastic, such as polycarbonates, polyvinyl chloride (PVC), polyurethanes, polyethers, polyamides, polyimides, or copolymers of these thermoplastics, such as PETG (glycol-modified polyethylene terephthalate), among other polymeric plastic materials, including polyester, where polyesters of interest may include, but are not limited to poly(alkylene terephthalates) such as poly(ethylene terephthalate) (PET), bottle-grade PET (a copolymer made based on monoethylene glycol, terephthalic acid, and other comonomers such as isophthalic acid, cyclohexene dimethanol, etc.), poly(butylene terephthalate) (PBT), and poly(hexamethylene terephthalate); poly(alkylene adipates) such as poly(ethylene adipate), poly(1,4-butylene adipate), and poly(hexamethylene adipate); poly(alkylene suberates) such as poly(ethylene suberate); poly(alkylene sebacates) such as poly(ethylene sebacate); poly(ε-caprolactone) and poly(β-propiolactone); poly(alkylene isophthalates) such as poly(ethylene isophthalate); poly(alkylene 2,6-naphthalene-dicarboxylates) such as poly(ethylene 2,6-naphthalenedicarboxylate); poly(alkylene sulfonyl-4,4'-dibenzoates) such as poly(ethylene sulfonyl-4,4'-dibenzoate); poly(p-phenylene alkylene dicarboxylates) such as poly(p-phenylene ethylene dicarboxylates); poly(trans-1,4-cyclohexanediyl alkylene dicarboxylates) such as poly(trans-1,4-cyclohexanediyl ethylene dicarboxylate); poly(1,4-cyclohexane-dimethylene alkylene dicarboxylates) such as poly(1,4-cyclohexane-dimethylene ethylene dicarboxylate); poly([2.2.2]-bicyclooctane-1,4-dimethylene alkylene dicarboxylates) such as poly([2.2.2]-bicyclooctane-1,4-dimethylene ethylene dicarboxylate); lactic acid polymers and copolymers such as (S)-polylactide, (R,S)-polylactide, poly(tetramethylglycolide), and poly(lactide-co-glycolide); and polycarbonates of bisphenol A, 3,3'-dimethylbisphenol A, 3,3',5,5'-tetrachlorobisphenol A, 3,3',5,5'-tetramethylbisphenol A; polyamides such as poly(p-phenylene terephthalamide); polyesters, e.g., polyethylene terephthalates, e.g., Mylar^{™} polyethylene terephthalate; etc. In some embodiments, the subject systems include a cuvette positioned in the sample interrogation region. In embodiments, the cuvette may pass light that ranges from 100 nm to 1500 nm, such as from 150 nm to 1400 nm, such as from 200 nm to 1300 nm, such as from 250 nm to 1200 nm, such as from 300 nm to 1100 nm, such as from 350 nm to 1000 nm, such as from 400 nm to 900 nm and including from 500 nm to 800 nm.

In certain embodiments, light detection systems having the plurality of photodetectors as described above are part of or positioned in a particle analyzer, such as a particle sorter. In certain embodiments, the subject systems are flow cytometric systems that includes the photodiode and amplifier component as part of a light detection system for detecting light emitted by a sample in a flow stream. Suitable flow cytometry systems may include, but are not limited to, those described in Ormerod (ed.), Flow Cytometry: A Practical Approach, Oxford Univ. Press (1997); Jaroszeski et al. (eds.), Flow Cytometry Protocols, Methods in Molecular Biology No. 91, Humana Press (1997); Practical Flow Cytometry, 3rd ed., Wiley-Liss (1995); Virgo, et al. (2012) Ann Clin Biochem. Jan;49(pt 1):17-28; Linden, et. al., Semin Throm Hemost. 2004 Oct;30(5):502-11; Alison, et al. J Pathol, 2010 Dec; 222(4):335-344; and Herbig, et al. (2007) Crit Rev Ther Drug Carrier Syst. 24(3):203-255. In certain instances, flow cytometry systems of interest include BD Biosciences FACSCanto^{™} flow cytometer, BD Biosciences FACSCanto^{™} II flow cytometer, BD Accuri^{™} flow cytometer, BD Accuri^{™} C6 Plus flow cytometer, BD Biosciences FACSCelesta^{™} flow cytometer, BD Biosciences FACSLyric^{™} flow cytometer, BD Biosciences FACSVerse^{™} flow cytometer, BD Biosciences FACSymphony^{™} flow cytometer, BD Biosciences LSRFortessa^{™} flow cytometer, BD Biosciences LSRFortessa^{™} X-20 flow cytometer, BD Biosciences FACSPresto^{™} flow cytometer, BD Biosciences FACSVia^{™} flow cytometer and BD Biosciences FACSCalibur^{™} cell sorter, a BD Biosciences FACSCount^{™} cell sorter, BD Biosciences FACSLyric^{™} cell sorter, BD Biosciences Via^{™} cell sorter, BD Biosciences Influx^{™} cell sorter, BD Biosciences Jazz^{™} cell sorter, BD Biosciences Aria^{™} cell sorter, BD Biosciences FACSAria^{™} II cell sorter, BD Biosciences FACSAria^{™} III cell sorter, BD Biosciences FACSAria^{™} Fusion cell sorter and BD Biosciences FACSMelody^{™} cell sorter, BD Biosciences FACSymphony^{™} S6 cell sorter or the like.

In some embodiments, the subject systems are flow cytometric systems, such those described in U.S. Patent Nos. 10,663,476; 10,620,111; 10,613,017; 10,605,713; 10,585,031; 10,578,542; 10,578,469; 10,481,074; 10,302,545; 10,145,793; 10,113,967; 10,006,852; 9,952,076; 9,933,341; 9,726,527; 9,453,789; 9,200,334; 9,097,640; 9,095,494; 9,092,034; 8,975,595; 8,753,573; 8,233,146; 8,140,300; 7,544,326; 7,201,875; 7,129,505; 6,821,740; 6,813,017; 6,809,804; 6,372,506; 5,700,692; 5,643,796; 5,627,040; 5,620,842; 5,602,039; 4,987,086; 4,498,766.

In some embodiments, systems include a particle sorter component. The term "sorting" is used herein in its conventional sense to refer to separating components (e.g., target cells with mitochondria morphology of interest, non-target cells with mitochondria morphology that is not of interest, non-cellular particles such as biological macromolecules) of the sample and in some instances delivering the separated components to one or more sample collection containers. For example, the subject systems may be configured for sorting samples having 2 or more components, such as 3 or more components, such as 4 or more components, such as 5 or more components, such as 10 or more components, such as 15 or more components and including soring a sample having 25 or more components. One or more of the sample components may be separated from the sample and delivered to a sample collection container, such as 2 or more sample components, such as 3 or more sample components, such as 4 or more sample components, such as 5 or more sample components, such as 10 or more sample components and including 15 or more sample components may be separated from the sample and delivered to a sample collection container.

In some embodiments, particle sorting systems of interest are configured to sort particles with an enclosed particle sorting module, such as those described in U.S. Patent Publication No. 2017/0299493, filed on March 28, 2017. In certain embodiments, particles (e.g., cells) of the sample are sorted using a sort decision module having a plurality of sort decision units, such as those described in U.S. Patent Publication No. 2020/0256781. In some embodiments, the subject systems include a particle sorting module having deflector plates, such as described in U.S. Patent Publication No. 2017/0299493, filed on March 28, 2017.

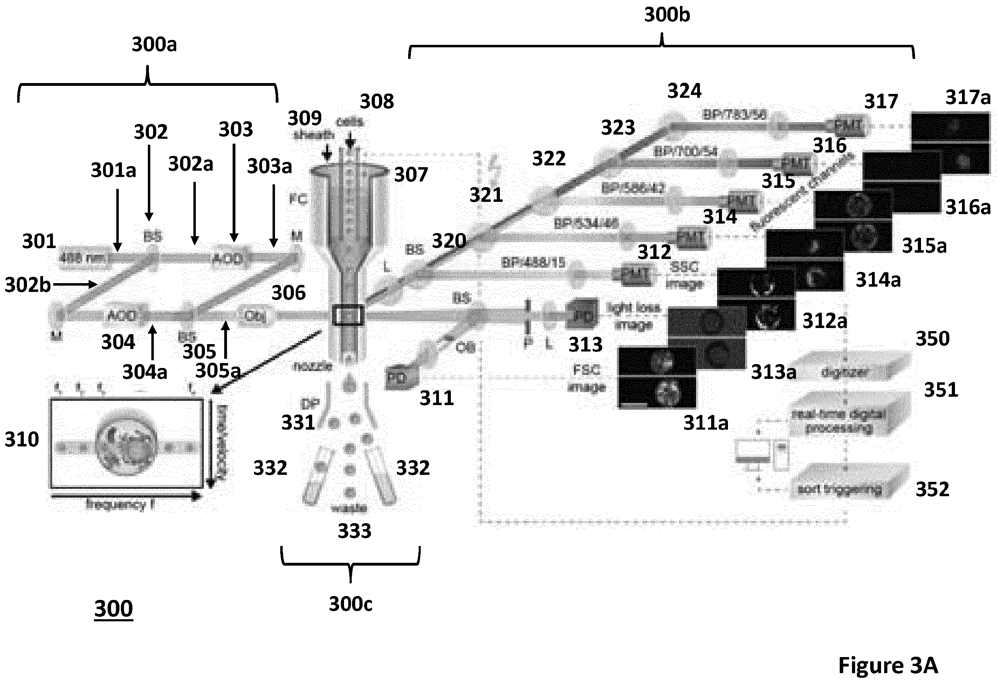

In certain embodiments, systems are an image-enabled particle sorter using frequency-encoded data, such as depicted in FIG. 3A. Particle sorter 300 includes a light irradiation component 300a which includes light source 301 (e.g., 488 nm laser) which generates output beam of light 301a that is split with beamsplitter 302 into beams 302a and 302b. Light beam 302a is propagated through acousto-optic device (e.g., an acousto-optic deflector, AOD) 303 to generate an output beam 303a having one or more angularly deflected beams of light. In some instances, output beam 303a generated from acousto-optic device 303 includes a local oscillator beam and a plurality of radiofrequency comb beams. Light beam 302b is propagated through acousto-optic device (e.g., an acousto-optic deflector, AOD) 304 to generate an output beam 304a having one or more angularly deflected beams of light. In some instances, output beam 304a generated from acousto-optic device 304 includes a local oscillator beam and a plurality of radiofrequency comb beams. Output beams 303a and 304a generated from acousto-optic devices 303 and 304, respectively are combined with beamsplitter 305 to generate output beam 305a which is conveyed through an optical component 306 (e.g., an objective lens) to irradiate particles in flow cell 307. In certain embodiments, acousto-optic device 303 (AOD) splits a single laser beam into an array of beamlets, each having different optical frequency and angle. Second AOD 304 tunes the optical frequency of a reference beam, which is then overlapped with the array of beamlets at beam combiner 305. In certain embodiments, the light irradiation system having a light source and acousto-optic device can also include those described in Schraivogel, et al. ("High-speed fluorescence image-enabled cell sorting" Science (2022), 375 (6578): 315-320) and United States Patent Publication No. 2021/0404943.

Output beam 305a irradiates sample particles 308 propagating through flow cell 307 (e.g., with sheath fluid 309) at irradiation region 310. As shown in irradiation region 310, a plurality of beams (e.g., angularly deflected radiofrequency shifted beams of light depicted as dots across irradiation region 310) overlaps with a reference local oscillator beam (depicted as the shaded line across irradiation region 310). Due to their differing optical frequencies, the overlapping beams exhibit a beating behavior, which causes each beamlet to carry a sinusoidal modulation at a distinct frequency f₁₋ₙ.

Light from the irradiated sample is conveyed to light detection system 300b that includes a plurality of photodetectors. Light detection system 300b includes forward scattered light photodetector 311 for generating forward scatter images 311a and a side scattered light photodetector 312 for generating side scatter images 312a. Light detection system 300b also includes brightfield photodetector 313 for generating light loss images 313a. In some embodiments, forward scatter detector 311 and side scatter detector 312 are photodiodes (e.g., avalanche photodiodes, APDs). In some instances, brightfield photodetector 313 is a photomultiplier tube (PMT). Fluorescence from the irradiated sample is also detected with fluorescence photodetectors 314-317. In some instances, photodetectors 314-317 are photomultiplier tubes. Light from the irradiated sample is directed to the side scatter detection channel 312 and fluorescence detection channels 314-317 through beamsplitter 320. Light detection system 300b includes bandpass optical components 321, 322, 323 and 324 (e.g., dichroic mirrors) for propagating predetermined wavelength of light to photodetectors 314-317. In some instances, optical component 321 is a 534 nm/40 nm bandpass. In some instances, optical component 322 is a 586 nm/42 nm bandpass. In some instances, optical component 323 is a 700 nm/54 nm bandpass. In some instances, optical component 324 is a 783 nm/56 nm bandpass. The first number represents the center of a spectral band. The second number provides a range of the spectral band. Thus, a 510/20 filter extends 10 nm on each side of the center of the spectral band, or from 500 nm to 520 nm.

Data signals generated in response to light detected in scattered light detection channels 311 and 312, brightfield light detection channel 313 and fluorescence detection channels 314-317 are processed by real-time digital processing with processors 350 and 351. Images 311a-317a can be generated in each light detection channel based on the data signals generated in processors 350 and 351. Image-enabled sorting is performed in response to a sort signal generated in sort trigger 352. Sorting component 300c includes deflection plates 331 for deflecting particles into sample containers 332 or to waste stream 333. In some instances, sort component 300c is configured to sort particles with an enclosed particle sorting module, such as those described in U.S. Patent Publication No. 2017/0299493, filed on March 28, 2017. In certain embodiments, sorting component 300c includes a sort decision module having a plurality of sort decision units, such as those described in U.S. Patent Publication No. 2020/0256781.

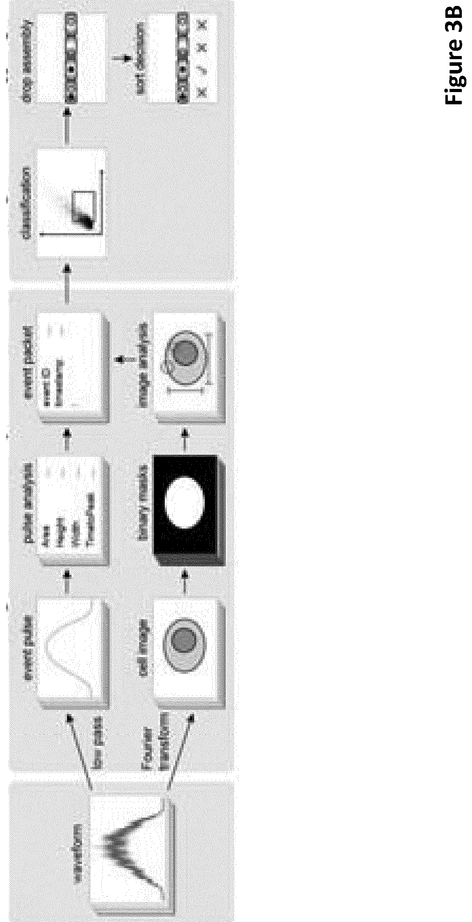

Figure 3B depicts image-enabled particle sorting data processing according to certain embodiments. In some instances, image-enabled particle sorting data processing is a low-latency data processing pipeline. Each photodetector produces a pulse with high-frequency modulations encoding the image (waveform). Fourier analysis is performed to reconstruct the image from the modulated pulse. An image processing pipeline produces a set of image features (image analysis), which are combined with features derived from a pulse processing pipeline (event packet). Real-time sort classification electronics then classify the particle based on image features, producing a sort decision that is used to selectively charge the droplets.

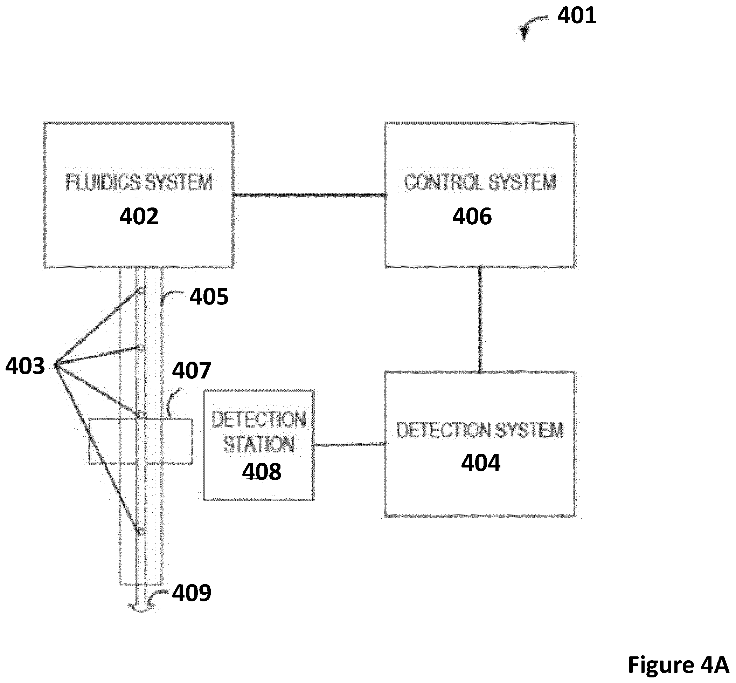

In some embodiments, systems are particle analyzers where the particle analysis system 401 (FIG. 4A) can be used to analyze and characterize particles, with or without physically sorting the particles into collection vessels. FIG. 4A shows a functional block diagram of a particle analysis system for computational based sample analysis and particle characterization. In some embodiments, the particle analysis system 401 is a flow system. The particle analysis system 401 shown in FIG. 4A can be configured to perform, in whole or in part, the methods described herein such as. The particle analysis system 401 includes a fluidics system 402. The fluidics system 402 can include or be coupled with a sample tube 405 and a moving fluid column within the sample tube in which particles 403 (e.g. cells) of a sample move along a common sample path 409.

The particle analysis system 401 includes a detection system 404 configured to collect a signal from each particle as it passes one or more detection stations along the common sample path. A detection station 408 generally refers to a monitored area 407 of the common sample path. Detection can, in some implementations, include detecting light or one or more other properties of the particles 403 as they pass through a monitored area 407. In FIG. 4A, one detection station 408 with one monitored area 407 is shown. Some implementations of the particle analysis system 401 can include multiple detection stations. Furthermore, some detection stations can monitor more than one area.

Each signal is assigned a signal value to form a data point for each particle. As described above, this data can be referred to as event data. The data point can be a multidimensional data point including values for respective properties measured for a particle. The detection system 404 is configured to collect a succession of such data points in a first-time interval.

The particle analysis system 401 can also include a control system 306. The control system 406 can include one or more processors, an amplitude control circuit and/or a frequency control circuit. The control system shown can be operationally associated with the fluidics system 402. The control system can be configured to generate a calculated signal frequency for at least a portion of the first-time interval based on a Poisson distribution and the number of data points collected by the detection system 404 during the first time interval. The control system 406 can be further configured to generate an experimental signal frequency based on the number of data points in the portion of the first time interval. The control system 406 can additionally compare the experimental signal frequency with that of a calculated signal frequency or a predetermined signal frequency.

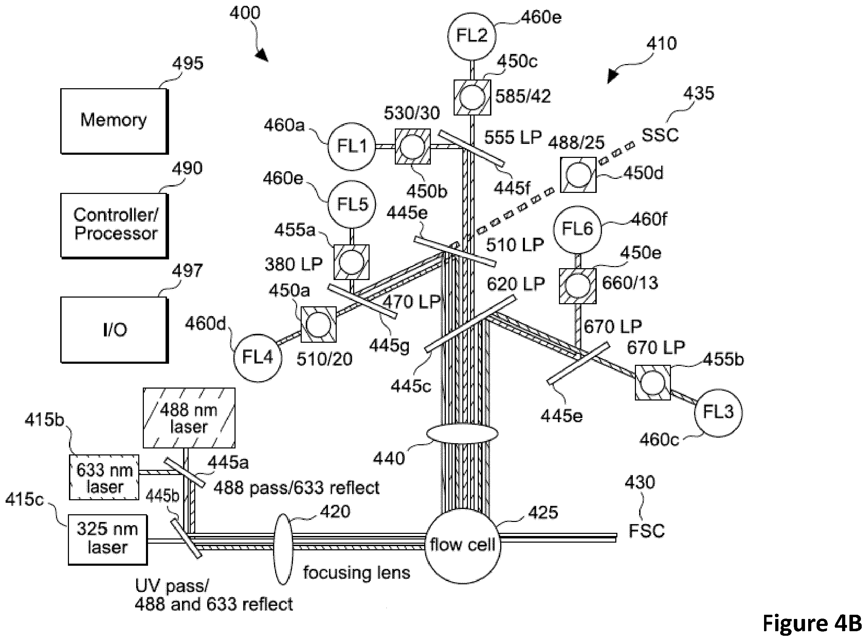

FIG. 4B shows a system 400 for flow cytometry in accordance with an illustrative embodiment of the present invention. The system 400 includes a flow cytometer 410, a controller/processor 490 and a memory 495. The flow cytometer 410 includes one or more excitation lasers 415a-415c, a focusing lens 420, a flow chamber 425, a forward scatter detector 430, a side scatter detector 435, a fluorescence collection lens 440, one or more beam splitters 445a-445g, one or more bandpass filters 450a-450e, one or more longpass ("LP") filters 455a-455b, and one or more fluorescent detectors 460a-460f.

The excitation lasers 115a-c emit light in the form of a laser beam. The wavelengths of the laser beams emitted from excitation lasers 415a-415c are 488 nm, 633 nm, and 325 nm, respectively, in the example system of FIG. 4B. The laser beams are first directed through one or more of beam splitters 445a and 445b. Beam splitter 445a transmits light at 488 nm and reflects light at 633 nm. Beam splitter 445b transmits UV light (light with a wavelength in the range of 10 to 400 nm) and reflects light at 488 nm and 633 nm.

The laser beams are then directed to a focusing lens 420, which focuses the beams onto the portion of a fluid stream where particles of a sample are located, within the flow chamber 425. The flow chamber is part of a fluidics system which directs particles, typically one at a time, in a stream to the focused laser beam for interrogation. The flow chamber can comprise a flow cell in a benchtop cytometer or a nozzle tip in a stream-in-air cytometer.

The light from the laser beam(s) interacts with the particles in the sample by diffraction, refraction, reflection, scattering, and absorption with re-emission at various different wavelengths depending on the characteristics of the particle such as its size, internal structure, and the presence of one or more fluorescent molecules attached to or naturally present on or in the particle. The fluorescence emissions as well as the diffracted light, refracted light, reflected light, and scattered light may be routed to one or more of the forward scatter detector 430, the side scatter detector 435, and the one or more fluorescent detectors 460a-460f through one or more of the beam splitters 445a-445g, the bandpass filters 450a-450e, the longpass filters 455a-455b, and the fluorescence collection lens 440.

The fluorescence collection lens 440 collects light emitted from the particle- laser beam interaction and routes that light towards one or more beam splitters and filters. Bandpass filters, such as bandpass filters 450a-450e, allow a narrow range of wavelengths to pass through the filter. For example, bandpass filter 450a is a 510/20 filter. The first number represents the center of a spectral band. The second number provides a range of the spectral band. Thus, a 510/20 filter extends 10 nm on each side of the center of the spectral band, or from 500 nm to 520 nm. Shortpass filters transmit wavelengths of light equal to or shorter than a specified wavelength. Longpass filters, such as longpass filters 455a-455b, transmit wavelengths of light equal to or longer than a specified wavelength of light. For example, longpass filter 455a, which is a 670 nm longpass filter, transmits light equal to or longer than 670 nm. Filters are often selected to optimize the specificity of a detector for a particular fluorescent dye. The filters can be configured so that the spectral band of light transmitted to the detector is close to the emission peak of a fluorescent dye.

Beam splitters direct light of different wavelengths in different directions. Beam splitters can be characterized by filter properties such as shortpass and longpass. For example, beam splitter 445g is a 620 SP beam splitter, meaning that the beam splitter 445g transmits wavelengths of light that are 620 nm or shorter and reflects wavelengths of light that are longer than 620 nm in a different direction. In one embodiment, the beam splitters 445a-445g can comprise optical mirrors, such as dichroic mirrors.

The forward scatter detector 430 is positioned slightly off axis from the direct beam through the flow cell and is configured to detect diffracted light, the excitation light that travels through or around the particle in mostly a forward direction. The intensity of the light detected by the forward scatter detector is dependent on the overall size of the particle. The forward scatter detector can include a photodiode. The side scatter detector 435 is configured to detect refracted and reflected light from the surfaces and internal structures of the particle, and tends to increase with increasing particle complexity of structure. The fluorescence emissions from fluorescent molecules associated with the particle can be detected by the one or more fluorescent detectors 460a-460f. The side scatter detector 435 and fluorescent detectors can include photomultiplier tubes. The signals detected at the forward scatter detector 430, the side scatter detector 435 and the fluorescent detectors can be converted to electronic signals (voltages) by the detectors. This data can provide information about the sample.

One of skill in the art will recognize that a flow cytometer in accordance with an embodiment of the present invention is not limited to the flow cytometer depicted in FIG. 4B, but can include any flow cytometer known in the art. For example, a flow cytometer may have any number of lasers, beam splitters, filters, and detectors at various wavelengths and in various different configurations.

In operation, cytometer operation is controlled by a controller/processor 490, and the measurement data from the detectors can be stored in the memory 495 and processed by the controller/processor 490. Although not shown explicitly, the controller/processor 190 is coupled to the detectors to receive the output signals therefrom, and may also be coupled to electrical and electromechanical components of the flow cytometer 400 to control the lasers, fluid flow parameters, and the like. Input/output (I/O) capabilities 497 may be provided also in the system. The memory 495, controller/processor 490, and I/O 497 may be entirely provided as an integral part of the flow cytometer 410. In such an embodiment, a display may also form part of the I/O capabilities 497 for presenting experimental data to users of the cytometer 400. Alternatively, some or all of the memory 495 and controller/processor 490 and I/O capabilities may be part of one or more external devices such as a general purpose computer. In some embodiments, some or all of the memory 495 and controller/processor 490 can be in wireless or wired communication with the cytometer 410. The controller/processor 490 in conjunction with the memory 495 and the I/O 497 can be configured to perform various functions related to the preparation and analysis of a flow cytometer experiment.

The system illustrated in FIG. 4B includes six different detectors that detect fluorescent light in six different wavelength bands (which may be referred to herein as a "filter window" for a given detector) as defined by the configuration of filters and/or splitters in the beam path from the flow cell 425 to each detector. Different fluorescent molecules used for a flow cytometer experiment will emit light in their own characteristic wavelength bands. The particular fluorescent labels used for an experiment and their associated fluorescent emission bands may be selected to generally coincide with the filter windows of the detectors. However, as more detectors are provided, and more labels are utilized, perfect correspondence between filter windows and fluorescent emission spectra is not possible. It is generally true that although the peak of the emission spectra of a particular fluorescent molecule may lie within the filter window of one particular detector, some of the emission spectra of that label will also overlap the filter windows of one or more other detectors. This may be referred to as spillover. The I/O 497 can be configured to receive data regarding a flow cytometer experiment having a panel of fluorescent labels and a plurality of cell populations having a plurality of markers, each cell population having a subset of the plurality of markers. The I/O 497 can also be configured to receive biological data assigning one or more markers to one or more cell populations, marker density data, emission spectrum data, data assigning labels to one or more markers, and cytometer configuration data. Flow cytometer experiment data, such as label spectral characteristics and flow cytometer configuration data can also be stored in the memory 495. The controller/processor 490 can be configured to evaluate one or more assignments of labels to markers.

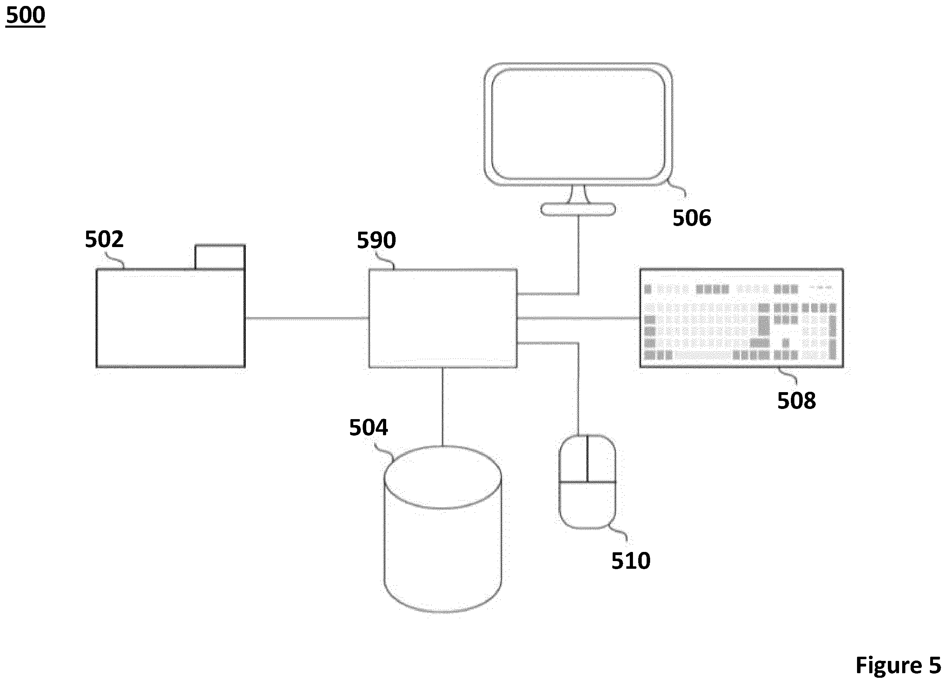

FIG. 5 shows a functional block diagram for one example of a particle analyzer control system, such as an analytics controller 500, for analyzing and displaying biological events. An analytics controller 500 can be configured to implement a variety of processes for controlling graphic display of biological events.

A particle analyzer or sorting system 502 can be configured to acquire biological event data. For example, a flow cytometer can generate flow cytometric event data. The particle analyzer 502 can be configured to provide biological event data to the analytics controller 500. A data communication channel can be included between the particle analyzer or sorting system 502 and the analytics controller 500. The biological event data can be provided to the analytics controller 500 via the data communication channel.

The analytics controller 500 can be configured to receive biological event data from the particle analyzer or sorting system 502. The biological event data received from the particle analyzer or sorting system 502 can include flow cytometric event data. The analytics controller 500 can be configured to provide a graphical display including a first plot of biological event data to a display device 506. The analytics controller 500 can be further configured to render a region of interest as a gate around a population of biological event data shown by the display device 506, overlaid upon the first plot, for example. In some embodiments, the gate can be a logical combination of one or more graphical regions of interest drawn upon a single parameter histogram or bivariate plot. In some embodiments, the display can be used to display particle parameters or saturated detector data.

The analytics controller 500 can be further configured to display the biological event data on the display device 506 within the gate differently from other events in the biological event data outside of the gate. For example, the analytics controller 500 can be configured to render the color of biological event data contained within the gate to be distinct from the color of biological event data outside of the gate. The display device 506 can be implemented as a monitor, a tablet computer, a smartphone, or other electronic device configured to present graphical interfaces.

The analytics controller 500 can be configured to receive a gate selection signal identifying the gate from a first input device. For example, the first input device can be implemented as a mouse 510. The mouse 510 can initiate a gate selection signal to the analytics controller 500 identifying the gate to be displayed on or manipulated via the display device 506 (e.g., by clicking on or in the desired gate when the cursor is positioned there). In some implementations, the first device can be implemented as the keyboard 508 or other means for providing an input signal to the analytics controller 500 such as a touchscreen, a stylus, an optical detector, or a voice recognition system. Some input devices can include multiple inputting functions. In such implementations, the inputting functions can each be considered an input device. For example, as shown in FIG. 5, the mouse 510 can include a right mouse button and a left mouse button, each of which can generate a triggering event.

The triggering event can cause the analytics controller 500 to alter the manner in which the data is displayed, which portions of the data is actually displayed on the display device 506, and/or provide input to further processing such as selection of a population of interest for particle sorting.

In some embodiments, the analytics controller 500 can be configured to detect when gate selection is initiated by the mouse 510. The analytics controller 500 can be further configured to automatically modify plot visualization to facilitate the gating process. The modification can be based on the specific distribution of biological event data received by the analytics controller 500.

The analytics controller 500 can be connected to a storage device 504. The storage device 504 can be configured to receive and store biological event data from the analytics controller 500. The storage device 504 can also be configured to receive and store flow cytometric event data from the analytics controller 500. The storage device 504 can be further configured to allow retrieval of biological event data, such as flow cytometric event data, by the analytics controller 500.

A display device 506 can be configured to receive display data from the analytics controller 500. The display data can comprise plots of biological event data and gates outlining sections of the plots. The display device 506 can be further configured to alter the information presented according to input received from the analytics controller 500 in conjunction with input from the particle analyzer 502, the storage device 504, the keyboard 508, and/or the mouse 510.

In some implementations, the analytics controller 500 can generate a user interface to receive example events for sorting. For example, the user interface can include a control for receiving example events or example images. The example events or images or an example gate can be provided prior to collection of event data for a sample, or based on an initial set of events for a portion of the sample.

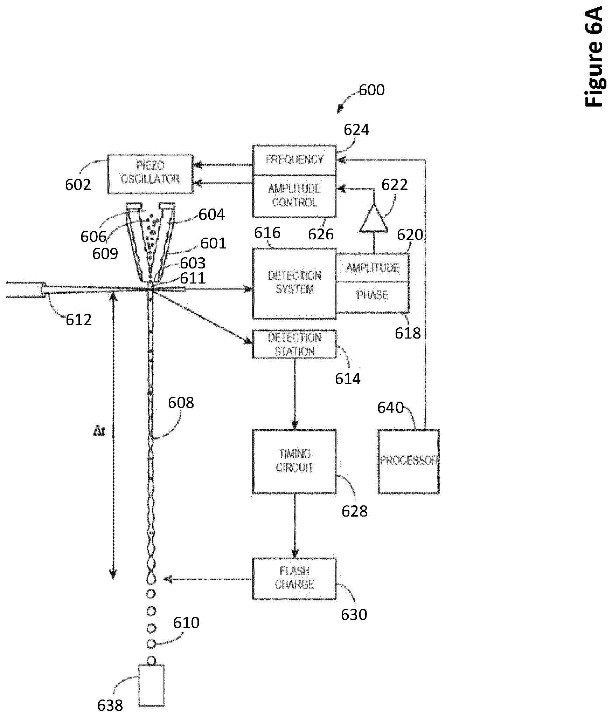

FIG. 6A is a schematic drawing of a particle sorter system 600 (e.g., the particle analyzer or sorting system 502) in accordance with one embodiment presented herein. In some embodiments, the particle sorter system 600 is a cell sorter system. As shown in FIG. 6A, a drop formation transducer 602 (e.g., piezo-oscillator) is coupled to a fluid conduit 601, which can be coupled to, can include, or can be, a nozzle 603. Within the fluid conduit 601, sheath fluid 604 hydrodynamically focuses a sample fluid 606 comprising particles 609 into a moving fluid column 608 (e.g., a stream). Within the moving fluid column 608, particles 609 (e.g., cells) are lined up in single file to cross a monitored area 611 (e.g., where laser-stream intersect), irradiated by an irradiation source 612 (e.g., a laser). Vibration of the drop formation transducer 602 causes moving fluid column 608 to break into a plurality of drops 610, some of which contain particles 609.

In operation, a detection station 614 (e.g., an event detector) identifies when a particle of interest (or cell of interest) crosses the monitored area 611. Detection station 614 feeds into a timing circuit 628, which in turn feeds into a flash charge circuit 630. At a drop break off point, informed by a timed drop delay (Δt), a flash charge can be applied to the moving fluid column 608 such that a drop of interest carries a charge. The drop of interest can include one or more particles or cells to be sorted. The charged drop can then be sorted by activating deflection plates (not shown) to deflect the drop into a vessel such as a collection tube or a multi- well or microwell sample plate where a well or microwell can be associated with drops of particular interest. As shown in FIG. 6A, the drops can be collected in a drain receptacle 638.

A detection system 616 (e.g., a drop boundary detector) serves to automatically determine the phase of a drop drive signal when a particle of interest passes the monitored area 611. An exemplary drop boundary detector is described in U.S. Pat. No. 7,679,039. The detection system 616 allows the instrument to accurately calculate the place of each detected particle in a drop. The detection system 616 can feed into an amplitude signal 620 and/or phase 618 signal, which in turn feeds (via amplifier 622) into an amplitude control circuit 626 and/or frequency control circuit 624. The amplitude control circuit 626 and/or frequency control circuit 624, in turn, controls the drop formation transducer 602. The amplitude control circuit 626 and/or frequency control circuit 624 can be included in a control system.

In some implementations, sort electronics (e.g., the detection system 616, the detection station 614 and a processor 640) can be coupled with a memory configured to store the detected events and a sort decision based thereon. The sort decision can be included in the event data for a particle. In some implementations, the detection system 616 and the detection station 614 can be implemented as a single detection unit or communicatively coupled such that an event measurement can be collected by one of the detection system 616 or the detection station 614 and provided to the non-collecting element.

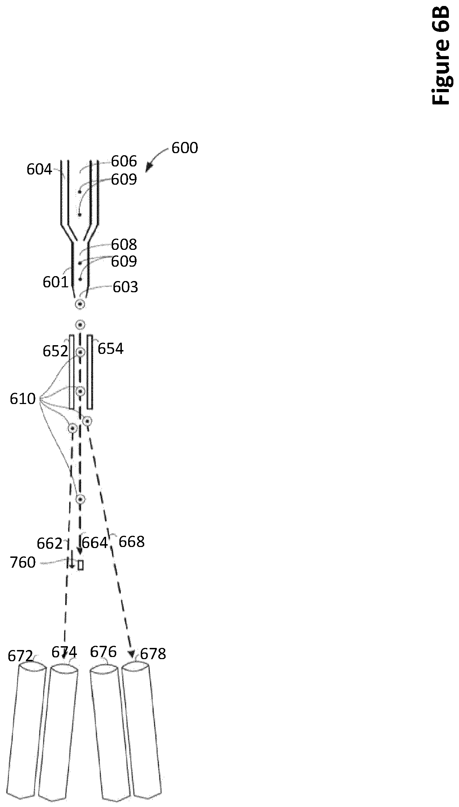

FIG. 6B is a schematic drawing of a particle sorter system, in accordance with one embodiment presented herein. The particle sorter system 600 shown in FIG. 6B, includes deflection plates 652 and 654. A charge can be applied via a stream-charging wire in a barb. This creates a stream of droplets 610 containing particles 610 for analysis. The particles can be illuminated with one or more light sources (e.g., lasers) to generate light scatter and fluorescence information. The information for a particle is analyzed such as by sorting electronics or other detection system (not shown in FIG. 6B). The deflection plates 652 and 654 can be independently controlled to attract or repel the charged droplet to guide the droplet toward a destination collection receptacle (e.g., one of 672, 674, 676, or 678). As shown in FIG. 6B, the deflection plates 652 and 654 can be controlled to direct a particle along a first path 662 toward the receptacle 674 or along a second path 668 toward the receptacle 678. If the particle is not of interest (e.g., does not exhibit scatter or illumination information within a specified sort range), deflection plates may allow the particle to continue along a flow path 664. Such uncharged droplets may pass into a waste receptacle such as via aspirator 670.

The sorting electronics can be included to initiate collection of measurements, receive fluorescence signals for particles, and determine how to adjust the deflection plates to cause sorting of the particles. Example implementations of the embodiment shown in FIG. 6B include the BD FACSAria^{™} line of flow cytometers commercially provided by Becton, Dickinson and Company (Franklin Lakes, NJ).

### NON-TRANSITORY COMPUTER-READABLE STORAGE MEDIA

Aspects of the present disclosure further include non-transitory computer readable storage media having instructions for practicing the subject methods. Computer readable storage media may be employed on one or more computers for complete automation or partial automation of a system for practicing methods described herein. In certain embodiments, instructions in accordance with the method described herein can be coded onto a computer-readable medium in the form of "programming", where the term "computer readable medium" as used herein refers to any non-transitory storage medium that participates in providing instructions and data to a computer for execution and processing. Examples of suitable non-transitory storage media include a floppy disk, hard disk, optical disk, magneto-optical disk, CD-ROM, CD-R, magnetic tape, non-volatile memory card, ROM, DVD-ROM, Blue-ray disk, solid state disk, and network attached storage (NAS), whether or not such devices are internal or external to the computer. A file containing information can be "stored" on computer readable medium, where "storing" means recording information such that it is accessible and retrievable at a later date by a computer. The computer-implemented method described herein can be executed using programming that can be written in one or more of any number of computer programming languages. Such languages include, for example, Python, Java, Java Script, C, C#, C++, Go, R, Swift, PHP, as well as many others.

Non-transitory computer readable storage media having instructions with algorithm for calculating a cell parameter for cells of a sample are also described. Non-transitory computer readable storage medium according to certain embodiments has algorithm for irradiating a sample comprising cells in a flow stream with frequency-modulated beams of light, algorithm for measuring light from the cells in the sample, algorithm for generating image data of the cells from the measured light and algorithm for calculating one or more cell parameters based on the generated image data. In some instances, the image data includes one or more waveforms generated in response to the measured light.

In some embodiments, the non-transitory computer readable storage medium includes an algorithm for generating an image of the cell from the image data. In some instances, the non-transitory computer readable storage medium includes algorithm for calculating an image parameter based on one or more of the image data and the image of the cell. In some instances, the non-transitory computer readable storage medium includes an algorithm for assessing a cell interaction parameter. In some instances, the non-transitory computer readable storage medium includes an algorithm for assessing an interaction between two or more cells in the sample. In some instances, the non-transitory computer readable storage medium includes an algorithm for assessing phagocytosis by one or more cells in the sample. In some instances, the non-transitory computer readable storage medium includes algorithm for assessing tumor cell killing by one or more cells in the sample. In some instances, the non-transitory computer readable storage medium includes an algorithm for calculating assessing co-localization between two or more cells in the sample. In some instances, the non-transitory computer readable storage medium includes an algorithm for assessing translocation of a cellular component within the cell. In some instances, the non-transitory computer readable storage medium includes an algorithm for assessing nuclear translocation of a cellular component within the cell.

In some embodiments, the non-transitory computer readable storage medium includes an algorithm for determining the mitotic phase of one or more cells in the sample. In some instances, the cell is identified as being in interphase. In some instances, the cell is identified as being in prophase. In some instances, the cell is identified as being in metaphase. In some instances, the cell is identified as being in anaphase. In some instances, the cell is identified as being in telophase. In certain instances, the cell is identified as being in cytokinesis.

In some embodiments, the non-transitory computer readable storage medium includes an algorithm for identifying that a cell is a plant cell. In some instances, the non-transitory computer readable storage medium includes algorithm for identifying the plant cell by assessing the morphology of the cell. In some instances, the non-transitory computer readable storage medium includes algorithm for assessing one or more of the size of the cell, the shape of the cell and the degree of punctateness of the cell.

In some embodiments, the non-transitory computer readable storage medium includes an algorithm for calculating a cell parameter by determining that a cell belongs to a rare cell population. The rare cell may be cells in the sample in which the abundance of the type of cell is 0.1% of the cell population or less, such as 0.05% of the cell population or less, such as 0.01% of the cell population or less, such as 0.005% of the cell population or less, such as 0.001% of the cell population or less, such as 0.0005% of the cell population or less and including where the type of cell is 0.0001% of the cell population or less. In some instances, the non-transitory computer readable storage medium includes an algorithm for calculating a cell parameter by determining that the rare cell is a stem cell. In some instances, the non-transitory computer readable storage medium includes an algorithm for calculating a cell parameter by determining that the rare cell is a circulating endothelial cell. In some instances, the non-transitory computer readable storage medium includes an algorithm for calculating a cell parameter by determining that the rare cell is a circulating tumor (malignant or benign) cell. In some instances, the non-transitory computer readable storage medium includes an algorithm for calculating a cell parameter by determining that the rare cell is a residual disease cell, such as a disease of the blood or bone marrow.

The non-transitory computer readable storage medium includes an algorithm for calculating the cell parameter based on an image parameter generated from an image of the cell. The image parameter is a quantitative image parameter. In some instances, the image parameter is selected from the group consisting of center of mass, delta center of mass, diffusivity, long axis moment, maximum intensity, short axis moment, size, total intensity, light loss by the cell nuclei, forward scattered light by the cell nuclei, side scattered light by the cell nuclei and combinations thereof. According to the present invention, eccentricity and radial moment are calculated based on the generated image data. In some instances, the non-transitory computer readable storage medium includes algorithm for calculating the image parameter from images generated based on fluorescence measured from the cells, light loss measured for the cells, forward scattered light measured for the cells, side scattered light measured for the cells and combinations thereof.

In some instances, the non-transitory computer readable storage medium includes an algorithm for classifying the cells based on the image data, a generated image of the cell and a calculated image parameter or a combination thereof. In some instances, the non-transitory computer readable storage medium includes an algorithm for classifying the cells by assigning the cells to one or more particle population clusters. In some embodiments, the non-transitory computer readable storage medium includes an algorithm for determining one or more sorting gates for the classified cells of the sample. In some instances, the one or more sorting gates capture cells of a target particle population cluster and exclude particles of a non-target particle population cluster. In some instances, the m non-transitory computer readable storage medium includes algorithm for calculating sorting gates that maximize the inclusion yield of cells of a target particle population cluster. In some instances, the non-transitory computer readable storage medium includes algorithm for calculating sorting gates that maximize the exclusion of cells of a non-target particle population cluster.

The non-transitory computer readable storage medium may be employed on one or more computer systems having a display and operator input device. Operator input devices may, for example, be a keyboard, mouse, or the like. The processing module includes a processor which has access to a memory having instructions stored thereon for performing the steps of the subject methods. The processing module may include an operating system, a graphical user interface (GUI) controller, a system memory, memory storage devices, and input-output controllers, cache memory, a data backup unit, and many other devices. The processor may be a commercially available processor or it may be one of other processors that are or will become available. The processor executes the operating system and the operating system interfaces with firmware and hardware in a well-known manner, and facilitates the processor in coordinating and executing the functions of various computer programs that may be written in a variety of programming languages, such as those mentioned above, other high level or low level languages, as well as combinations thereof, as is known in the art. The operating system, typically in cooperation with the processor, coordinates and executes functions of the other components of the computer. The operating system also provides scheduling, input-output control, file and data management, memory management, and communication control and related services, all in accordance with known techniques.

### KITS

Aspects of the present disclosure further include kits, where kits include one or more of the integrated circuits described herein. In some embodiments, kits may further include programming for the subject systems, such as in the form of a computer readable medium (e.g., flash drive, USB storage, compact disk, DVD, Blu-ray disk, etc.) or instructions for downloading the programming from an internet web protocol or cloud server. Kits may further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, and the like. Yet another form of these instructions is a computer readable medium, e.g., diskette, compact disk (CD), portable flash drive, and the like, on which the information has been recorded. Yet another form of these instructions that may be present is a website address which may be used via the internet to access the information at a removed site.

### UTILITY

The subject systems, methods and computer systems find use as an upstream assessment for cells such as for applications where cells having particular morphologies, cell mitotic stage, cellular developmental stage, cell interactions, intracellular translocation, cell type (e.g., rare cells) may be used, such as in molecular biology assays (e.g., in gene expression analysis). In some instances, the present disclosure provides for high quality and purity cells isolated from a heterogeneous sample. In addition, the subject systems and methods find use in a variety of applications where it is desirable to analyze and sort particle components in a sample in a fluid medium. In some embodiments, the systems and methods described herein find use in flow cytometry characterization of cell isolates. Embodiments of the present disclosure find use where it is desirable to provide a flow cytometer with improved cell sorting accuracy, enhanced particle collection, particle charging efficiency, more accurate particle charging and enhanced particle deflection during cell sorting.

Embodiments of the present disclosure also find use in applications where cells prepared from a biological sample may be desired for research, laboratory testing or for use in therapy. In some embodiments, the subject methods and devices may facilitate obtaining individual cells prepared from a target fluidic or tissue biological sample. For example, the subject methods and systems facilitate obtaining particular cells from fluidic or tissue samples to be used as a research or diagnostic specimen. Methods and devices of the present disclosure allow for separating and collecting cells from a biological sample (e.g., organ, tissue, tissue fragment, fluid) with enhanced efficiency and low cost as compared to traditional flow cytometry systems.

### EXAMPLES

The following examples are put forth so as to provide those of ordinary skill in the art with a complete disclosure and description of how to make and use the present invention, and are not intended to limit the scope of what the inventors regard as their invention.

### Evaluation of cell-cell interaction parameters with an imaging flow cytometer

Cell samples were stained with a plurality (up to 20 different colors) of different fluorescence dyes and visualized using an imaging flow cytometer (FACSDiscover^{™}, Bection Dickinson Biosciences, San Jose CA). Cells were visualized by generating an image of the cells. Figure 2A depicts calculating a cell-cell interaction parameter by immune synapse interrogation. The cell images showed engaged doublets and coincident doublets which were gated for and sorted. The engaged doublets included cells having active, potentially active and passive synapses demonstrating cell-cell interaction through physical contact. The coincident doublets were not in physical contact. The cells of the sample included B cell-T cell interactions, T cell-monocyte interactions, B cell-monocyte interactions and cancer cell-T cell interactions.

Figure 2B depicts visualization of a tumor cell killing assay by imaging flow cytometry according to certain embodiments. The tumor cell killing assay was gated for the imaging parameters of radial moment (light loss) and eccentricity (light loss). Lysed whole blood was spiked with Epcam+ cell line and heterogeneous doublets and homogenous doublets were visualized. The heterogenous doublets were gated and visualized in order to determine image parameters for lymphocyte-cancer cell interaction. The homogeneous doublets were gated and visualized in order to determine image parameters for lymphocyte-lymphocyte interaction.

Figure 2C depicts visualization of T cell-monocyte cell-cell interactions according to certain embodiments. As shown in the cartoon depiction, adhesion molecules expressed on leukocytes and monocytes provide for formation of complexes between lymphocytes and monocytes which can be visualized by a panel of fluorophores (e.g., BUV395, BV780, RB780 and BB515). The cells of the sample were classified into population clusters using the image parameters of radial moment (light loss) and eccentricity (light loss). Gating for T cell-monocyte complexes generated light loss images which were used to determine the cell-cell interaction parameters for these complexes based on the generated image and image parameters. Figure 2D depicts calculating sorting gates for the T cell-monocyte complexes. The sorting gates separated the T cell-monocyte complexes which exhibited correlated interactions, complexes in physical contact and coincident complexes. The sorting gates were generated using the image parameters of correlation between fluorescence detected for CD54 (fluorophore BB515) and CD11a (fluorophore RB780) and delta center of mass (Delta COM) of CD54 and CD11a. Figure 2E depicts classifying the T cell-monocyte complexes using a 20-color spectral panel as imaging parameters. The population clusters generated by classifying using a side-scattered light, light loss image parameter followed by gating for radial moment and eccentricity were then gated using fluorescence from the multi-color spectral panel. This multicolor panel provides for calculating the cell-cell interaction parameters for T cells-monocyte complexes using fluorescence imaging parameters. The multicolor panel was further used to visualize the T cell-monocyte complexes which exhibited correlated interactions, complexes in physical contact and coincident complexes as shown in Figure 2F.

Figure 2G depicts classifying and sorting extracellular vesicles-CD4 T cell complexes based on cell-cell interaction parameters according to certain embodiments. Samples containing extracellular vesicles (EVs) which were in physical contact with and bound to CD4 T cells were analyzed using an imaging flow cytometer. HeLa derived EVs were labeled with PKH67 and incubated with PBMCs. Supernatant from Tu167 cell line cells was poured off to collect the EV fraction. The EV fraction was mixed with ExoQuick O/N and polymer-based precipitation was performed by freezing the pellet until ready for use or ultrafiltration. Ultrafiltration followed by stain of the EVs with PKH67 was performed and then incubated with purified T cells or PBMCs for 2 hours. Population clusters were generated by classifying using a side-scattered light and lightloss image parameter followed by gating for difference fluorescence (CD3 RB780, CD4 BV421-A and EVs PKH67-A) Figure 2H depicts images of different complexes formed between EVs and CD4 T cells according to certain embodiments. Using imaging parameters for the EVs and assessing the size of the EVs, each of the different complexes can be gated for and sorted using these parameters.

### Evaluation of mitotic phase parameters with an imaging flow cytometer

Mitotic phases of cells in a sample were visualized with an imaging flow cytometer. A sample of cells undergoing mitosis was stained using mitochondrial labelling dyes (e.g., MitoTracker dyes) and images were generated of the cells to identify the mitotic stage of the cell. Figure 2I depicts calculating cell parameters for gating and sorting cells of different mitotic phases. Each cell in the sample was identified as being in interphase, prophase, prometaphase, metaphase, anaphase, telophase or undergoing cytokinesis. Each population cluster was gated using different image parameters used to identify each mitotic phase. The morphology of the mitochondria was used to verify the mitotic phase of the cell. Image parameters including fluorescence for DAPI (DNA stain), histone H2B and AF647 (anti-pS10H3 phospho histone H3) were used along with radial moment, max intensity and eccentricity of the histone H2B dye were used to classify the cells of the sample. Using this gating strategy, samples containing cells in different mitotic phases were collected.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

Accordingly, the preceding merely illustrates the principles of the invention. It will be appreciated that those skilled in the art will be able to devise various arrangements which, although not explicitly described or shown herein, embody the principles of the invention and are included within its scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.
The scope of the present invention, therefore, is not intended to be limited to the exemplary embodiments shown and described herein. Rather, the scope of present invention is embodied by the appended claims.

## Claims

1. A method comprising:
irradiating a sample comprising cells in a flow stream with frequency-modulated beams of light;
measuring light from the cells in the sample;
generating image data of the cells from the measured light;
calculating radial moment and eccentricity cell parameters based on the generated image data; **characterised by**
assessing an interaction between two or more cells in the sample based on the radial moment and eccentricity cell parameters calculated from the image data.

2. The method according to claim 1, wherein the image data comprises one or more waveforms generated in response to the measured light.

3. The method according to claim 1, wherein calculating the cell interaction parameter comprises assessing phagocytosis by one or more cells in the sample and/or
wherein calculating the cell interaction parameter comprises assessing tumor cell killing by one or more cells in the sample.

4. The method according to claim 1, wherein calculating the cell interaction parameter comprises assessing internalization of a cell or cellular component by one or more cells in the sample or
wherein calculating the cell interaction parameter comprises assessing co-localization between two or more cells in the sample.

5. The method according to any one of claims 1-4, wherein the cell parameter is calculated based on an image parameter generated from an image of the cell
wherein the image parameter is calculated from images generated based on fluorescence measured from the cells, light loss measured for the cells, forward scattered light measured for the cells, side scattered light measured for the cells and combinations thereof.

6. The method according to any one of claims 1-5, wherein the method comprises classifying the cells based on the calculated cell parameter, particularly
wherein classifying the cells comprises assigning the cells to one or more particle population clusters.

7. The method according to claim 6, wherein the method further comprises determining one or more sorting gates for the classified cells of the sample particularly
wherein the one or more sorting gates capture cells of a target particle population cluster and exclude cells of a non-target particle population cluster or particularly wherein the sorting gates maximize the inclusion yield of cells of a target particle population cluster, particularly.
wherein the sorting gates maximize the inclusion yield of cells of a target particle population cluster and/or particularly
wherein the sorting gates maximize the exclusion of cells of a non-target particle population cluster.

8. The method according to any one of claims 1-7, wherein the method further comprises sorting cells of the sample into a plurality of sample containers and/or
wherein measuring light from the cells in the flow stream comprises detecting fluorescence, light absorption, scattered light or a combination thereof and/or
wherein the image data is calculated from frequency-encoded fluorescence data from fluorescently-labeled cells and/or
wherein the frequency-modulated beams of light comprise a first beam of frequency shifted light and a second beam of frequency shifted light, particularly wherein the first beam of frequency shifted light comprises a local oscillator (LO) beam and the second beam of frequency shifted light comprises a radiofrequency comb beam and/or particularly
further comprising:
applying a radiofrequency drive signal to an acousto-optic device; and
irradiating the acousto-optic device with a laser to generate the first beam of frequency shifted light and the second beam of frequency shifted light, particularly
wherein the laser is a continuous wave laser.

9. A system comprising:
a light source (301) configured to irradiate cells of a sample (308) in a flow stream with frequency-modulated beams of light;
a light detection system (300b) comprising a photodetector (311, 312, 313, 314, 315, 316, 317) for measuring light from the cells; and
a processor (350, 351) comprising memory operably coupled to the processor wherein the memory comprises instructions stored thereon, which when executed by the processor, cause the processor to:
generate image data of the cells from the measured light;
calculate radial moment and eccentricity cell parameters based on the generated image data; **characterised by**
assessing an interaction between two or more cells in the sample based on the radial moment and eccentricity cell parameters calculated from the image data.

10. A non-transitory computer readable storage medium comprising instructions stored thereon wherein the non-transitory computer readable storage medium comprises:
algorithm for irradiating a sample comprising cells in a flow stream with frequency-modulated beams of light;
algorithm for measuring light from the cells in the sample;
algorithm for generating image data of the cells from the measured light;
algorithm for calculating radial moment and eccentricity cell parameters based on the generated image data;
**characterised by** an algorithm for assessing an interaction between two or more cells in the sample based on the radial moment and eccentricity cell parameters calculated from the image data.

## Patentansprüche

1. Verfahren, umfassend:
Bestrahlen einer Probe, die Zellen enthält, in einem Fließstrom mit frequenzmodulierten Lichtstrahlen;
Messen von Licht von den Zellen in der Probe;
Erzeugen von Bilddaten der Zellen aus dem gemessenen Licht;
Berechnen von Zellparametern für das radiale Moment und die Exzentrizität auf der Grundlage der erzeugten Bilddaten; **gekennzeichnet durch** Bewertung einer Wechselwirkung zwischen zwei oder mehr Zellen in der Probe auf der Grundlage der aus den Bilddaten berechneten Zellparameter für das radiale Moment und die Exzentrizität.

2. Verfahren nach Anspruch 1, wobei die Bilddaten eine oder mehrere Wellenformen umfassen, die als Reaktion auf das gemessene Licht erzeugt werden.

3. Verfahren nach Anspruch 1, wobei die Berechnung des Zellinteraktionsparameters die Beurteilung der Phagozytose durch eine oder mehrere Zellen in der Probe umfasst und/oder wobei die Berechnung des Zellinteraktionsparameters die Beurteilung der Abtötung von Tumorzellen durch eine oder mehrere Zellen in der Probe umfasst.

4. Verfahren nach Anspruch 1, wobei die Berechnung des Zellinteraktionsparameters die Bewertung der Internalisierung einer Zelle oder einer zellulären Komponente durch eine oder mehrere Zellen in der Probe umfasst oder wobei die Berechnung des Zellinteraktionsparameters die Bewertung der Kolokalisierung zwischen zwei oder mehr Zellen in der Probe umfasst.

5. Verfahren nach einem der Ansprüche 1 bis 4, wobei der Zellparameter auf der Grundlage eines Bildparameters berechnet wird, der aus einem Bild der Zelle generiert wird, wobei der Bildparameter aus Bildern berechnet wird, die auf der Grundlage von an den Zellen gemessener Fluoreszenz, an den Zellen gemessenem Lichtverlust, an den Zellen gemessenem Vorwärtsstreulicht, an den Zellen gemessenem Seitenstreulicht und Kombinationen davon generiert werden.

6. Verfahren nach einem der Ansprüche 1 bis 5, wobei das Verfahren das Klassifizieren der Zellen auf der Grundlage des berechneten Zellparameters umfasst, insbesondere wobei das Klassifizieren der Zellen das Zuordnen der Zellen zu einem oder mehreren Partikelpopulationsclustern umfasst.

7. Verfahren nach Anspruch 6, wobei das Verfahren ferner das Bestimmen eines oder mehrerer Sortiergatter für die klassifizierten Zellen der Probe umfasst, insbesondere wobei das eine oder die mehreren Sortiergatter Zellen eines Zielpartikelpopulationsclusters erfassen und Zellen eines Nicht-Zielpartikelpopulationsclusters ausschließen, oder insbesondere wobei die Sortiergatter die Einschlussausbeute von Zellen eines Zielpartikelpopulationsclusters maximieren, insbesondere wobei die Sortiergatter die Einschlussausbeute von Zellen eines Zielpartikelpopulationsclusters maximieren und/oder insbesondere wobei die Sortiergatter den Ausschluss von Zellen eines Nicht-Zielpartikelpopulationsclusters maximieren.

8. Verfahren nach einem der Ansprüche 1-7, wobei das Verfahren ferner das Sortieren von Zellen der Probe in eine Vielzahl von Probenbehältern umfasst und/oder wobei das Messen von Licht von den Zellen im Fließstrom das Erfassen von Fluoreszenz, Lichtabsorption, Streulicht oder eine Kombination davon umfasst und/oder wobei die Bilddaten aus frequenzcodierten Fluoreszenzdaten von fluoreszenzmarkierten Zellen berechnet werden, und/oder wobei die frequenzmodulierten Lichtstrahlen einen ersten Strahl aus frequenzverschobenem Licht und einen zweiten Strahl aus frequenzverschobenem Licht umfassen, insbesondere wobei der erste Strahl aus frequenzverschobenem Licht einen Lokaloszillator-Strahl und der zweite Strahl aus frequenzverschobenem Licht einen Hochfrequenz-Kamm-Strahl umfasst, und/oder insbesondere ferner umfassend:
Anlegen eines Hochfrequenz-Ansteuersignals an eine akustooptische Vorrichtung; und
Bestrahlen der akustooptischen Vorrichtung mit einem Laser, um den ersten Strahl aus frequenzverschobenem Licht und den zweiten Strahl aus frequenzverschobenem Licht zu erzeugen, insbesondere wobei der Laser ein Dauerstrichlaser ist.

9. System, umfassend:
eine Lichtquelle (301), die so konfiguriert ist, dass sie Zellen einer Probe (308) in einem Fließstrom mit frequenzmodulierten Lichtstrahlen bestrahlt;
ein Lichtdetektionssystem (300b), das einen Fotodetektor (311, 312, 313, 314, 315, 316, 317) zur Messung des von den Zellen ausgehenden Lichts umfasst; und
einen Prozessor (350, 351), der einen mit dem Prozessor funktionsfähig gekoppelten Speicher umfasst, wobei der Speicher darauf gespeicherte Befehle enthält, die, wenn sie vom Prozessor ausgeführt werden, den Prozessor veranlassen:
Bilddaten der Zellen aus dem gemessenen Licht zu erzeugen;
Zellparameter für das radiale Moment und die Exzentrizität auf der Grundlage der erzeugten Bilddaten zu berechnen; **gekennzeichnet durch**
Bewertung einer Wechselwirkung zwischen zwei oder mehr Zellen in der Probe auf der Grundlage der aus den Bilddaten berechneten Zellparameter für das radiale Moment und die Exzentrizität.

10. Nichttransitorisches, computerlesbares Speichermedium, auf dem Befehle gespeichert sind, wobei das nichttransitorische, computerlesbare Speichermedium umfasst:
einen Algorithmus zum Bestrahlen einer Probe, die Zellen in einem Fließstrom enthält, mit frequenzmodulierten Lichtstrahlen;
einen Algorithmus zum Messen von Licht von den Zellen in der Probe;
einen Algorithmus zum Erzeugen von Bilddaten der Zellen aus dem gemessenen Licht;
einen Algorithmus zum Berechnen von Zellparametern für das radiale Moment und die Exzentrizität auf der Grundlage der erzeugten Bilddaten; **gekennzeichnet durch** einen Algorithmus zur Bewertung einer Wechselwirkung zwischen zwei oder mehr Zellen in der Probe auf der Grundlage der aus den Bilddaten berechneten Zellparameter für das radiale Moment und die Exzentrizität.

## Revendications

1. Procédé comprenant:
l'irradiation d'un échantillon comprenant des cellules dans un flux avec des faisceaux lumineux modulés en fréquence ;
la mesure de la lumière provenant des cellules de l'échantillon ;
la génération de données d'image des cellules à partir de la lumière mesurée ;
le calcul des paramètres cellulaires de moment radial et d'excentricité sur la base des données d'image générées ; **caractérisé par**
l'évaluation d'une interaction entre deux ou plusieurs cellules de l'échantillon sur la base des paramètres cellulaires de moment radial et d'excentricité calculés à partir des données d'image.

2. Procédé selon la revendication 1, dans lequel les données d'image comprennent une ou plusieurs formes d'onde générées en réponse à la lumière mesurée.

3. Procédé selon la revendication 1, dans lequel le calcul du paramètre d'interaction cellulaire comprend l'évaluation de la phagocytose par une ou plusieurs cellules de l'échantillon et/ou dans lequel le calcul du paramètre d'interaction cellulaire comprend l'évaluation de la destruction de cellules tumorales par une ou plusieurs cellules de l'échantillon.

4. Procédé selon la revendication 1, dans lequel le calcul du paramètre d'interaction cellulaire comprend l'évaluation de l'internalisation d'une cellule ou d'un composant cellulaire par une ou plusieurs cellules de l'échantillon, ou dans lequel le calcul du paramètre d'interaction cellulaire comprend l'évaluation de la colocalisation entre deux ou plusieurs cellules de l'échantillon.

5. Procédé selon l'une quelconque des revendications 1 à 4, dans lequel le paramètre cellulaire est calculé sur la base d'un paramètre d'image généré à partir d'une image de la cellule, dans lequel le paramètre d'image est calculé à partir d'images générées sur la base de la fluorescence mesurée sur les cellules, de la perte de lumière mesurée pour les cellules, de la lumière diffusée vers l'avant mesurée pour les cellules, de la lumière diffusée latéralement mesurée pour les cellules et de combinaisons de celles-ci.

6. Procédé selon l'une quelconque des revendications 1 à 5, dans lequel le procédé comprend le classement des cellules sur la base du paramètre cellulaire calculé, en particulier dans lequel le classement des cellules comprend l'affectation des cellules à un ou plusieurs groupes de populations de particules.

7. Procédé selon la revendication 6, dans lequel le procédé comprend en outre la détermination d'une ou plusieurs portes de tri pour les cellules classées de l'échantillon, en particulier dans lequel la ou les portes de tri capturent les cellules d'un groupe de population de particules cible et excluent les cellules d'un groupe de population de particules non cible, ou en particulier dans lequel les portes de tri maximisent le rendement d'inclusion des cellules d'un groupe de population de particules cible, en particulier dans lequel les portes de tri maximisent le rendement d'inclusion des cellules d'un groupe de population de particules cible et/ou, en particulier, dans lequel les portes de tri maximisent l'exclusion des cellules d'un groupe de population de particules non cible.

8. Procédé selon l'une quelconque des revendications 1 à 7, dans lequel le procédé comprend en outre le tri des cellules de l'échantillon dans une pluralité de récipients d'échantillons et/ou dans lequel la mesure de la lumière provenant des cellules dans le flux comprend la détection de la fluorescence, de l'absorption de la lumière, de la lumière diffusée ou une combinaison de celles-ci et/ou dans lequel les données d'image sont calculées à partir de données de fluorescence codées en fréquence provenant de cellules marquées par fluorescence et/ou dans lequel les faisceaux lumineux modulés en fréquence comprennent un premier faisceau de lumière à fréquence décalée et un deuxième faisceau de lumière à fréquence décalée, en particulier dans lequel le premier faisceau de lumière à fréquence décalée comprend un faisceau d'oscillateur local (LO) et le deuxième faisceau de lumière à fréquence décalée comprend un faisceau en peigne radiofréquence et/ou en particulier comprenant en outre :
l'application d'un signal d'excitation radiofréquence à un dispositif acousto-optique ; et
l'irradiation du dispositif acousto-optique par un laser afin de générer le premier faisceau de lumière à fréquence décalée et le deuxième faisceau de lumière à fréquence décalée, en particulier dans lequel le laser est un laser à onde continue.

9. Système comprenant :
une source lumineuse (301) configurée pour irradier les cellules d'un échantillon (308) dans un flux avec des faisceaux lumineux modulés en fréquence ;
un système de détection de lumière (300b) comprenant un photodétecteur (311, 312, 313, 314, 315, 316, 317) destiné à mesurer la lumière provenant des cellules ; et
un processeur (350, 351) comprenant une mémoire couplée de manière opérationnelle au processeur, la mémoire comprenant des instructions qui, lorsqu'elles sont exécutées par le processeur, amènent ce dernier à :
générer des données d'image des cellules à partir de la lumière mesurée ;
calculer des paramètres cellulaires de moment radial et d'excentricité sur la base des données d'image générées ; **caractérisé par**
l'évaluation d'une interaction entre deux ou plusieurs cellules de l'échantillon sur la base des paramètres cellulaires de moment radial et d'excentricité calculés à partir des données d'image.

10. Support de stockage non transitoire lisible par ordinateur, sur lequel sont stockées des instructions, ledit support de stockage non transitoire lisible par ordinateur comprenant :
un algorithme permettant d'irradier un échantillon comprenant des cellules dans un flux avec des faisceaux lumineux modulés en fréquence ;
un algorithme permettant de mesurer la lumière provenant des cellules de l'échantillon ;
un algorithme permettant de générer des données d'image des cellules à partir de la lumière mesurée ;
un algorithme permettant de calculer les paramètres cellulaires de moment radial et d'excentricité sur la base des données d'image générées ; **caractérisé par** un algorithme permettant d'évaluer une interaction entre deux ou plusieurs cellules de l'échantillon sur la base des paramètres cellulaires de moment radial et d'excentricité calculés à partir des données d'image.
